(19) Europäisches Patentamt / European Patent Office / Office européen des brevets

(11) **EP 4 289 953 A1**

(12) **EUROPEAN PATENT APPLICATION**
published in accordance with Art. 153(4) EPC

(43) Date of publication:
**13.12.2023 Bulletin 2023/50**

(21) Application number: **22749094.3**

(22) Date of filing: **28.01.2022**

(51) International Patent Classification (IPC):
***C12N 15/113*** *(2010.01)* ***C12N 15/10*** *(2006.01)*
***A61K 48/00*** *(2006.01)* ***A61K 31/713*** *(2006.01)*
***A61P 35/00*** *(2006.01)*

(52) Cooperative Patent Classification (CPC):
**A61K 31/713; A61K 48/00; A61P 35/00; C12N 15/10; C12N 15/113**

(86) International application number:
**PCT/CN2022/074637**

(87) International publication number:
**WO 2022/166815 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.02.2021 CN 202110175652**

(71) Applicant: **Ractigen Therapeutics**
**Nantong, Jiangsu 226400 (CN)**

(72) Inventors:
- **LI, Longcheng**
  **Nantong, Jiangsu 226400 (CN)**
- **KANG, Moorim**
  **Nantong, Jiangsu 226400 (CN)**

(74) Representative: **Goddar, Heinz J.**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

# (54) DOUBLE-STRANDED NUCLEIC ACID MOLECULE FOR TREATING PROLIFERATIVE VITREORETINOPATHY AND USE THEREOF

(57) A double-stranded nucleic acid molecule, in particular a small activating nucleic acid molecule, and the use of the double-stranded nucleic acid molecule in the preparation of a drug, in particular in the preparation of a drug for activating a p21 gene and in the treatment or alleviation of proliferative vitreoretinopathy. The small activating nucleic acid molecule contains a first oligonucleotide chain and a second oligonucleotide chain which form a double-stranded structure by means of complete complementation or incomplete complementation, wherein the first oligonucleotide chain has at least 75% homology or complementarity with any continuous fragment with a length of 16-35 nucleotides of the promoter region of a human p21 gene.

EP 4 289 953 A1

## Description

TECHNICAL FIELD

The present application relates to the field of biomedicine, in particular, to gene activation related double-stranded nucleic acid molecules, such as small activating nucleic acid molecules, and use of small activating nucleic acid molecules in activating/up-regulating the transcription of Cyclin-dependent kinase inhibitor 1 (CDKN1A, p21) gene, as well as its role in regulating related diseases, for example, in the treatment of proliferative vitreoretinopathy or use in the preparation of related drugs.

BACKGROUND

Proliferative vitreoretinopathy (PVR) is an ophthalmic disease, which is a major complication after rhegmatogenous retinal detachment and retinal detachment surgery. In rhegmatogenous retinal detachment cases, the incidence of PVR is as high as 10%, which is also the main reason for poor function after successful retinal detachment surgery (Sadaka and Giuliari 2012; Kwon, Song, and Roh 2016). PVR is characterized by the formation of a periretinal membrane due to the migration and proliferation of cells after retinal rupture or trauma, which leads to retinal contraction and traction, causing retinal detachment (Zandi et al. 2019). The main cell types involved in PVR are retinal pigment epithelium (RPE) cells, glial cells (mainly Muller cells) and inflammatory cells (macrophages and lymphocytes). RPE is a layer of pigment cells arranged close to the retinal sensory nerve, which constitutes the main cells participated in PVR proliferation. RPE cells enter the vitreous and lead to exposure to a large number of cell growth factors and inflammatory factors, and the blood eye barrier is destroyed, thus triggering a series of cellular processes, such as epithelial mesenchymal transition, cell migration and diffusion, contraction of basement membrane and collagen, and finally leading to retinal disease (Mudhar 2020).

PVR is an excessive repair process after body injury, for which attempts have been made to intervene with some drugs that are still in the research and trial stage from the perspective of anti-proliferation and anti-metabolism. Although surgical treatment of PVR has certain effect, it is limited to the removal of hyperplastic tissue of the lesion and cannot prevent and treat the occurrence of PVR. Therefore, it has become a research focus to find a safe and effective drug for preventing and treating the occurrence of PVR.

CDKN1A (p21) gene encodes p21 protein, which is an important member of cyclin dependent kinase inhibitor family. It inhibits benign proliferation of tumor cells and other cells by inhibiting cell cycle progression and promoting cell senescence (Fillies et al. 2007; Romanov and Rudolph 2016). Recent studies have found that the decreased expression of p21 is associated with ocular proliferative diseases such as pterygium and retinoblastoma (Ueda et al. 2001; Audo et al. 2003); in the rabbit model of glaucoma, the recombinant adenovirus vector expressing p21 was injected under the conjunctiva, and it was found that fibrogenesis and wound healing were inhibited (Heatley et al. 2004). The potential to intervene or treat PVR by overexpressing p21 needs to be further explored.

SUMMARY

The present application provides a small activating nucleic acid molecule based on RNA activation process, such as a small activating RNA (saRNA) molecule, which treats diseases or conditions correlated with p21 gene expression, such as proliferative vitreoretinopathy, by activating/up-regulating p21 gene expression.

At least one object of the present application is to provide a RNA activation-based small activating nucleic acid molecule, which increases the expression of p21 protein by activating/up-regulating p21 gene transcription; further, the present application also provides a composition or preparation comprising above small activating nucleic acid molecule.

Another object of the present application is to provide use of a RNA activation-based small activating nucleic acid molecule or a composition containing the same in the preparation of drugs for activating/up-regulating the expression of p21 gene in cells.

Another object of the present application is to provide use of a RNA activation-based small activating nucleic acid molecule in the preparation of drugs for the treatment of diseases or conditions correlated with p21 gene expression. Further, the diseases or conditions correlated with p21 gene expression may be proliferative vitreoretinopathy.

Another object of the present application is to provide a method for activating/up-regulating the expression of p21 gene in cells and a method for treating proliferative vitreoretinopathy.

The inventor was surprised to find that when the small activating nucleic acid molecule of the present application (1) has a GC content of 35-65%; (2) does not contain 5 or more consecutive identical nucleotides; and (3) does not contain three or more duplicated or triplicated nucleotides, it can exhibit better activity in activation of p21 gene expression.

It is also a surprising finding that the targets of functional small activating nucleic acid molecules that can up-regulate p21 gene expression by at least 10% are not evenly or randomly distributed in the promoter region of p21 gene,

but are clustered. In some embodiments, several gene promoter regions with a length of at least 25 bp show aggregation of targets for the functional small activating nucleic acid molecule, that is, at least 30% of the small activating nucleic acid molecules targeting these "hotspot" regions can induce target gene mRNA expression by 1.1-fold or more; in particular, in other embodiments, the hotspots of interest are gene promoter regions with a length of at least 30 bp. In these regions, there is aggregation of targets for the functional small activating nucleic acid molecule, that is, at least 60% of small activating nucleic acid molecules targeting these hotspots can induce the mRNA expression of target genes by 1.5-fold or more.

Those skilled in the art can easily perceive other aspects and advantages of the present application from the following detailed description. Only exemplary embodiments of the present application are shown and described in the following detailed description. As those skilled in the art will recognize, the content of the present application enables those skilled in the art to make changes to the disclosed specific embodiments without departing from the spirit and scope of the invention involved in the present application. Accordingly, the descriptions in the drawings and specification of the present application are merely exemplary and not restrictive.

DESCRIPTION OF DRAWINGS

The specific features of the invention involved in the present application are shown in the appended claims. The features and advantages of the invention according to the present application can be better understood by referring to the exemplary embodiments and drawings described in detail below. A brief description of the drawings is as follows:

Fig. 1 shows the p21 gene promoter sequence, from -1000 bp upstream of the transcription start site (TSS) to 3 bp downstream of the TSS, that is, SEQ ID NO: 469. The TSS is indicated by a bent arrow.

Figs. 2A-2B show the screening of the hotspot regions for small activating RNAs on the promoter of p21 gene. According to the p21 promoter sequence shown in Fig. 1, 439 double-stranded RNA molecules were designed and chemically synthesized and transfected into PC3 cells, respectively. The p21 gene mRNA level was analyzed by QuantiGene 2.0 method after 72 hours. Fig. 2A shows the fold change (Y axis) of p21 mRNA level caused by 439 double-stranded RNA molecules (X axis) relative to the control treatment. The double-stranded RNA molecules on the X axis are sorted according to their positions relative to the TSS of the p21 gene (from the most upstream RAG-898 to the most downstream RAG-177). Eight hotspot regions (light gray rectangular box) are marked in the figure. Fig. 2B sorts the data of Fig. 2A according to the change fold of p21 mRNA expression induced by each double-stranded RNA molecule from low to high. Dashed lines in Figs. 2A and 2B indicate 2-fold induction.

Figs. 3A-3D and 3E-3H show the p21 gene activating effect of double-stranded RNA molecules targeting small activating RNA hotspots 1 to 8.

Figs. 4A-4B show that the p21 gene mRNA level was analyzed by RT-qPCR method to verify the experimental results of QuantiGene 2.0. Fig. 4A shows that 439 double-stranded RNA molecules were divided into four regions (groups) according to their activity in inducing p21 mRNA expression. Five double-stranded RNA molecules were randomly selected from each group and transfected into PC3 cells at a concentration of 10 nM. After 72 hours, the total RNA of cells was extracted, and the mRNA level of p21 gene was analyzed by RT-qPCR after reverse transcription. Fig. 4B shows the correlation of p21 gene relative mRNA levels induced by double-stranded RNA molecules detected by QuantiGene 2.0 (X axis) and RT-qPCR (Y axis).

Fig. 5 shows that a representative saRNA activateed the expression of p21 mRNA in retinal pigment epithelial cells. ARPE-19 cells were transfected with saRNA at a final concentration of 10 nM for 72 hours. After transfection, the relative expression level of p21 mRNA in each cell sample was analyzed. After transfection, RNA was extracted with Qiagen RNeasy kit, reverse transcribed and qPCR amplified with ABI 7500 fast real-time PCR system, and GAPDH gene was amplified as an internal reference. Control and dsCon2 were blank transfection and transfection with scramble double-stranded RNA, respectively. The ordinate values represent the mean ± SD of 2 replicate treatments.

Fig. 6 shows the activation of p21 mRNA expression after transfection of retinal pigment epithelial cells with different concentrations of saRNA. ARPE-19 cells were transfected with saRNA (RAG1-40-53) at final concentrations of 1, 5, 10, and 50 nM for 72 hours. After transfection, RNA was extracted with Qiagen RNeasy kit. After reverse transcription, qPCR amplification was performed with ABI 7500 fast real-time PCR system, and GAPDH gene was amplified as an internal reference. Control and dsCon2 were blank transfection and transfection with scramble sequence double-stranded RNA, respectively. The ordinate values represent the mean ± SD of 2 replicate treatments.

Figs. 7A-7B show the activation of p21 protein expression after transfection of retinal pigment epithelial cells with different concentrations of saRNA. ARPE-19 cells were transfected with saRNA (RAG1-40-53) at final concentrations of 1, 5, 10, and 50 nM for 72 hours. After the cells were collected and the total protein was extracted, the p21 protein expression level was detected by Western blot, and tubulin ($\alpha/\beta$-Tubulin) was detected at the same time as an internal reference. Fig. 7A shows the results detected by Western blot after the cells were treated with different concentrations of saRNA, and Fig. 7B shows the p21 protein expression level after the bands in Fig. 7A were quantitatively analyzed by Image J software. Control and dsCon2 were blank transfection and transfection with scramble sequence double-stranded RNA, respectively.

Figs. 8A-8B show that saRNA RAG1-40-53 inhibited retinal pigment epithelial cell growth at different concentrations. ARPE-19 cells were transfected with saRNA (RAG1-40-53) at a final concentration of 0.01-50nM. The cells were fixed with 50% TCA and dried. The experiment lasted for 7 days. The same treatment was carried out at the same time every day. After 7 days, all samples were subjected to SRB staining experiment. The growth and proliferation of cells were detected at 560 nm by microplate reader, and the data on Day 0 was taken as the baseline of cell growth. Fig. 8A shows the relative proliferation percentage of cells relative to the blank control 7 days after treatment with different concentrations of saRNA. Fig. 8B shows the relative proliferation percentage of cells 7 days after treatment with different concentrations of saRNA relative to that before treatment.

Fig. 9 shows that treatment with saRNA RAG1-40-53 did not induce apoptosis of retinal pigment epithelial cells. ARPE-19 cells were transfected with the indicated saRNA (RAG1-40-53) at final concentrations of 1, 5, 10, and 50 nM for 48 hours, and the cells were collected by centrifugation after transfection. 5 $\mu$l of each of FITC Annexin V and PI reagent was added to centrifuge tubes containing $2 \times 10^5$ cells, and the early and late apoptosis of cells were detected by flow cytometry after incubation in the dark at room temperature for 15 minutes. Control and dsCon2 were blank transfection and transfection with scramble sequence double stranded RNA, respectively. The ordinate values represent the mean $\pm$ SD of 2 replicate treatments.

Figs. 10A-10B show that the migration of retinal pigment epithelial cells was inhibited after treatment with saRNA RAG1-40-53. Adhered ARPE-19 cells were scratched and photographed as 0 h treatment, and then treated with saRNA (RAG1-40-53, 10 nM) and TGF-$\beta$ (10 ng/ml). After 24 hours, the cells were photographed at the scratch. The cell migration was analyzed by ImageJ software. Fig. 10A shows the pictures of cell migration for different treatments taken after transfection for 24 hours; Fig. 10B shows the relative area statistics of cell migration in each group of Fig. 10A with ImageJ software. The control was blank transfection.

Fig. 11 shows that cholesterol conjugated saRNA inhibited the proliferation of ARPE-19 cells after its self-transfection into said cells. ARPE-19 cells were treated with saRNA RAG1-40-53-C1 at a final concentration of 0.01-3 $\mu$M via self-delivery without using transfection reagents. After 5 days of saRNA treatment, 10 $\mu$l of CCK8 reagent was added into a 96 well plate containing 100 $\mu$L, and the proliferation of cells was detected at 450 nm after incubation at 37°C for 1 hour.

Figs. 12A-12B show the pharmacokinetic study of saRNA in a rabbit model. Fifteen female rabbits were randomly divided into three groups, one in the normal saline control group and seven in each administration group. saRNA RAG1-40-53-C1 was injected into the vitreous of rabbit eyes at doses of 0.1 and 0.3 mg, respectively, in injection volumes of 50 $\mu$L. Samples were taken at 4 hours, 1, 2, 4, 8, 14 and 21 days, respectively, and the amounts of saRNA RAG1-40-53-C1 in the vitreous and retina of rabbits were detected. Fig. 12A shows the change in saRNA amount in vitreous and retina at different time points after administration; and Fig. 12B shows the change in saRNA amount in rabbit eyes at different time points after administration.

Fig. 13 shows the pharmacodynamic study in the rabbit model. 19 female rabbits were randomly divided into 8 groups with 1-4 rabbits in each group. saRNA RAG1-40-53-C1 was injected into the vitreous of rabbit eyes at a total dose of 0.03, 0.1, 0.24, 0.3 and 1 mg, respectively. The injection volume was 100 $\mu$L. The expression of p21 mRNA in the vitreous of rabbits was detected 3 days later. RAG1-40-53-SCR-C1 (total injection dose of 0.3 and 1 mg) was used as scramble sequence control, and normal saline was used as blank control.

Fig. 14 shows the expression level of p21 mRNA after transfecting human Ku-7-LG cells with different saRNAs. Ku-7-LG cells were transfected with multiple saRNAs at a final concentration of 10 nM for 72 hours. After transfection, the relative expression level of p21 mRNA in each cell sample was analyzed. Control and dsCon2 were blank

transfection and transfection with scramble sequence double stranded RNA, respectively. The ordinate values represent the mean ± SD of 2 replicate treatments.

Figs. 15A-15C show the expression levels of p21 mRNA and protein in retinal pigment epithelial cells APRE-19 transfected with chemically modified saRNA RAG1-0M4 for different periods. ARPE-19 cells were transfected with saRNA RAG1-0M4 at a final concentration of 25 nM for 72 hours. After transfection, the expression levels of p21 mRNA and protein (Fig. 15B and Fig. 15C) in each cell sample were analyzed. Fig. 15A shows the expression level of p21 mRNA after transfection of retinal pigment epithelial cells APRE-19 with chemically modified saRNA RAG1-0M4, and Figs. 15B-15C show the protein expression level of p21 after transfection of retinal pigment epithelial cells APRE-19 with chemically modified saRNA RAG1-0M4. Control and dsCon2 were blank transfection and transfection with scramble sequence double-stranded RNA, respectively. The ordinate values represent the mean ± SD of 2 replicate treatments.

Figs. 16A-16C show the expression levels of p21 mRNA and protein after transfection of retinal pigment epithelial cells with different concentrations of chemically modified saRNA RAG1-0M4. ARPE-19 cells were transfected with saRNA RAG1-0M4 at final concentrations of 100, 50, 25, 12.5, 6.25, 3.13, 1.56, 0.78, 0.39, 0.2, and 0.1 nM for 72 hours. The expression levels of p21 mRNA and protein in each cell sample were analyzed after transfection. Fig. 16A shows the expression level of p21 mRNA after transfection of retinal pigment epithelial cells with different concentrations of chemically modified saRNA RAG1-0M4, and Fig. 16B shows the expression level of p21 protein after transfection of retinal pigment epithelial cells with different concentrations of chemically modified saRNA RAG1-0M4. Control and dsCon2 were blank transfection and transfection with scramble sequence double stranded RNA, respectively. The ordinate values represent the mean ± SD of 2 replicate treatments.

Figs. 17A-17B show the pharmacokinetic study of chemically modified saRNA RAG1-0M4-C3 (3') in a rabbit model. Four female rabbits were randomly divided into 2 groups with 2 rabbits in each group. saRNA RAG1-0M4-C3 (3') was injected into the vitreous of rabbits at a dose of 0.1 mg and 0.3mg, respectively. The change in the amount of saRNA RAG1-0M4-C3 (3') in the vitreous, plasma and retina of rabbits was detected by stem-loop PCR at 0, 0.17, 1, 3, 7 and 14 days after injection. Fig. 17A shows the change in the amount of saRNA RAG1-0M4-C3 (3') in vitreous, plasma and retina at different time points after low-dose administration; Fig. 17B shows the change in the amount of saRNA RAG1-0M4-C3 (3') in vitreous, plasma and retina at different time points after high-dose administration.

Fig. 18 shows the pharmacodynamic study in the rabbit model. Nine female rabbits were randomly divided into 4 groups, with 2-3 rabbits in each group. The saRNA RAG1-0M4-C3 (3') was injected into the vitreous of rabbit eyes at a dose of 0.3 mg with an injection volume of 0.5 ml. The expression of p21 mRNA in the vitreous of rabbits was detected 3 days and 7 days after injection. Normal saline was used as blank control.

Fig. 19 shows the expression level of p21 mRNA after transfection of ARPE-19 cells with different concentrations of chemically modified saRNA RAG1-0M4v. ARPE-19 cells were transfected with saRNA RAG1-0M4v at final concentrations of 3, 10, and 30 nM for 72 hours. After transfection, p21 mRNA expression levels in each cell sample were analyzed. Control and dsCon2 were blank transfection and transfection with scramble sequence double stranded RNA, respectively. The ordinate values represent the mean ± SD of 2 replicate treatments.

Figs. 20A-20B show the growth and apoptosis levels of ARPE-19 cells transfected with chemically modified saRNA. Fig. 20A shows the growth and proliferation proportion of ARPE-19 cells transfected with saRNA RAG1-0M4v at final concentrations of 3, 10 and 30 nM for 72 hours. Fig. 20B shows the Caspase-3/7 activity in ARPE-19 cells transfected with saRNA RAG1-0M4v at final concentrations of 3, 10, and 30 nM for 72 hours. Control and dsCon2 were blank transfection and transfection with scramble sequence double stranded RNA, respectively. The ordinate values represent the mean ± SD of 2 replicate treatments.

Figs. 21A-21B show the expression level of p21 mRNA and the level of apoptosis in ARPE-19 cells transfected with different chemically modified saRNAs RAG1-0, RAG1-0M4v and RAG1-0M4v-C3 (3'). ARPE-19 cells were transfected with saRNAs RAG1-0, RAG1-0M4v and RAG1-0M4v-C3 (3') at a final concentration of 10 nM for 72 hours. After transfection, p21 mRNA expression and Caspase-3/7 activity in each cell sample were analyzed. Fig. 21A shows the expression level of p21 mRNA after transfection of retinal pigment epithelial cells with different chemically modified saRNAs RAG1-0, RAG1-0M4v and RAG1-0M4v-C3 (3'), Fig. 21B shows the activity of Caspase-3/7 after transfection of retinal pigment epithelial cells with different chemically modified saRNAs RAG1-0, RAG1-0M4v and RAG1-0M4v-C3 (3'). Control and dsCon2 were blank transfection and transfection with scramble sequence double-stranded RNA, respectively. The ordinate values represent the mean ± SD of 2 replicate treatments.

Figs. 22A-22C show the expression levels of p21 mRNA and protein after transfection of ARPE-19 cells with different concentrations of chemically modified saRNA RAG1-0M4v-C3 (3'). ARPE-19 cells were transfected with saRNA RAG1-0M4 at final concentrations of 100, 33.3, 11.1, 3.7, 1.2, 0.412, 0.137, 0.046, 0.015, 0.005, and 0.002 nM for 72 hours. The expression levels of p21 mRNA and protein in each cell sample were analyzed after transfection. Fig. 22A shows the expression level of p21 mRNA after transfection of retinal pigment epithelial cells with different concentrations of chemically modified saRNA RAG1-0M4v-C3 (3'), and Figs. 22B-22C show the expression level of p21 protein after transfection of retinal pigment epithelial cells with different concentrations of chemically modified saRNA RAG1-0M4v-C3 (3'). Control and dsCon2 were blank transfection and transfection with scramble sequence double-stranded RNA, respectively. The ordinate values represent the mean ± SD of 2 replicate treatments.

Figs. 23A-23C show the levels of various inflammatory factors in the culture medium after transfection of peripheral blood mononuclear cells (PBMCs) with chemically modified saRNAs RAG1-0M4v and RAG1-0M4v-C3 (3') at different concentrations. The amount of inflammatory factors IL-6, IFN-α and TNF-α after transfection of PBMC cells with saRNAs RAG1-0M4v and RAG1-0M4v-C3 (3') at 133 nM for 24 hours. Fig. 23A shows the levels of the inflammatory factor IL-6 after transfection of PBMC cells with different concentrations of chemically modified saRNAs RAG1-0M4v and RAG1-0M4v-C3 (3'); Fig. 23B shows the levels of the inflammatory factor IFN-α after transfection of PBMC cells with different concentrations of chemically modified saRNAs RAG1-0M4v and RAG1-0M4v-C3 (3'); and Fig. 23C shows the levels of inflammatory factor TNF-α after transfection of PBMC cells with different concentrations of chemically modified saRNAs RAG1-0M4v and RAG1-0M4v-C3 (3'). LPS (50 ng/ml) and RAG-IS-1 were used as positive control group. Untreated means that no transfection reagent was added, wherein only PBMC cells and culture medium were used. Control and dsCon2 were blank transfection and transfection with scramble sequence double stranded RNA, respectively. The ordinate values represent the mean ± SD of 2 replicate treatments.

Fig. 24 shows the pharmacodynamic study in the rabbit model. Nine female rabbits were randomly divided into 4 groups with 2 rabbits in each group. The saRNAs RAG1-0M4v and RAG1-0M4v-C3 (3') were mixed with $2 \times 10^6$ ARPE-19 cells/0.05ml and injected into the vitreous of rabbit eyes at a dose of 0.3 mg in an injection volume of 0.5 ml. The expression of p21 mRNA in the vitreous of rabbits was detected three days later. Normal saline and dsCon2M4 were blank transfection and transfection with chemically modified scramble double-stranded RNA, respectively.

## DETAILED DESCRIPTION

The following is a description of the embodiments of the invention according to the present application by specific examples. Those skilled in the art can easily understand other advantages and effects of the invention according to the contents disclosed in the specification.

In one aspect of the present application, a small activating nucleic acid molecule, such as a small activating RNA (saRNA) molecule, which activates or up-regulates the expression of p21 gene in cells, is provided, wherein the small activating nucleic acid molecule comprises a first oligonucleotide strand and a second oligonucleotide strand forming a duplex structure with complete or incomplete complementation, the first oligonucleotide strand has at least 75% homology or complementarity with any segment with a length of 16-35 consecutive nucleotides in the promoter region of a human p21 gene, wherein the small activating nucleic acid molecule (1) has a GC content of 35-65%; (2) does not contain 5 or more consecutive identical nucleotides; and (3) does not contain 3 or more duplicated or triplicated nucleotides. In some embodiments, the promoter region of p21 gene is a region optionally spanning from the transcription start site (TSS) of the p21 gene to 1000 nucleotides upstream thereof (SEQ ID NO: 469). In some embodiments, the first oligonucleotide strand has at least 75% homology or complementarity with any segment of 16-35 consecutive nucleotides in length in a region from the transcription start site of the p21 gene to 1000 nucleotides upstream thereof (SEQ ID NO: 469).

In some embodiments, the first oligonucleotide strand of the small activating nucleic acid molecule has at least 75% homology or complementarity with any segment of a length of 16-35 consecutive nucleotides in any sequence of SEQ ID NOs: 5, 6, 7, 8, 9, 10, 11, and 12. In some embodiments, at least 75% of the homology or complementarity means that the first oligonucleotide strand has only 3, 2 or 1 nucleotides non-homologous or mismatched with any segment with a length of 16-35 consecutive nucleotides in any sequence of SEQ ID NOs: 5, 6, 7, 8, 9, 10, 11, and 12. In some embodiments, the non-homologous or mismatched nucleotides are located at the both ends of the first oligonucleotide strand, or 0-3 nucleotides away from the most central nucleotide.

In some embodiments, the first oligonucleotide strand of the small activating nucleic acid molecule has at least 75%, at least 80%, at least 85%, at least 90%, or at least 95% complementarity or homology with any sequence selected from SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412 , 415-416, 419-424,

429-432, 439-442, 447-450, 453-458, 463-468, and 479-486. In some embodiments, at least 75% of the homology or complementarity means there are only three, two or one nucleotide non-homologous or mismatched. In some embodiments, the non-homologous or mismatched nucleotides are located at the both ends of the first oligonucleotide strand, or 0-3 nucleotides away from the most central nucleotide position.

In some embodiments, the first oligonucleotide strand or the second oligonucleotide strand of the small activating nucleic acid molecule is completely identical or complementary to any nucleotide sequence of SEQ ID NOs:13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 3 99-400, 405-412, 415-416, 419-424, 429-432, 439-442, 447-450, 453-458, 463-468, and 479-486, or has 1-2 different or mismatched bases therewith.

In some embodiments, the small activating nucleic acid molecule of the present application is a double-stranded nucleic acid, and the first oligonucleotide strand and the second oligonucleotide strand are located on the two strands of the double-stranded nucleic acid, respectively. Double-stranded nucleic acid means that the first oligonucleotide strand and the second oligonucleotide strand can form a duplex nucleic acid structure by complementation.

The first oligonucleotide strand and the second oligonucleotide strand of the small activating nucleic acid molecule of the present application may be present on two different oligonucleotide strands or on the same oligonucleotide strand. When the first oligonucleotide strand and the second oligonucleotide strand are located on two strands, separately, at least one strand of the small activating nucleic acid molecule can have overhang(s) (or projection(s)) at the 5' and/or 3' ends. For example, there can be an overhang of 0-6 nucleotides, such as an overhang of 0, 1, 2, 3, 4, 5 or 6 nucleotides at the 3' end. In some embodiments, both strands of the small activating nucleic acid molecule of the present application have overhangs. In further embodiments, the 3' ends of both strands of the small activating nucleic acid molecule may each have an overhang of 0-6 nucleotides; for example, an overhang of 0, 1, 2, 3, 4, 5, or 6 nucleotides; alternatively, an overhang of 2 or 3 nucleotides. In some embodiments, the overhang can comprise nucleotide(s) in the type of dT (thymine deoxynucleotide, or T). In some embodiments, the overhang(s) at the 5' and/or 3' ends may be dTdT or dTdTdT or identical or complementary to nucleotides at corresponding positions in the target gene.

When the first oligonucleotide strand and the second oligonucleotide strand of the small activating nucleic acid molecule of the present application are present on the same oligonucleotide strand, the small activating nucleic acid molecule of the present application is a consecutive single-stranded nucleic acid, in which the first oligonucleotide strand and the second oligonucleotide strand are located on the consecutive single-stranded nucleic acid and have a complementary region that can form a duplex structure, so that the single-stranded nucleic acid can have a hairpin structure that can form a duplex region. In some embodiments, the single-stranded oligonucleotide structure can promote the expression of p21 gene in cells through, for example, RNA activation mechanism.

In the above small activating nucleic acid molecules, the length of the first oligonucleotide strand and the second oligonucleotide strand can be 16-35 nucleotides, respectively, for example, 16, 17, 18, 19, 20, 21, 22, 23, 24, 25, 26, 27, 28, 29, 30, 31, 32, 33, 34 or 35 nucleotides. The length of the first oligonucleotide strand of the small activating nucleic acid molecule can be the same as or different from the length of the second oligonucleotide strand.

In some embodiments, a segment of at least 15 nucleotides in the first oligonucleotide strand sequence forms base complementarity with the second oligonucleotide strand. For example, the length of the nucleotide segment in the first oligonucleotide strand or the second oligonucleotide strand matching the target gene promoter sequence may be 15, 16, 17, 18, 19, 20, 21, 22, 23, 24, 25, 26, 27, or 28 nucleotides. In some embodiments, the first oligonucleotide strand and the second oligonucleotide strand may have at least 75%, at least 80%, at least 85%, at least 90%, or at least 95% complementarity. In some embodiments, the complementarity can mean that there are only three, two or one mismatched nucleotide(s). In some embodiments, the mismatched nucleotide(s) may be located at the 3' or 5' end or both ends of the first oligonucleotide strand or the second oligonucleotide strand, or 0-3 nucleotides away from the most central nucleotide. In some embodiments, the mismatched nucleotide(s) can be located at the 3' or 5' end of the first oligonucleotide strand or the second oligonucleotide strand, and the other end is a blunt end or has an overhang of 1-3 nucleotides.

In some embodiments, there may be at least 1 mismatched nucleotide , for example, 1, 2, 3 or 4 mismatched nucleotides between the 3' or 5' region of the first oligonucleotide strand and the coding strand or template strand of the target gene promoter sequence. In some embodiments, there may be at least 1 mismatched nucleotides, for example, 1, 2, 3 or 4 mismatched nucleotides between the 3' or 5' region of the second oligonucleotide strand and the coding strand or template strand of the target gene promoter sequence. In some embodiments, there may be 1 mismatched nucleotide between the nucleotide(s) at the 3' end of the first oligonucleotide strand and the corresponding position(s) of the coding strand or template strand of the target gene promoter sequence. In some embodiments, there may be 1 mismatched nucleotide between the 5' end nucleotide(s) of the first oligonucleotide strand and the corresponding position(s) of the coding strand or template strand of the target gene promoter sequence.

The nucleotides in the small activating nucleic acid molecule described herein can be naturally occurring nucleotides without chemical modification, and can also include one or more modified nucleotides. In some embodiments, the modification of nucleotides in the small activating nucleic acid molecule described herein may include one or more

chemical modifications. For example, at least one nucleotide may have chemical modifications at or between the two ends of an oligonucleotide strand. The chemical modifications mentioned and used in the present application may include or be selected from one of the following modifications or any combination thereof:

(1) Modification of phosphodiester bonds of nucleotides;

(2) Modification of 2'-OH of ribose in nucleotides;

(3) Modification of base(s) in nucleotides;

(4) Inclusion of locked nucleic acid(s); and

(5) The 5' terminal nucleotide(s) of the first oligonucleotide strand and/or the second oligonucleotide strand being vinyl phosphonate modified (5'-(E)-vinylphosphonate).

The chemical modification adopts the nucleotide modifications commonly used in the art. For example, one of the modifications of the phosphodiester bond is to modify the oxygen in the phosphodiester bond, which can include, but is not limited to, phosphorothioate modification or boranophosphate modification. Both modifications are beneficial to stabilize the saRNA structure and maintain the high specificity and affinity of base pairing. Ribose modification refers to the modification of 2'-OH in nucleotide pentose, for example, the introduction of some substituents at the hydroxyl position of ribose. Examples can include but are not limited to 2'-fluoro modification, 2'-methoxy modification, 2'-oxyethylenemethoxy modification, 2,4'-dinitrophenol modification, locked nucleic acid (LNA) modification, 2'-amino modification, 2'-deoxy modification, etc. Base modification refers to the modification of nucleotide base, for example, 5'-bromouracil modification, 5'-iodouracil modification, N-methyluracil modification, 2,6-diaminopurine modification, etc. The modification can be the replacement of 2'-OH with 2'-H, and the chemically modified nucleotide can be changed from ribonucleic acid (RNA) to deoxyribonucleic acid (DNA). The modification can also be any nucleotide being a locked nucleic acid (LNA). For another example, the 5' terminal nucleotide(s) of the first oligonucleotide strand and/or the second oligonucleotide strand comprise(s) a vinyl phosphonate modification (5'-(E)-vinylphosphonate), etc. These modifications can increase the bioavailability of small activating nucleic acid molecules, improve the affinity with target sequences, or enhance the ability to resist nuclease hydrolysis in cells.

In some embodiments, the first oligonucleotide strand or the second oligonucleotide strand of the small activating nucleic acid molecule described herein may contain at least one chemically modified nucleotide. The chemical modification can be the chemical modification of 2'-OH of ribose in nucleotides, the chemical modification of phosphodiester bonds between nucleotides, the chemical modification of bases in nucleotides, the replacement of any nucleotide with locked nucleic acid (LNA), or the vinyl phosphonate modification of 5' terminal nucleotides of oligonucleotides. In some embodiments, the first oligonucleotide strand or the second oligonucleotide strand may contain 2, 3, 4, 5, 6, 7, 8, 9, 10, 11, 12, 13, 14, 15, 16, 17, 18, 19, 20, 21, 22, 23, or more chemically modified nucleotides. In some embodiments, the first oligonucleotide strand or the second oligonucleotide strand may contain at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 97%, or 100% of chemically modified nucleotides. In some embodiments, all nucleotides constituting the first oligonucleotide strand or the second oligonucleotide strand may be chemically modified nucleotides.

In addition, in some embodiments, the small activating nucleic acid molecule described herein may be conjugated with one or more of the following combinations: lipids, fatty acids, fluorescent groups, ligands, sugars, high-molecular compounds, polypeptides, antibodies, and cholesterol. The group conjugation can be either at the 3' end or 5' end of the first oligonucleotide strand or the second oligonucleotide strand, or between the 3' end and the 5' end. In some embodiments, the first oligonucleotide strand or the second oligonucleotide strand of the small activating RNA of the present application may be conjugated with at least one lipid group, located at the 3' end or 5' end of the oligonucleotide strand. In some embodiments, the lipid group is selected from one or more of fatty acyl, cationic lipid, anionic lipid, ionizable lipid, saccharolipid, glycerolipid, glycerophospholipid, sterol lipid, sphingolipid, prenol lipid, and polyketide. In some embodiments, the first oligonucleotide strand or the second oligonucleotide strand of the small activating nucleic acid molecule of the present application may be conjugated with at least one cholesterol group, located at the 3' end or 5' end of the oligonucleotide strand. In some embodiments, the first oligonucleotide strand or the second oligonucleotide strand of the small activating nucleic acid molecule of the present application may be conjugated with at least one sugar group, located at the 3' end or 5' end of the oligonucleotide strand. The sugar group includes, for example, N-acetylgalactosamine (GalNAc), glucose, mannose, and other suitable sugar groups. In some embodiments, the conjugation of one or more groups enables the small activating nucleic acid molecule of the present application to show better delivery into specific organs, tissues or cells, and also enables the small activating nucleic acid molecule of the present application

to have desired pharmaceutical characteristics, such as pharmacokinetics (pK), pharmacodynamics (pD), toxicity, and characteristics of the absorption, distribution, metabolism and excretion processes of exogenous chemicals by the body.

In order to promote the entry of small activating nucleic acid molecules into a cell, some embodiments can introduce lipophilic groups at the ends of the first oligonucleotide strand and/or the second oligonucleotide strand of the small activating nucleic acid molecules in addition to the above modifications, so as to facilitate the passage through the cell membrane and nuclear membrane composed of lipid bilayer and interaction with the gene promoter region in the nucleus. In some embodiments, the chemical group conjugated with the small activating nucleic acid molecule can be selected from one or more of structures (A), (B), or (C) :

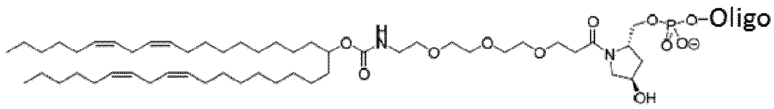

(A)

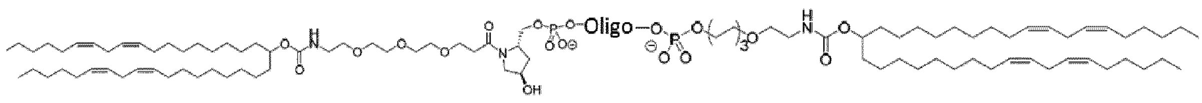

(B)

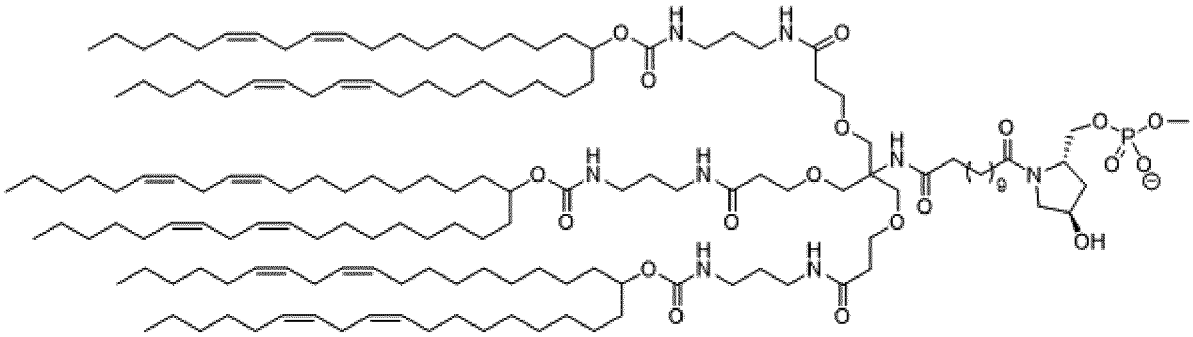

(C)

The small activating nucleic acid molecule provided in the present application can effectively activate or up-regulate the expression of CDKN1A (p21) gene in cells after contacting with the cells or being introduced into the cells. In some embodiments, the small activating nucleic acid molecule can up-regulate the expression of p21 gene by at least 10%, at least 20%, or at least 30%. After *in vitro* cell transfection test, the inventor was surprised to find that when the small activating nucleic acid molecule of the application (1) has a GC content of between 35-65%; (2) does not contain 5 or more consecutive identical nucleotides; and (3) does not contain three or more duplicated or triplicated nucleotides, it can exhibit better activation activity of p21 gene expression. For example, as one of the specific embodiments, the sequences of the first oligonucleotide strand and the second oligonucleotide strand of the small activating nucleic acid molecule are selected from the sequences shown in SEQ ID NO: 17 to SEQ ID NO: 468 in the sequence listing. For another example, as further embodiments, the sequences of the first oligonucleotide strand and the second oligonucleotide strand of the small activating nucleic acid molecule are selected from the sequences shown in Table 4, including: (1) SEQ ID NO:479 and SEQ ID NO:480; (2) SEQ ID NO:481 and SEQ ID NO:482; (3) SEQ ID NO:483 and SEQ ID NO:484; (4) SEQ ID NO:485 and SEQ ID NO:486; (5) SEQ ID NO:487 and SEQ ID NO:488; (6) SEQ ID NO:489 and SEQ ID NO:488; (7) SEQ ID NO:61 and SEQ ID NO:62; (8) SEQ ID NO:496 and SEQ ID NO:497; (9) SEQ ID NO:498 and SEQ ID NO:499; (10) SEQ ID NO:498 and SEQ ID NO:500; (11) SEQ ID NO:501 and SEQ ID NO:499; (12) SEQ ID NO:501 and SEQ ID NO:500; (13) SEQ ID NO:502 and SEQ ID NO:503; (14) SEQ ID NO:504 and SEQ ID NO:505; (15) SEQ ID NO:506 and SEQ ID NO:507; (16) SEQ ID NO:508 and SEQ ID NO:509; or (17) SEQ ID NO:510 and SEQ ID NO:511.

It was also surprising to find that the targets of functional small activating nucleic acid molecules that could up-regulate p21 gene expression by at least 10% may not be evenly or randomly distributed in the promoter region of p21 gene, but are clustered. In some embodiments, several gene promoter regions with a length of at least 25 bp can be observed with the aggregation of targets for the functional small activating nucleic acid molecules, that is, at least 30% of the small activating nucleic acid molecules targeting these "hotspot" regions can induce the mRNA expression of the target gene by 1.1-fold or more; in particular, in further embodiments, the hotspots of interest may be gene promoter regions with a length of at least 30 bp. In these regions, the aggregation of targets for the functional small activating nucleic acid moleculesis observed, that is, at least 60% of small activating nucleic acid molecules targeting these hotspots can induce the mRNA expression of the target gene by 1.5-fold or more.

These hotspot regions include H1-H8. Among them, the first oligonucleotide strand can have at least 75%, such as at least about 79%, about 80%, about 85%, about 90%, about 95%, about 99%, or about 100% homology or complementarity with 16-35 consecutive nucleotides in the -893 to -801 region (region H1, SEQ ID NO:5), -717 to -632 region (region H2, SEQ ID NO:6), -585 to -551 region (region H3, SEQ ID NO:7), -554 to -504 region (region H4, SEQ ID NO:8), -514 to -485 region (region H5, SEQ ID NO:9), -442 to -405 region (region H6, SEQ ID NO:10) - 352 to -313 region (region H7, SEQ ID NO:11) and -325 to -260 region (region H8, SEQ ID NO:12). More specifically, the first oligonucleotide strand has at least 75%, such as at least about 79%, about 80, about 85%, about 90%, about 95%, about 99%, or about 100% homology or complementarity with any nucleotide sequence selected from SEQ ID NOs: 13-468.

Another aspect of the present application is to provide the target site sequence of the isolated p21 gene for small activating nucleic acid molecules, wherein the target site sequence can include a sequence of 16-35 consecutive nucleotides of any sequence selected from SEQ ID NOs: 5-12, or a sequence with at least 75%, such as at least about 79%, about 80%, about 85%, about 90%, about 95%, about 99% or 100% homology with any sequence described above composed of 16-35 consecutive nucleotides. In some embodiments, the first oligonucleotide strand may have at least 75%, such as at least about 79%, about 80, about 85%, about 90%, about 95%, about 99%, or about 100% homology or complementarity with any nucleotide sequence selected from SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412, 415-416, 419-424, 429-432, 439-442, 447-450, 453-458, 463-468, and 479-489. In some embodiments, the homology or complementarity refers to that the two oligonucleotide sequences are the same or complementary to each other, or there are 1-2 different or mismatched bases.

The small activating nucleic acid molecule of the present application can be artificially synthesized, *in vitro* transcribed or expressed by a vector.

Another aspect of the present application also relates to a nucleic acid molecule encoding the small activating nucleic acid molecule described herein. In some embodiments, the nucleic acid molecule may be a DNA molecule. In some embodiments, the nucleic acid molecule may be an expression vector. In some embodiments, the expression vector may contain a segment encoding the small activating nucleic acid molecule described herein. After the expression vector is introduced into cells, the small activating nucleic acid molecule described herein can be expressed.

In another aspect of the present application, a cell comprising the small activating nucleic acid molecule or the nucleic acid encoding the small activating nucleic acid molecule is provided. In some embodiments, the small activating nucleic acid molecule may be a double-stranded small activating nucleic acid molecule targeting the promoter region of p21 gene, such as a double-stranded small activating RNA (saRNA) molecule, which includes a first oligonucleotide strand and a second oligonucleotide strand. In other embodiments, the small activating nucleic acid molecule may be a single-stranded small activating nucleic acid molecule targeting the promoter region of p21 gene, such as a double-stranded small activating RNA (saRNA) molecule.

Another aspect of the present application provides a composition (e.g., a pharmaceutical composition) comprising a small activating nucleic acid molecule described herein or a nucleic acid molecule encoding a small activating nucleic acid molecule described herein, and optionally, a pharmaceutically acceptable carrier. In some embodiments, the pharmaceutically acceptable carrier may include or be selected from liposomes, high molecular polymers, or polypeptides.

Alternatively, the pharmaceutical composition may be a pharmaceutical composition targeting p21 gene, and may comprise a small activating nucleic acid molecule targeting p21 gene, a nucleic acid molecule encoding the small activating nucleic acid molecule described herein, and optionally, a pharmaceutically acceptable carrier. Alternatively, the preparation is a preparation targeting p21 gene, containing the small activating nucleic acid molecule targeting p21 gene, a nucleic acid molecule encoding the small activating nucleic acid molecule described herein, a cell, or a pharmaceutical composition described herein. Alternatively, the kit contains a small activating nucleic acid molecule targeting p21 gene, a nucleic acid molecule encoding the small activating nucleic acid molecule described herein, a cell, a pharmaceutical composition, or a preparation described herein.

The pharmaceutically acceptable carriers described herein can generally include lipid based encapsulation

systems such as lipid nanoparticles (LNPS) used in the field of small nucleic acid active molecules, conjugates containing, for example, N-acetylgalactosamine (GalNAc), glucose, mannose, and other applicable carbohydrate groups targeting carbohydrate receptors in the body, covalent conjugates with different lipid or polypeptide groups as ligands, for example, one or more chemical modifications in structures (A), (B), or (C) described above. Appropriate carriers may be selected not only to help small nucleic acid molecules reach the organs or tissues to be applied, but also to help small nucleic acid molecules enter the target cells and even subcellular structures, such as double-stranded siRNA or antisense oligonucleotide strands (ASOs) into the cytoplasm, or saRNA molecules into the nucleus, so as to obtain the biological regulation effects of corresponding small nucleic acid molecules.

In another aspect of the application, a preparation is provided, which comprises: the small activating nucleic acid molecule described herein, the nucleic acid encoding the small activating nucleic acid molecule described herein, the cell containing the small activating nucleic acid molecule described herein or the nucleic acid molecule encoding the small activating nucleic acid molecule described herein, or the composition containing the small activating nucleic acid molecule described herein.

In another aspect of the application, a kit is provided, which comprises: the small activating nucleic acid molecule of the present application, the nucleic acid molecule encoding the small activating nucleic acid molecule of the present application, the cell containing the small activating nucleic acid molecule of the present application or the nucleic acid encoding the small activating nucleic acid molecule of the present application, or the composition containing the small activating nucleic acid molecule of the present application.

Another aspect of the application relates to use of the small activating nucleic acid molecule of the present application, the nucleic acid encoding the small activating nucleic acid molecule of the present application, the cell containing the small activating nucleic acid molecule of the present application or the nucleic acid encoding the small activating nucleic acid molecule of the present application, or the composition containing the small activating nucleic acid molecule of the present application in the preparation of drugs or preparations for activating or up-regulating the expression of p21 gene in cells. In some embodiments, the use is to prepare drugs or preparations for treating or alleviating proliferative vitreoretinopathy. In some embodiments, the cells include mammalian cells, such as cells from human body, such as human retinal pigment epithelial cells. In some embodiments, the cells can be *ex vivo* or *in vivo* in mammals, such as human body.

Another aspect of the present application also relates to a method of activating or up-regulating the expression of p21 gene in cells, which comprises administering to the cells the small activating nucleic acid molecule of the present application, the nucleic acid encoding the small activating nucleic acid molecule of the present application, or the composition or preparation containing the small activating nucleic acid molecule of the present application. In some embodiments, the method of activating or up-regulating the expression of p21 gene in cells comprises administering the small activating nucleic acid molecule described herein, the nucleic acid encoding the small activating nucleic acid molecule described herein, or the composition containing the small activating nucleic acid molecule described herein to the cells. In some embodiments, the cells include mammalian cells, such as cells from the human body, such as human retinal pigment epithelial cells. In some embodiments, the cells can be *ex vivo* or present in mammals, such as human body.

The small activating nucleic acid molecule of the present application can be directly introduced into cells, or it can be generated in cells after the nucleic acid sequence encoding the small activating nucleic acid molecule of the present application is introduced into the cells. In some embodiments, the cells may include mammalian cells, such as cells from human body, such as human retinal pigment epithelial cells. In some embodiments, the cells may be *ex vivo,* such as cell lines or cell strains, or may be present in mammals, such as human body. The human body can be an individual with a disease or condition associated with insufficient or reduced expression of p21 protein, such as a patient with proliferative vitreoretinopathy. The small activating nucleic acid molecule described herein can be administrated in a sufficient amount for the treatment of diseases or conditions related to the lack of p21 protein amount or the insufficient or reduced expression of p21 protein. In specific cases, the diseases or conditions associated with insufficient or reduced expression of p21 protein may include, for example, proliferative vitreoretinopathy.

Another aspect of the application relates to a method for treating a disease or condition associated with insufficient or reduced expression of p21 protein in an individual, comprising administering to the individual a therapeutically effective amount of the small activating nucleic acid molecule of the present application, the nucleic acid encoding the small activating nucleic acid molecule of the present application, the cell containing the small activating nucleic acid molecule of the present application or the nucleic acid encoding the small activating nucleic acid molecule of the present application, or the composition comprising the small activating nucleic acid molecule of the present application. In some embodiments, the method for treating a disease or condition associated with insufficient or reduced expression of p21 protein in an individual of the present application may comprise administering to the individual a therapeutically effective amount of the small activating nucleic acid molecule of the present application, the nucleic acid encoding the small activating nucleic acid molecule of the present application, the cell containing the small activating nucleic acid molecule of the present application or the nucleic acid encoding the small activating nucleic acid molecule of the present application, or the composition comprising the small activating nucleic acid molecule of the present application and other agents in

therapeutically effective amounts, including, for example, small molecule compounds, antibodies, polypeptides, proteins, etc. In some embodiments, the individual may be a mammal, including, for example, human. In some embodiments, the disease or condition associated with insufficient or reduced expression of p21 protein may include, for example, proliferative vitreoretinopathy.

Another aspect of the present application relates to a method for treating or alleviating proliferative vitreoretinopathy in an individual, comprising administering to the individual a therapeutically effective amount of the small activating nucleic acid molecule of the present application, the nucleic acid encoding the small activating nucleic acid molecule of the present application, the cell containing the small activating nucleic acid molecule of the present application or the nucleic acid encoding the small activating nucleic acid molecule of the present application, or the composition comprising the small activating nucleic acid molecule of the present application. In some embodiments, the method for treating or alleviating proliferative vitreoretinopathy of the present application comprises administering to an individual a therapeutically effective amount of the small activating nucleic acid molecule of the present application, the nucleic acid encoding the small activating nucleic acid molecule of the present application, the cell containing the small activating nucleic acid molecule of the present application or the nucleic acid encoding the small activating nucleic acid molecule of the present application, or the composition comprising the small activating nucleic acid molecule of the present application and other agents in therapeutically effective amounts, including, for example, small molecule compounds, antibodies, polypeptides, proteins, etc. In some embodiments, the individual may be a mammal, including, for example, human.

In comparison with the prior art, the present application has at least one or more of the following beneficial effects: The small activating nucleic acid molecule, such as small activating RNA (saRNA) capable of activating/up-regulating the expression of p21 gene provided by the present application can reliably activate p21 gene, so it can efficiently and specifically up-regulate or restore the expression of p21 gene and protein and has good safety at the same time. It can be used to treat diseases or disorders associated insufficient or reduced expression of p21 protein. The effect of inhibiting cell proliferation can also be achieved by increasing the level of p21 protein. Diseases or disorders that can be treated include benign cell proliferative diseases, such as proliferative vitreoretinopathy (PVR). PVR is retinal re-detachment caused by the contraction and traction of extensive fibroproliferative membrane on the retinal surface and behind the vitreous after rhegmatogenous retinal detachment surgery. p21 saRNA can be used as a drug component to inhibit the proliferation of one or more of pigment epithelial cells, glial cells, fibrocytes, fibroblasts and macrophages that cause fibroproliferative membrane.

In the present application, relevant terms are defined as follows:

As used herein, the term "complementary" or percentage "complementarity" refers to the ability of two oligonucleotide strands to form base pairs with each other. Base pairs are usually formed by hydrogen bonds between nucleotides in antiparallel oligonucleotide strands. Complementary oligonucleotide strands can be base paired in Watson-Crick manner (e.g., A-T, A-U, C-G), or in any other manner that allows duplex formation (e.g., Hoogsteen type or reverse Hoogsteen type base pairing). Complementarity includes complete complementarity and incomplete complementarity. Complete complementarity or 100% complementarity refers to the situation in which each nucleotide from the first oligonucleotide strand in the double-stranded region of a double-stranded oligonucleotide molecule can form a hydrogen bond with the nucleotide at the corresponding position of the second oligonucleotide strand without mismatch. Incomplete complementarity refers to the situation that not all the nucleotide units of two strands can form a hydrogen bond with each other. For example, for two oligonucleotide strands with double-stranded regions of 20 nucleotides in length, if only two base pairs on each strand can form a hydrogen bond with each other, the oligonucleotide strand shows 10% complementarity. In the same example, if 18 base pairs on each strand can form a hydrogen bond with each other, the oligonucleotide strand exhibits 90% complementarity. In the present application, the complementarity without giving a specific percentage refers to at least about 75%, about 79%, about 80%, about 85%, about 90%, about 95%, about 99%, or about 100% complementarity.

As used herein, the term "identity" or "homology" refers to the similarity between one oligonucleotide strand (sense strand or antisense strand) of the small activating RNA and the other oligonucleotide strand, or the coding strand or template strand of a region of the promoter sequence of a target gene. When the maximum correspondence is obtained by using sequence comparison algorithms (e.g., BLASTP and BLASTN or other algorithms available) or comparing and aligning by visual inspection, two or more oligonucleotide sequences have a certain percentage of same nucleotides. An example of an algorithm suitable for determining percentage identity and homology is the BLAST algorithm described in Altschul et al., J. Mol. Biol. 215:403-410 (1990). In the present application, "identity" or "homology" without giving a specific percentage refers to homology of at least about 75%, 79%, 80%, 85%, 90%, 95%, 99% or 100%.

As used herein, the term "oligonucleotide" refers to a polymer of nucleotides, including but not limited to single-stranded or double-stranded molecules of DNA, RNA or DNA/RNA hybrids, including oligonucleotide strands of regularly and irregularly alternating deoxyribosyl moieties and ribosyl moieties, as well as modifications of these kinds of oligonucleotides and natural or unnatural backbones. The oligonucleotides for activating the transcription of target genes described herein are small activating nucleic acid molecules.

As used herein, the terms "oligonucleotide strand" and "oligonucleotide sequence" are used interchangeably

to refer to the general term of short strand nucleotides with less than 35 bases (including nucleotide strands including deoxyribonucleic acid DNA and ribonucleic acid RNA, as well as mixed oligonucleotide strands formed by one or more deoxyribonucleotides and one or more ribonucleotides). In the present application, the length of the oligonucleotide strand can be any length of 16 to 35 nucleotides.

As used herein, the term "first oligonucleotide strand" can be either a sense strand or an antisense strand. The sense strand of a small activating RNA refers to the nucleic acid strand in the small activating RNA duplex that has identity with the coding strand of the promoter DNA sequence of the target gene, and the antisense strand refers to the nucleic acid strand complementary to the sense strand in the small activating RNA duplex.

As used herein, the term "second oligonucleotide strand" can also be a sense strand or an antisense strand. When the first oligonucleotide strand is a sense strand, the second oligonucleotide strand is an antisense strand, and when the first oligonucleotide strand is an antisense strand, the second oligonucleotide strand is a sense strand.

As used herein, the term "gene" refers to all nucleotide sequences required to encode a polypeptide strand or transcribe a functional RNA. "Genes" can be endogenous or fully or partially recombined genes for host cells (for example, due to the introduction of exogenous oligonucleotides and coding sequences encoding promoters or the introduction of heterologous promoters adjacent to endogenous coding sequences into host cells). For example, the term "gene" includes nucleic acid sequences that can be composed of exons and introns. The sequence encoding a protein is, for example, a sequence composed of exons in the open reading frame between the start codon and the stop codon. As used herein, "gene" may include, for example, gene regulatory sequences such as promoters, enhancers, and any other sequences known in the art that control the transcription, expression, or activity of another gene, no matter the other gene contains coding or non-coding sequences. In one case, for example, "gene" may be used to describe functional nucleic acids containing regulatory sequences such as promoters or enhancers. The expression of recombinant genes can be controlled by one or more heterologous regulatory sequences.

As used herein, the term "target gene" may be naturally present in nucleic acid sequence, transgene, viral or bacterial sequence, chromosome, or a sequence extra-chromosomally and/or transiently or stably transfected or incorporated into cells and/or chromatin thereof. Target genes can be protein coding genes or non-protein coding genes (such as microRNA genes and long non-coding RNA genes). Target genes usually contain promoter sequences, and small activating nucleic acid molecules designed with an identity (also known as homology) with the promoter sequences can achieve positive regulation of target genes, which is manifested as the up-regulation of target gene expression. "A promoter sequence of a target gene" or "target gene promoter sequence" refers to the non-coding sequence of the target gene. In the present application, the promoter sequence of the target gene in "complementary to the promoter sequence of a target gene" refers to the coding strand of the sequence, also known as the non-template strand, that is, the nucleotide sequence that is the same sequence as the coding sequence of the gene. "Target" or "target sequence" refers to the sequence segment in the promoter sequence of the target gene the sense oligonucleotide strand or antisense oligonucleotide of the small activating nucleic acid molecule is homologous or complementary with.

As used herein, the terms "sense strand" and "sense nucleic acid strand" can be used interchangeably. The sense oligonucleotide strand of a small activating nucleic acid molecule refers to the first oligonucleotide strand in the duplex of a small activating nucleic acid molecule that contains the same coding strand as the promoter sequence of the target gene.

As used herein, the terms "antisense strand" and "antisense nucleic acid strand" are used interchangeably. The antisense oligonucleotide strand of a small activating nucleic acid molecule refers to the second oligonucleotide strand complementary to the sense oligonucleotide strand in the duplex of small activating nucleic acid molecule.

As used herein, the term "coding strand" refers to the DNA strand in the target gene that cannot be transcribed, and the nucleotide sequence of this strand is consistent with the sequence of RNA generated by transcription (T in DNA is replaced with U in RNA). The coding strand of the double-stranded DNA sequence of the promoter of the target gene described herein refers to the promoter sequence on the same DNA strand as the coding strand of the target gene DNA.

As used herein, the term "template strand" refers to the other strand complementary to the coding strand in the double-stranded DNA of the target gene, which can be transcribed into RNA as a template. This strand is complementary to the transcribed RNA base (A-U, G-C). During transcription, a RNA polymerase binds to the template strand and moves along the 3' →5' direction of the template strand to catalyze the synthesis of RNA in the 5'→3' direction. The template strand of the double-stranded DNA sequence of the promoter of the target gene described herein refers to the promoter sequence on the same DNA strand as the target gene DNA template strand.

As used herein, the term "promoter" refers to a sequence that regulates the transcription of protein coding or RNA coding nucleic acid sequences by positional association with them. Generally, eukaryotic gene promoters contain 100~5000 base pairs, though this length range does not mean to limit the term "promoter" used herein. Although promoter sequences are generally located at the 5' end of protein coding or RNA coding sequences, promoter sequences can also exist in exon and intron sequences.

As used herein, the term "transcription start site" or its abbreviation "TSS" refers to the nucleotide that marks the transcription start on the template strand of the gene. The transcription start site can appear on the template strand

of the promoter region. A gene may have more than one transcription start site.

As used herein, the terms "overhang" and "projection" are used interchangeably to refer to the non-base paired nucleotide at the (5' or 3') end of the oligonucleotide strand, which is generated by one strand extending beyond the other strand within the double stranded oligonucleotide. Single-stranded regions extending beyond the 3' and/or 5' ends of a duplex are called overhangs.

As used herein, the term "gene activation" or "activated gene" or "gene up-regulation" or "up-regulated gene" can be used interchangeably to the increase in transcription, translation or expression or activity of a nucleic acid determined by measuring the gene transcription level, mRNA level, protein level, enzymatic activity, methylation state, chromatin state or configuration, translation level, or its activity or state in cells or biological systems. These activities or states can be measured directly or indirectly. In addition, "gene activation", "activated gene", "gene up-regulation", "up-regulated gene" refers to the increase in activity related to a nucleic acid sequence, regardless of the mechanism of such activation, such as a regulatory role as a regulatory sequence, transcription into RNA, translation into protein and increased protein expression.

As used herein, the terms "small activating RNA", "saRNA" and "small activating nucleic acid molecule" are used interchangeably to refer to nucleic acid molecules that can promote gene expression, and can be composed of a first nucleic acid fragment (antisense nucleic acid strand, also called antisense oligonucleotide strand) containing a nucleotide sequence with sequence identity or homology with the non-coding nucleic acid sequence (such as promoter, enhancer, etc.) of the target gene and a second nucleic acid fragment (sense nucleic acid strand, also called sense or sense oligonucleotide strand) containing a nucleotide sequence complementary to the first nucleic acid fragment, wherein the first nucleic acid fragment and the second nucleic acid fragment form a duplex. Small activating nucleic acid molecules may also be composed of synthetic or vector expressed single-stranded RNA molecules that can form a hairpin structure with a duplex region, comprising a first region of a nucleotide sequence having sequence identity with the promoter target sequence of the gene, and a second region comprising a nucleotide sequence that is complementary to the first region. The length of the duplex region of small activating nucleic acid molecules may generally be about 10 to about 50 base pairs, about 12 to about 48 base pairs, about 14 to about 46 base pairs, about 16 to about 44 base pairs, about 18 to about 42 base pairs, about 20 to about 40 base pairs, about 22 to about 38 base pairs, about 24 to about 36 base pairs, about 26 to about 34 base pairs, about 28 to about 32 base pairs, usually about 10, about 15, about 20, about 25, about 30, about 35, about 40, about 45, about 50 base pairs. In addition, the terms "saRNA", "small activating RNA" and "small activating nucleic acid molecule" also contain nucleic acids other than ribonucleotide moieties, including but not limited to modified nucleotides or analogues.

As used herein, the term "hotspot (H region)" refers to gene promoter regions with a length of at least 25 bp. In these regions, there is aggregation of targets for functional small activating nucleic acid molecules, that is, at least 30% of the small activating nucleic acid molecules targeting these hotspots can induce the mRNA expression of target genes by 1.1-fold or more; in particular, the hotspots of interest are gene promoter regions with a length of at least 30 bp. In these regions, the aggregation of targets of functional small activating nucleic acid molecules is observed, that is, at least 60% of small activating nucleic acid molecules targeting these hotspots can induce the mRNA expression of target genes by 1.5-fold or more.

As used herein, the term "duplicated nucleotide" refers to two consecutive identical nucleotides existing in a nucleotide sequence, such as GG, CC, TT, AA, or UU. As used herein, the term "triplicated nucleotide" refers to three consecutive identical nucleotides existing in a nucleotide sequence, such as GGG, CCC, TTT, AAA, or UUU.

As used herein, the term "synthesis" refers to a synthesis method of oligonucleotides, including any method that can synthesize RNA, such as chemical synthesis, *in vitro* transcription, expression by vector, etc.

The present application up-regulates the expression of p21 gene by RNA activation and treats related diseases, especially proliferative vitreoretinopathy, by increasing the expression of p21 protein. The p21 gene in the present application is sometimes referred to as a target gene.

The preparation method of the small activating nucleic acid molecule provided by the present application comprises sequence design and sequence synthesis.

The small activating nucleic acid molecular sequence of the present application may be synthesized by using chemical synthesis methods, or by a biotechnology company specializing in nucleic acid synthesis. Generally, the chemical synthesis methods include the following four processes: (1) synthesis of an oligoribonucleotide; (2) deprotection; (3) purification and separation; and (4) desalting and annealing.

For example, the chemical synthesis of the saRNA described herein can comprise steps as follows:

(1) Synthesis of oligoribonucleotides

The synthesis of 1 micromole of RNA is set on an automatic DNA/RNA synthesizer (for example, Applied Biosystems EXPORTE8909), and the coupling time for each cycle is set to be 10-15 minutes. The starting material is a solid-phase attached 5'-O-p-dimethoxytriphenyl-thymidine support. In the first cycle, a base is attached to the solid-phase support, and then in the $n^{th}$ cycle (19≥n≥2), a base is attached to the base attached in the $(n-1)^{th}$ cycle. This cycle is repeated until the synthesis of the whole nucleic acid sequence is completed.

(2) Deprotection

The solid-phase support attached with saRNA is added to a test tube, and 1 ml of ethanol/ammonia solution (volume ratio of 1:3) is added to this test tube, then it is sealed and placed in a 25-70°C incubator, and incubated for 2-30 hours. The solution of the solid-phase support containing saRNA is filtered and the filtrate is collected. The solid-phase support is rinsed with double distilled water twice (1 ml each time). The filtrate is collected. The eluent is combined and collected, and dried under vacuum conditions for 1-12 hours. Then, 1 ml tetrahydrofuran solution containing tetrabutylammonium fluoride (1M) is added, standing at room temperature for 4-12 hours, and 2 ml n-butanol is added, and the precipitate is collected by high-speed centrifugation to obtain the crude product of a single-strand saRNA.

(3) Purification and separation

The crude product of the obtained saRNA is dissolved in 2 ml acetate solution containing triethylamine with a concentration of 1 mol/L, and then separated by high-pressure liquid chromatography reverse phase C18 column to obtain the purified single-stranded saRNA product.

(4) Desalting and annealing

Salt is removed by volume exclusion gel filtration. The sense and antisense oligonucleotide single strands were mixed at the same molar ratio in 1-2 ml of buffer (10 mm Tris, pH = 7.5-8.0, 50 mM NaCl). The solution is heated to 95°C, and then slowly cooled to room temperature to obtain a solution containing saRNA.

It is found in the application that the above saRNA can effectively increase the expression of p21 mRNA and protein after being introduced into cells.

The present application is further described below in combination with specific embodiments and accompanying drawings. It should be understood that these embodiments are only used to illustrate the application and not to limit the scope of the application. The experimental methods without specific conditions indicated in the following examples are usually based on conventional conditions, such as those described in Sambrook, Molecular Cloning: A Laboratory Manual (New York: Cold Spring Harbor Laboratory Press, 1989), or the conditions recommended by the manufacturer.

The general experimental conditions in the present application are as follows.

**RNA isolation and reverse transcription polymerase chain reaction**

Cells were seeded at 2-3 × $10^5$ cells/well in 6-well plates and reverse transfected with oligonucleotide duplexes. Total cellular RNA was extracted using RNeasy Plus Mini Kit (Qiagen) according to its instructions. RNA (1 μg) was reverse transcribed into cDNA with PrimeScript RT kit containing gDNA Eraser (Takara, Shiga, Japan). QPCR was performed using ABI 7500 Fast Real-time PCR System (Applied Biosystems) and SYBR Premix Ex Taq II (Takara, shlga, Japan) reagents. The reaction conditions were: 95 °C for 3 seconds, 60 °C for 30 seconds, and 40 cycles of amplification. GAPDH was used as an internal reference. The primer sequences used in each example are listed in Table 1.

**TABLE 1 PRIMER SEQUENCES IN QRT-PCR ANALYSIS**

| Primer name | SEQ ID NO | Sequence (5'-3') |
|---|---|---|
| p21 F1<br>p21 R1 | SEQ ID NO:3<br>SEQ ID NO:4 | GGAAGACCATGTGGACCTGT<br>GGATTAGGGCTTCCTCTTGG |
| RAG1 SL-RT | SEQ ID NO:470 | GTCGTATCCAGTGCAGGGTCCGAGGTATTCGCACTGGATACGACTGCCAA |
| AS-6 F1<br>UniR R1 | SEQ ID NO:471<br>SEQ ID NO:472 | CGCGTATACTTGGAGAATGAGTT<br>GTGCAGGGTCCGAGGT |
| TBP-F1<br>TBP-R1 | SEQ ID NO:473<br>SEQ ID NO:474 | CCAGGTGATGCCCTTCTGTA<br>GAGCAACTCACAGTCACGCT |

(continued)

| Primer name | SEQ ID NO | Sequence (5'-3') |
|---|---|---|
| B2M-F1<br>B2M-R1 | SEQ ID NO:475<br>SEQ ID NO:476 | GATAGTTAAGTGGGATCGAGACA T<br>AGCAAGCAAGCAGAATTTGGA |
| GAPDH F<br>GAPDH R | SEQ ID NO:1<br>SEQ ID NO:2 | ATCACCATCTTCCAGGAGCGA<br>TTCTCCATGGTGGTGAAGACG |

**QuantiGene 2.0 analysis**

The cells were plated in 96 well plates and transfected with oligonucleotide duplexes. 72 hours after transfection, the mRNA level of the target gene was quantitatively detected using QuantiGene 2.0 Kit (AffyMetrix). QuantiGene 2.0 kit is a hybridization-based method that uses gene specific probes to directly quantify mRNA levels.

The experimental procedures are described briefly as follows:

Lysates were added to lyse transfected cells, cell lysates were loaded into capture well plates coated with CDKN1A (p21) and HPRT1 (housekeeping gene) probes, and hybridized overnight at 55°C. To enhance the hybridization signal, cells were hybridized with 2.0 PreAMP, 2.0 AMP and 2.0Lable Probe in sequence in 100 μL of the corresponding buffer (provided in QuantiGene 2.0 Kit). All hybridizations were shaken at 50-55 °C for 1 h. After the final washing step, 2.0 Substrate was added and incubated for 5 min at room temperature. The optical signal was then detected using Infinite 200 PRO plate reader (Tecan, Switzerland).

**Statistical analysis**

Results are expressed as mean ± standard deviation. One way ANOVA was performed using GraphPad Prism software (GraphPad Software), followed by Tukey's t test for statistical analysis. The criteria for statistical significance were set as * $P < 0.05$, ** $P < 0.01$, and *** $P < 0.001$.

The embodiments of the present application will be described in detail in combination with the following examples, but those skilled in the art will understand that the following examples are only used to illustrate the application and should not be regarded as limiting the scope of the application. If the specific conditions are not indicated in the examples, the conventional conditions or the conditions recommended by the manufacturer shall be followed. The reagents or instruments used without the manufacturer indicated are all conventional products available in market.

EXAMPLES

**EXAMPLE 1. SCREENING OF FUNCTIONAL SMALL ACTIVATING RNA (SARNA) TARGETING THE PROMOTER REGION OF P21 GENE**

To screen functional small activating RNAs that can activate p21 gene expression, 1 kb promoter sequence of p21 gene was retrieved from UCSC Genome Database (Fig. 1; SEQ ID NO: 469). A target with a size of 19 bp was selected from -1 bp upstream of the transcription start site (TSS), and a total of 982 target sequences were obtained by moving 1 bp towards the TSS site each time. The target sequences were filtered to exclude target sequences with a GC content of higher than 65% or lower than 35%, and those containing 5 or more consecutive identical nucleotides, as well as those containing 3 or more duplicated or triplicated nucleotides. After filtering, 439 target sequences remained as candidates to enter the screening process. Based on these candidates, the corresponding double-stranded RNA molecules were chemically synthesized. The sense and antisense strands of the double-stranded RNA molecules used in this experiment were 21 nucleotides in length. The 19 nucleotides in the 5' region of the first ribonucleic acid strand (sense strand) of the double-stranded RNA molecule, such as the double-stranded saRNA, had 100% identity with the promoter target sequence, with its 3' end comprising a dTdT overhang; the 19 nucleotides in the 5' region of the second ribonucleic acid strand were completely complementary to the 19 nucleotides of the 3' region sequence of the first ribonucleic acid strand, with its 3 'end comprising a dTdT overhang.

The aforementioned double-stranded RNA molecules were transfected into PC3 cells at a final concentration of 10 nM, and 72 hours later, the p21 gene mRNA level was detected using QuantiGene 2.0 kit. The fold change of p21 mRNA level of each double-stranded RNA molecule relative to the blank control treatment was calculated and plotted in Fig. 2. The fold change of p21 gene mRNA caused by all double-stranded RNA molecules in the test ranged from 0.66 (inhibition) to 8.12 (induction) (Fig. 2B). 361 (82.2%) double-stranded RNA molecules induced p21 expression by

1.01 to 8.12-fold; 74 (16.9%) double-stranded RNA molecules showed inhibition (0.99 to 0.66 fold); four double-stranded RNA molecules (0.9%) had no effect on p21 gene mRNA level (1.0-fold).

Among the 439 double-stranded RNA molecules screened, 132 double-stranded RNA molecules (30.1%) were able to induce p21 mRNA by at least 2-fold; 229 (52.4%) by at least 1.5-fold. These double-stranded RNA molecules induced p21 mRNA expression by more than 10% were identified as saRNAs, that is, functional saRNAs. These functional saRNAs are dispersed throughout the p21 promoter region. However, aggregation of functional small activating RNAs is observed in eight separate regions, which are referred to as "hotspots". A hotspot region is defined as a region targeted by at least 10 corresponding small activating RNAs, of which at least 60% is capable of inducing p21 mRNA expression by 1.5-fold or more (Figs. 2A and 3). The target sequences of hotspots 1 to 8 (H1-H8) and the corresponding small activating RNA sequences are listed in Table 2 and the sequence listing, respectively.

**TABLE 2 TARGET SEQUENCES IN HOTSPOTS IN P21 PROMOTER**

| Hotspot | SEQ ID NO | DNA sequence (5'-3') | Range (relative to TSS) | Length (bp) |
|---|---|---|---|---|
| Hotspot 1 | SEQ ID NO: 5 | | -893~-801 | 92 |
| Hotspot 2 | SEQ ID NO: 6 | | -717~-632 | 86 |
| Hotspot 3 | SEQ ID NO: 7 | | -585~-551 | 35 |
| Hotspot 4 | SEQ ID NO: 8 | | -554~-504 | 51 |
| Hotspot 5 | SEQ ID NO: 9 | aggtgatattgtggggcttttctggaaatt | -514~-485 | 30 |
| Hotspot 6 | SEQ ID NO: 10 | | -442~-405 | 38 |
| Hotspot 7 | SEQ ID NO: 11 | | -352~-313 | 40 |
| Hotspot 8 | SEQ ID NO: 12 | | -325~-260 | 66 |

These hotspots comprise:

The corresponding target sequence of Hotspot region 1 (H1) is -893 bp to -801 bp of p21 promoter sequence. The sequence is shown in SEQ ID NO: 5. Forty-four (44) functional saRNAs are found to target in this region (Fig. 3A), which are RAG-834, RAG-845, RAG-892, RAG-846, RAG-821, RAG-884, RAG-864, RAG-843, RAG-854, RAG-844, RAG-887, RAG-838, RAG-858, RAG-835, RAG-876, RAG-870, RAG-853, RAG-881, RAG-828, RAG-872, RAG-841 RAG-831, RAG-829, RAG-820, RAG-822, RAG-868, RAG-849, RAG-862, RAG-865, RAG-893, RAG-848, RAG-824, RAG-866, RAG-840, RAG-875, RAG-880, RAG-871, RAG-888, RAG-885, RAG-894, RAG-833, RAG-825, RAG-889, RAG-823;

The corresponding target sequence of Hotspot region 2 (H2) is -717 to -632 bp of p21 promoter sequence. The sequence is shown in SEQ ID NO: 6. Thirty-one (31) functional saRNAs are found to target in this region (Fig. 3B), which are RAG-693, RAG-692, RAG-688, RAG-696, RAG-694, RAG-687, RAG-691, RAG-690, RAG-689, RAG-682, RAG-686, RAG-662, RAG-695, RAG-654, RAG-658, RAG-685, RAG-704, RAG-714, RAG-705, RAG-661, RAG-656, RAG-698 RAG-697, RAG-657, RAG-715, RAG-652, RAG-651, RAG-650, RAG-716, RAG-717, RAG-711;

The corresponding target sequence of Hotspot region 3 (H3) is -585 bp to -551 bp of p21 promoter sequence. The sequence is shown in SEQ ID NO: 7. Nine (9) functional saRNAs are found to target in this region (Fig. 3C), namely RAG-580, RAG-577, RAG-569, RAG-576, RAG-570, RAG-574, RAG-585, RAG-579, RAG-584, respectively;

The corresponding target sequence of Hotspot region 4 (H4) is -554 bp to -505 bp of p21 promoter sequence. The sequence is shown in SEQ ID NO: 8. Seventeen (17) functional saRNAs are found to target in this region (Fig. 3D), namely RAG-524, RAG-553, RAG-537, RAG-526, RAG-554, RAG-523, RAG-534, RAG-543, RAG-525, RAG-535, RAG-546, RAG-545, RAG-542, RAG-531, RAG-522, RAG-529, RAG-552, respectively;

The corresponding target sequence of Hotspot region 5 (H5) is -514 bp to -485 bp of p21 promoter sequence. The sequence is shown in SEQ ID NO: 97. Nine (9) functional saRNAs are found to target in this region (Fig. 3E), namely RAG-503, RAG-504, RAG-505, RAG-506, RAG-507, RAG-508, RAG-509, RAG-510, RAG-511, RAG-512, RAG-513, RAG-514, respectively;

The corresponding target sequence of Hotspot region 6 (H6) is -442 bp to -405 bp of p21 promoter sequence. The sequence is shown in SEQ ID NO: 98. Twelve (12) functional saRNAs are found to target in this region (Fig. 3F), namely RAG-427, RAG-430, RAG-431, RAG-423, RAG-425, RAG-433, RAG-435, RAG-434, RAG-439, RAG-426, RAG-428, RAG-442, respectively;

The corresponding target sequence of Hotspot region 7 (H7) is -352 bp to -313 bp of p21 promoter sequence, and the sequence is shown in SEQ ID NO: 99. Thirteen (13) functional saRNAs were found to target in this region (Fig. 3G), namely RAG-335, RAG-351, RAG-352, RAG-331, RAG-344, RAG-342, RAG-341, RAG-333, RAG-345, RAG-346,

RAG-336, RAG-332, RAG-343, respectively;

The corresponding target sequence of Hotspot region 8 (H8) is -325 bp to -260 bp of p21 promoter sequence, and the sequence is shown in SEQ ID NO: 100. Eighteen (18) functional saRNAs were found to target in this region (Fig. 3H), namely RAG-294, RAG-285, RAG-286, RAG-292, RAG-291, RAG-284, RAG-279, RAG-280, RAG-325, RAG-293, RAG-322, RAG-321, RAG-281, RAG-289, RAG-278, RAG-283, RAG-282, RAG-295, respectively.

To further verify the QuantiGene 2.0 detection results, 439 double-stranded RNA molecules were divided into four groups (bins) according to the activity of activating p21 mRNA expression. Five double-stranded RNA molecules were randomly selected from each group and transfected into PC3 cells at a concentration of 10 nM. After 72 hours, the total RNA of cells was extracted and reverse transcribed, and the mRNA level of p21 gene was analyzed by RT qPCR. The p21 gene mRNA expression levels revealed by the two methods showed a significant correlation ($R^2$ = 0.82) (Fig. 4). All functional saRNAs obtained by QuantiGene 2.0 method can be verified as real functional small activating RNAs by RT qPCR method, and some functional saRNAs showed stronger induction of p21 mRNA expression in RT qPCR analysis (Table 3)

**TABLE 3 VERIFICATION OF 2.0 METHOD**

| Group | Name | Relative level of p21 mRNA | |
|---|---|---|---|
| | | Quantigene 2.0 | RT-qPCR |
| Bin-1 | RAG-693 | 8.12 | 48.20 |
| | RAG-834 | 8.07 | 9.64 |
| | RAG-692 | 7.69 | 29.53 |
| | RAG-845 | 6.67 | 7.15 |
| | RAG-688 | 6.55 | 42.91 |
| Bin-2 | RAG-531 | 2.06 | 3.11 |
| | RAG-705 | 2.05 | 11.33 |
| | RAG-322 | 2.04 | 7.53 |
| | RAG-840 | 2.03 | 7.01 |
| | RAG-741 | 2.02 | 5.45 |
| Bin-3 | RAG-883 | 1.00 | 1.76 |
| | RAG-177 | 1.00 | 0.31 |
| | RAG-530 | 1.00 | 1.41 |
| | RAG-879 | 1.00 | 0.98 |
| | RAG-527 | 0.99 | 1.06 |
| Bin-4 | RAG-830 | 0.72 | 0.45 |
| | RAG-419 | 0.71 | 0.41 |
| | RAG-420 | 0.71 | 0.93 |
| | RAG-700 | 0.69 | 0.32 |
| | RAG-589 | 0.66 | 0.80 |

After screening functional small activating RNAs, some representative RNAs were selected for further testing and optimization. The detailed information of saRNAs used in Examples 2-12 below is shown in Table 4.

**TABLE 4 SARNAS FOR IN VITRO TRANSFECTION OF CELLS OR ANIMAL ADMINISTRATION**

| Name | Target gene | SEQ ID NO | Sequence (5'-3') | Length (nt) |
|---|---|---|---|---|
| dsCon2 | n/a (scramble sequence control) | 477 | ACUACUGAGUGACAGUAGATT | 21 |
| | | 478 | UCUACUGUCACUCAGUAGUTT | 21 |
| RAG1-36 | CDKN1A | 479 | CCAACUCAUUCUCCAAGUC | 19 |
| | | 480 | UACUUGGAGAAUGAGUUGGCAC | 22 |

(continued)

| Name | Target gene | SEQ ID NO | Sequence (5'-3') | Length (nt) |
|---|---|---|---|---|
| RAG 1-40 | CDKN1A | 481 | CCAACUCAUUCUCCAAGUC | 19 |
| | | 482 | UACUUGGAGAAUGAGUUGGCA | 21 |
| RAG1-554 | CDKN1A | 483 | UUCUGGGAGAGGUGACCUATT | 21 |
| | | 484 | UAGGUCACCUCUCCCAGAATT | 21 |
| RAG1-884 | CDKN1A | 485 | GGGAGUAUUCAGGAGACAGTT | 21 |
| | | 486 | CUGUCUCCUGAAUACUCCCTT | 21 |
| RAG1-40-53 | CDKN1A | 487 | mCmCAACfUCAfUfUCfUCCAAG*mU*mC | 19 |
| | | 488 | fUAfCfUfUGGAGAAfUGAGfUfUGG*fC*fA | 21 |
| RAG1-40-53-C1 | CDKN1A | 489 | Chol-TEG-mCmCAACfUCAfUfUCfUCCAAG*mU*mC | 19 |
| | | 488 | fUAfCfUfUGGAGAAfUGAGfUfUGG*fC*fA | 21 |
| RAG1-40-53-Scr-C1 | n/a | 490 | Chol-TEG-mCmUAGCfUCGfUfUCfUUCCAU*mG*mC | 19 |
| | | 491 | fUCfAfUfGGAAGAAfCGAGfCfUAG*fC*fA | 21 |
| dsCon2M4 | n/a | 492 | fC*mG*fAmCfUmAfCmUfGfAfGmUfGmAfCmAfGmUfA*mG*fA | 21 |
| | | 493 | VpmU*fC*mUfAmCfUmGfUmCfAmCfUmCfAmGfUmAfGmUfCmG*fU*mC | 23 |
| RAG-IS-1 | n/a | 494 | GUCAUCACACUGAAUACCAAU | 21 |
| | | 495 | AUUGGUAUUCAGUGUGAUGACAC | 23 |
| RAG1-0 | CDKN1A | 61 | CCAACUCAUUCUCCAAGUATT | 21 |
| | | 62 | UACUUGGAGAAUGAGUUGGTT | 21 |
| RAG1-0N4 | CDKN1A | 496 | UGCCAACUCAUUCUCCAAGUA | 21 |
| | | 497 | UACUUGGAGAAUGAGUUGGCACU | 23 |
| RAG1-0M4 | CDKN1A | 498 | fU*mG*fCmCfAmAfCmUfCfAfUmUfCmUfCmCfAmAfG*mU*fA | 21 |
| | | 499 | mU*fA*mCfUmUfGmGfAmGfAmAfUmGfAmGfUmUfGmGfCmA*fC*mU | 23 |
| RAG1-0M4v | CDKN1A | 498 | fU*mG*fCmCfAmAfCmUfCfAfUmUfCmUfCmCfAmAfG*mU*fA | 21 |
| | | 500 | VPmU*fA*mCfUmUfGmGfAmGfAmAfUmGfAmGfUmUfGmGfCmA*fC*mU | 23 |
| RAG1-0M4-C3 < 3') | CDKN1A | 501 | fU*mG*fCmCfAmAfCmUfCfAfUmUfCmUfCmCfAmAfG*mU*fA-C3 | 21 |
| | | 499 | mU*fA*mCfUmUfGmGfAmGfAmAfUmGfAmGfUmUfGmGfCmA*fC*mU | 23 |

(continued)

| Name | Target gene | SEQ ID NO | Sequence (5'-3') | Length (nt) |
|---|---|---|---|---|
| RAG1-0M4v-C3 < 3') | CDKN1A | 501 | fU*mG*fCmCfAmAfCmUfCfAfUmUfCmUfCmCfAmAfG*mU*fA-C3 | 21 |
| | | 500 | VPmU*fA*mCfUmUfGmGfAmGfAmAfUmGfAmGfUmUfGmGfCmA*fC*mU | 23 |
| RAG-326 | CDKN1A | 502 | AGUGCCAACUCAUUCUCCA[dT] [dT] | 21 |
| | | 503 | UGGAGAAUGAGUUGGCACU[dT] [dT] | 21 |
| RAG-327 | CDKN1A | 504 | GAGUGCCAACUCAUUCUCC[dT] [dT] | 21 |
| | | 505 | GGAGAAUGAGUUGGCACUC [dT] [dT] | 21 |
| RAG-328 | CDKN1A | 506 | AGAGUGCCAACUCAUUCUC[dT] [dT] | 21 |
| | | 507 | GAGAAUGAGUUGGCACUCU[dT] [dT] | 21 |
| RAG-329 | CDKN1A | 508 | GAGAGUGCCAACUCAUUCU[dT] [dT] | 21 |
| | | 509 | AGAAUGAGUUGGCACUCUC[dT] [dT] | 21 |
| RAG-3 3 0 | CDKN1A | 510 | GGAGAGUGCCAACUCAUUC[dT] [dT] | 21 |
| | | 511 | GAAUGAGUUGGCACUCUCC[dT] [dT] | 21 |

In Table 4, some saRNAs were chemically modified. Asterisk (*) indicates that nucleotides are connected by phosphorothioate bond; f indicates 2'-fluoro (2'F) modification of nucleotide pentose; m indicates 2'-O-methyl (2'OME) modification of nucleotide pentose; VP indicates 5'-(E)-vinylphosphonate; CHOL-TEG indicates cholesterol (Chol) conjugated via triethyleneglycol (TEG) linker; C3 or C3 (3') indicates conjunction of oligonucleotide (oligo) to the following liposome structural group; (3') indicates conjugation at the 3 'end of the oligonucleotide:

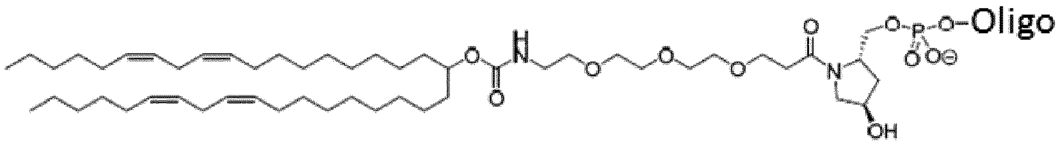

.

**EXAMPLE 2. SARNAS PROMOTE P21 MRNA EXPRESSION IN RETINAL PIGMENT EPITHELIAL CELLS**

(1) Cell culture and transfection

Human retinal pigment epithelial cells (ARPE-19) (purchased from Shanghai EK Bioscience Co., Ltd.) were cultured in DMEM medium (Gibco) containing 10% calf serum (Sigma-Aldrich) and 1% penicillin/streptomycin (Gibco). Cells were cultured in 5% $CO_2$ at 37 °C. ARPE-19 cells were plated at 10 × $10^4$ cells/well into 12 well plates, and transfected with small RNAs at a concentration of 10 nM (unless otherwise specified) with RNAiMax (Invitrogen, Carlsbad, CA) according to the manufacturer's instructions for 72 hours, and 2 duplicate wells were set for each treatment.

(2) Two-step RT qPCR

After transfection, the medium was discarded, and 500 µl cell lysate was added to each well, and incubated at room temperature for 5 minutes. RNA was extracted with Qiagen RNeasy kit, reverse transcribed and subjected to qPCR analysis in ABI 7500 Fast Real-time PCR system (Applied Biosystems). Each sample was amplified in three duplicate wells. See Table 5 for PCR reaction conditions.

**TABLE 5 PREPARATION OF REVERSE TRANSCRIPTION (RT) REACTION**

| Reagents (RT reaction 1) | Volume |
|---|---|
| 5 × gDNA Eraser buffer | 2 μl |
| gDNA Eraser | 1 μl |
| Total RNA (1 μg) + D.W | 7 μl |
| Final volume | 10 μl |
| 42°C 2 min, stored at 4°C | |
| | |
| Reagents (RT reaction 2) | Volume |
| 5×PrimeScript buffer 2 | 4 μl |
| PrimeScript RT enzyme mix 1 | 1 μl |
| RT primer mix | 1 μl |
| RNase-free dHzO | 4 μl |
| RT reaction 1 | 10 μl |
| Final volume | 20 μl |
| 37°C 15 min, 85°C 5 sec, stored at 4°C | |

The PCR reaction conditions were: 95 °C for 30 seconds, 95 °C for 5 seconds, 60 °C for 30 seconds, and 40 cycles of amplification. Meanwhile, GAPDH gene was amplified as an internal reference, and p21 was amplified with p21 F1/R1 primer pair. See Table 1 for the specific sequences of amplification primers.

To calculate the expression level (Erel) of p21 (target gene) of a saRNA transfected sample relative to the control treatment, the Ct values of the target gene and an internal reference gene were substituted into Formula 1.

$$E_{rel} = 2^{(CtTm-CtTs)} / 2^{(CtRm-CtRs)} \quad \text{(Formula 1)}$$

Where, CtTm is the Ct value of the target gene from the control treated sample; CtTs is the Ct value of the target gene from the saRNA treated sample; CtRm is the Ct value of the internal reference gene from the control treated sample; and CtRs is the Ct value of the internal reference gene from the saRNA treated sample.

The p21 mRNA level in ARPE-19 cells treated with different saRNAs in this example is shown in Fig. 5. A blank transfection is used as a control group. In comparison with the control group, the relative mRNA expression of RAG1-36, RAG1-40, RAG1-554 and RAG1-884 groups increased by 3.6-, 5.2-, 5.0- and 2.7-fold, respectively, and the activation effect of RAG1-40 was particularly significant. This indicates that the randomly selected functional saRNAs can promote the expression of p21 mRNA in ARPE-19 cells to different degrees.

## EXAMPLE 3. CHEMICAL MODIFICATION OF SARNAS AND CONJUGATION WITH CHOLESTEROL

To increase the stability of saRNAs *in vivo* and endow them with self-delivery ability, saRNA RAG1-40 was chemically modified, including the introduction of 2'-O-methyl modification at specific sites, 2'-fluoro modification and phosphorothioate bond modification between nucleotides to obtain saRNA RAG1-40-53. Further, cholesterol group was conjugated at the 5' end of the sense strand by TEG to obtain saRNA RAG1-40-53-C1 (Table 4). The control sequence RAG1-40-53-Scr-C1 was obtained by scrambling the sequence of RAG1-40-53-C1 (Table 4).

## EXAMPLE 4. UP-REGULATION OF P21 MRNA EXPRESSION IN RETINAL PIGMENT EPITHELIAL CELLS WITH DIFFERENT DOSES OF SARNAS

(1) Cell culture and transfection

Cell culture was performed as described in Example 2. ARPE-19 cells were plated at $10 \times 10^4$ cells/well into 12 well plates. Small activating RNAs were transfected using RNAiMax (Invitrogen, Carlsbad, CA) at final concentrations of 1, 5, 10, and 50 nM for 72 hours, using 2 duplicate wells for each treatment. Two step RT-qPCR was performed as described in Example 2.

The experimental results of Example 4 are shown in Fig. 6. Based on the results of Example 2, RAG1-40, which can effectively activate p21, was selected for chemical modification, and RAG1-40-53 was the chemically modified p21 saRNA (see Table 4 for specific modification sites and methods). The blank transfection control was used as a control group. In comparison with the control group, the expression of p21 mRNA in cells transfected with RAG1-40-53 at concentrations of 1, 5, 10 and 50 nM increased by 2.0-, 3.1-, 2.8- and 2.3-fold, respectively, and the expression of p21 mRNA was the highest at 5 nM, indicating that different concentrations of RAG1-40-53 can promote the expression of p21 mRNA to different degrees.

## EXAMPLE 5. UP-REGULATION OF P21 PROTEIN EXPRESSION IN RETINAL PIGMENT EPITHELIAL CELLS WITH DIFFERENT DOSES OF SARNAS

(1) Protein collection and detection

Cell culture was performed as described in Example 2, and cell transfection was performed as described in Example 4. 72 hours after transfection, lysing was performed with an appropriate amount of cell lysate containing protease inhibitors (1 × RIPA buffer, Cell Signaling Technology). Protein quantification was performed by BCA method, followed by polyacrylamide gel electrophoresis and transferred to a 0.45 μm PVDF membrane. The primary antibody used was rabbit monoclonal anti-p21 (Cell Signaling Technology, 2947s), and α/β-Tubulin (Cell Signaling Technology, 2148s) was used to detect the blots; the secondary antibody was anti-rabbit IgG, HRP linked antibody (Cell Signaling Technology). The membrane was scanned with Image Lab (Bio-Rad, Chemistry Doctm MP Imaging System) to detect the signal.

The experimental results are shown in Fig. 7. Different doses of saRNAs can up-regulate the expression of p21 protein. The blank transfection control was used as a control group. In comparison with the control group, the expression of p21 protein increased by 1.4-, 1.3-, 1.4- and 1.4-fold at the transfection concentrations of 1, 5, 10 and 50 nM, respectively. The experimental results showed that there was no strict dose-dependent relationship between the expression of p21 protein and the transfection concentration of RAG1-40-53, and a relatively high protein expression was observed at a low transfection concentration (1 nM).

## EXAMPLE 6. INHIBITION OF THE GROWTH OF RETINAL PIGMENT EPITHELIAL CELLS WITH SARNAS THAT ACTIVATE P21

(1) Cell culture and transfection

Cell culture was performed as described in Example 2. ARPE-19 cells were plated at 2000 cells per well into 96 well plates, transfected with small activating RNA using RNAiMax, and transfected with RAG1-40-53 at final concentrations of 0.01, 0.05, 0.1, 0.5, 1, 5, 10, 25, and 50 nM. A group of blank transfection was set as the control, and the day of transfection was Day 0 (D0), and the experimental time after transfection was 7 days. Three duplicate wells were set for each treatment.

(2) SRB staining experiment

Within 7 days after ARPE-19 cell transfection, cells were fixed with 50% TCA at the same time every day at 4°C for 1 hour, rinsed with distilled water and dried at room temperature for standby; after the experiment, the samples of 7 days were uniformly subjected to SRB staining experiment, and 50 μl of 0.4% SRB staining solution was added to each well for incubation for 30 minutes at room temperature; after the incubation, the SRB dye was removed and 200 μl of 1% acetic acid was added to rinse for 4 times to remove the excess SRB dye, and dried at room temperature; 100 μl of 10 mM Tris base (pH 10.5) was added and incubated on a shaker for 10 min; the absorbance was read at 560 nm using a microplate reader, thus reflecting the growth and proliferation of cells. The data on Day 0 was taken as the baseline value of cell growth, and then the data of blank transfection group were used as the reference of cell proliferation on Day 1-7, and the relative proliferation percentage of RAG1-40-53 transfected cells at each concentration compared

with blank transfected cells on the same sampling day was calculated.

Fig. 8A shows the relative proliferation percentage of cells after treatment with different concentrations of saRNA in 7 days. ARPE-19 cells did not show a decrease in the relative proliferation percentage of cells at any concentration of RAG1-40-53 on Day 1 and Day 2. A decrease in the relative proliferation percentage of ARPE-19 cells was observed at a concentration of 0.1 nM from Day 3. A significant decrease in the relative proliferation percentage of cells was observed at a dose of 1 nM after Day 4. The relative proliferation percentage of cells at a dose of 10 nM on Day 7 was only about 10%. Fig. 8B shows the percentage of the number of cells in each group on Days 1-7 relative to the number of cells on Day 0 after treating cells with different concentrations of RAG1-40-53. In the first 3 days of ARPE-19 cell treatment, the cells in the RAG1-40-53 treatment groups at various concentrations only proliferated slightly, and as the RAG1-40-53 transfection concentration increased, the growth of cell number gradually decreased, and after Day 4, the cells in the 0.01 nM treatment group showed obvious proliferation, but as saRNA (RAG1-40-53) concentration increased, the growth of cell number began to decrease. Although the number of cells in each concentration of RAG1-40-53 treatment groups was less than that in the blank transfection group within 7 days, the number of cells in each group 1-7 days after transfection was still higher than that on Day 0, which indicated that the overall mitigation of cell proliferation caused by p21 saRNA (RAG1-40-53) was not due to cytotoxicity but inhibition of cell growth.

## EXAMPLE 7. THE INHIBITION OF THE GROWTH OF RETINAL PIGMENT EPITHELIAL CELLS BY P21 ACTIVATING SARNA IS NOT CAUSED BY APOPTOSIS

(1) Cell culture and transfection

Cell culture was performed as described in Example 2. ARPE-19 cells were plated at 10 × $10^4$ cells/well into 12-well plates. Small activating RNAs were transfected using RNAiMax (Invitrogen, Carlsbad, CA) at final concentrations of 1, 5, 10, and 50 nM for 48 hours, using 2 duplicate wells for each treatment.

(2) Apoptosis was detected by flow cytometry

After cell transfection, the supernatant was discarded to collect cells, 500 μl PBS was added to each well to wash once, PBS was discarded, 100 μl trypsin was added to each well, 900 μl medium was added to terminate digestion and transferred to a 1.5 ml centrifuge tube, centrifuged (400rcf, 5min) to collect all cells and counted (1~2 × $10^5$ cells /100 μl Binding Buffer). 100 μl of 1 × Binding buffer (B&D Systems, 51-66121E), 5 μl of FITC annexin V (B&D Systems, 51-65874X) and 5 μl PI (B&D Systems, 51-66211E) were incubated at room temperature in dark for 15 min. After the incubation, 100 μl of 1 × Binding Buffer was mixed and transferred to a 96 well plate for analysis with a flow cytometer (Beckman Coulter). The sample should be analyzed within 1 hour.

As shown in Fig. 9, the proportion of viable cells and early and late apoptosis in the control group were 96.2% and 1.4%, respectively, and the number of viable cells in each treatment group of p21 saRNA (RAG1-40-53) was more than 90%. At the treatment concentrations of 1, 5, 10 and 50 nM, the proportions of early and late apoptosis were 2.5%, 3.2%, 3.2% and 2.9%, respectively. In comparison with the control group, there was no significant difference in p21 saRNA (RAG1-40-53) treatment groups, and no significant increase in apoptosis. This indicates that the inhibition of ARPE-19 cell growth by human p21 saRNA is not caused by apoptosis.

## EXAMPLE 8. P21 ACTIVATING SARNA INHIBITS RETINAL PIGMENT EPITHELIAL CELL MIGRATION

(1) Cell culture and transfection

Cell culture was performed as described in Example 2. ARPE-19 cells were plated at 30 × $10^4$ cells/well into 12-well plates. After the cells adhered to wall, a sterilized tip was used to scratch the well plates, and 500 μl PBS was added to wash the floating cells in the well plates for three times. The cells were photographed, observed and recorded as 0 H. RNAiMax (Invitrogen, Carlsbad, CA) was used to transfect small activating RNA (RAG1-40-53) at a final concentration of 10 nM or TGF-β (10 ng/ml) was added, TGF-β was used alone as a growth factor as a positive reference, or in combination with p21 saRNA (RAG1-40-53). 24 hours after transfection, pictures were taken to observe cell migration.

Fig. 10A shows the pictures of cell migration under the microscope after 0 and 24 hours. Image J software was used to analyze the area of cell migration. Fig. 10B shows the relative area of cell migration corresponding to Fig. 10A (based on the area of cell migration in the control group, recorded as 1). In comparison with the control group, the relative area of cell migration in the TGF-β treatment group was 1.41, indicating that TGF-β could successfully induce cell migration within 24 h. In comparison with the control group, the relative area of cell migration in the RAG1-40-53 treatment group was 0.82, indicating that RAG1-40-53 can inhibit cell migration. In comparison with TGF-β or RAG1-40-53 alone

treatment, the relative area of cell migration for the combination treatment of RAG1-40-53 + TGF-β was 0.98, indicating that RAG1-40-53 can inhibit TGF-β induced cell migration.

## EXAMPLE 9. CHOLESTEROL CONJUGATED P21 ACTIVATING SARNA CAN BE SELF-DELIVERED TO RETINAL PIGMENT EPITHELIAL CELLS AND INHIBIT THEIR PROLIFERATION

(1) Cell culture and treatment

Cell culture was performed as described in Example 2. ARPE-19 cells were plated into 96 well plates at 2000 cells per well without RNAiMax. saRNA (RAG1-40-53-C1, see Table 4 for details) in Fig. 11 was used at the final concentrations of 0.01, 0.03, 0.1, 0.3, 1, and 3 μM to treat ARPE-19 cells, and a group of cells without any treatment were set as the control. The day of treatment was Day 0 (D0), and the cells were allowed to take up saRNA freely. The treatment time was 5 days, and 3 duplicate wells were set for each treatment.

(2) CCK8 experiment

After cell treatment, 10 μl of CCK8 reagent was added to 100 μL cell culture medium, and the cells were incubated in a cell incubator for 1 hour. After incubation, the absorbance was detected at 450 nm using a microplate reader, and the control treated cells were used as the control. The absorbance reflects the growth and proliferation of cells. The data on Day 0 was taken as the baseline value of cell growth, and the data of the control group at the end of treatment was taken as the reference of cell proliferation on that day (recorded as 1), and the relative proliferation proportion of cells in the RAG1-40-53-C1 treatment group compared with the control group was calculated.

As shown in Fig. 11, as the concentration of saRNA (RAG1-40-53-C1) increased, the relative proliferation proportion of cells gradually decreased and stabilized at a certain level. After treatment with saRNA (RAG1-40-53-C1) at a concentration of 0.3 μM, the relative proliferation proportion of cells was about 50%. After treatment with saRNA (RAG1-40-53-C1) at a concentration of 1 μM, the relative proliferation proportion of cells basically reached a plateau and maintained at about 50%. This indicates that cholesterol modified saRNA (RAG1-40-53-C1) can be self-delivered to ARPE-19 cells and inhibit the growth and proliferation of the cells.

## EXAMPLE 10. PHARMACOKINETICS STUDY IN A RABBIT MODEL

(1) Animal grouping

Fifteen female New Zealand white rabbits were ordered from Pizhou Dongfang Breeding Co., Ltd., Jiangsu Province, and were raised in a common rabbit house, single cage, and free to eat and drink. Fifteen female rabbits were randomly divided into three groups, one in the normal saline control group and seven in each administration group.

(2) Formulation of drugs

saRNA (RAG1-40-53-C1) lyophilized powder was centrifuged at 13000 rpm for 5 min at room temperature, re-dissolved in PBS to 60 mg/ml, and diluted with PBS to the desired concentration.

(3) Administration by intravitreal injection

The weight of the rabbit was weighed and fixed with a rabbit box. 3% sodium pentobarbital solution was injected intravenously into the ear margin at the dose of 30 mg/kg for anesthesia. 1-2 drops of compound tropicamide were dripped into the eye for mydriasis (for about 15 minutes), and 2-3 drops of lidocaine were dripped at intervals of 1-2 minutes for local anesthesia. After that, 0.05 ml aqueous humor was drawn from the anterior chamber with a 30 G syringe, 0.05 ml of saRNA (RAG1-40-53-C1) solution at corresponding concentrations (0.1 mg and 0.3 mg) was injected into the vitreous of each eye. Samples were taken at 4 hours, 1, 2, 4, 8, 14, and 21 days, respectively, and the amounts of saRNA in rabbit vitreous and retina were detected. The day of the first injection was recorded as Day 0.

(4) Stem-loop PCR

The vitreous bodies of rabbit eyes treated at different time points were extracted and placed in a 1.5 ml centrifuge tube, into which 500 μl of Buffer-2 (Ractigen) was added, fully mixed and dissolved, and centrifuged at 3000 rpm for 3 minutes. After centrifugation, the sample was heated at 95 °C for 5 minutes and quickly transferred to ice for standby. The reaction system and conditions of RT-PCR are shown in Table 6, and the primer sequences are shown in Table 1.

**TABLE 6 PREPARATION OF RT-PCR REACTION**

| Reagent | Volume (μL) |
|---|---|
| 5×PrimeScript Buffer | 2.0 |
| PrimeScript RT enzyme mix 1 | 0.5 |
| SL RT primers | 0.5 |
| Lysis solution | 1.0 |
| RNase-free dH2O | 6.0 |
| Final colume | 10.0 |
| 16°C 30 min, 42°C 30 min, 85°C 5 min, stored at 4°C. | |

Reaction conditions: 95 °C for 30 seconds, 95 °C for 5 seconds, 60 °C for 20 seconds, 40 cycles of amplification, 72 °C for 10 seconds.

As shown in Fig. 12A, the amount of saRNA (RAG1-40-53-C1) in vitreous and retina gradually decreased over time after administration. 4 hours after administration, the amount of saRNA (RAG1-40-53-C1) in vitreous and retina decreased by more than 50%. 14 days after administration, saRNA (RAG1-40-53-C1) was almost completely metabolized. Fig. 12B shows the amount of saRNA (RAG1-40-53-C1) in the whole rabbit eye. 4 hours after administration, the amount of saRNA (RAG1-40-53-C1, 0.1 mg) was about 81%. 1 day after administration, the amount of saRNA sharply decreased, and the amount of saRNA was about 23% 2 days after administration. The saRNA was almost completely metabolized 14 days after administration, indicating that the low dose of saRNA (RAG1-40-53-C1, 0.1 mg) given to rabbit eyes can maintain a certain drug level in a short time, and the amount of saRNA was gradually metabolized after 2 days. After treatment of rabbit eyes with high dose of saRNA (RAG1-40-53-C1, 0.3 mg) for 4 hours, the amount of saRNA was about 57%, and the amount of saRNA was about 21% 2 days after administration. The saRNA was almost completely metabolized 14 days after administration, indicating that the metabolism of the high dose of drug in rabbit eyes was faster and the maintenance was short.

## EXAMPLE 11. PHARMACODYNAMIC STUDY IN A RABBIT MODEL

(1) Animal grouping

19 female New Zealand white rabbits were ordered and raised in a common rabbit room, single cage, free to eat and drink. 19 female rabbits were randomly divided into eight groups, with 1-4 rabbits in each group. The drug preparation was performed as described in Example 10.

(2) Administration by intravitreal injection

The weight of the rabbit was weighed and fixed with a rabbit box. 3% sodium pentobarbital solution was injected intravenously into the ear margin at the dose of 30 mg/kg for anesthesia. 1-2 drops of compound tropicamide were dripped into the eye for mydriasis (about 15 minutes), and 2-3 drops of lidocaine were dripped at intervals of 1-2 minutes for local anesthesia. After that, 0.05 ml aqueous humor was drawn from the anterior chamber with a 30 F syringe, each eye was injected with 0.1 ml of RAG1-40-53-C1 solution of different concentrations (the total amount of saRNA injected in each group was 0.03mg, 0.1 mg, 0.24mg, 0.3 mg, 1mg, respectively) or 0.1ml of control RAG1-40-53-Scr-C1 solution of different concentrations (as a scramble sequence control, the total amount of RAG1-40-53-Scr-C1 injected in each group was 0.3mg, 1mg, respectively).

(3) Two-step RT-qPCR

3 days after administration, the rabbit eyes were harvested, the eyeballs were enucleated, the cornea was cut, the lens was stripped, the vitreous was collected as much as possible and transferred to a 1.5 ml centrifuge tube, centrifuged at 1000 rpm for 5 minutes, and the supernatant was discarded. The vitreous body precipitated about 200 μL, and then added 1 ml of Trizol for shaking and mixing, and left at room temperature for 5 minutes. RNA was extracted with Qiagen RNeasy kit, reverse transcribed and analyzed by qPCR in ABI 7500 Fast Real-time PCR system (Applied Biosystems). PCR reaction conditions: 95 °C for 30 seconds, 95 °C for 5 seconds, 60 °C for 30 seconds, 40 cycles of amplification. TBP and B2M genes were simultaneously amplified as internal references, and p21 was amplified with

p21 F1/R1 primer pair. See Table 1 for primers.

To calculate the expression level (Erel) of p21 (target gene) of a saRNA (such as RAG1-40-53-C1) sample relative to the control treatment (normal saline), the Ct values of the target gene and two internal reference genes were substituted into formula 2.

$$E_{rel} = 2^{(CtTm-CtTs)} / ((2^{(CtR1m-CtR1s)} * 2^{(CtR2m-CtR2s)})^{(1/2)}) \quad \text{(Formula 2)}$$

Where, CtTm is the Ct value of the target gene from the control treated (normal saline) sample; CtTs is the Ct value of the target gene from the saRNA (RAG1-40-53-C1) treated sample; CtR1m is the Ct value of the internal reference gene 1 from the control treated sample; CtR1s is the Ct value of the internal reference gene 1 from the saRNA treated sample; CtR2m is the Ct value of the internal reference gene 2 from the control treated sample; and CtR2s is the Ct value of the internal reference gene 2 from the saRNA treated sample.

Fig. 13 shows the expression levels of p21 gene mRNA under different treatments. In comparison with the control group (normal saline), the expression of p21 mRNA in the two RAG1-40-53-Scr-C1 groups (0.3 and 1 mg) did not increase, indicating that RAG1-40-53-Scr-C1 as a scramble sequence control of saRNA (RAG1-40-53-C1) had no effect on p21 expression. In comparison with the control group (normal saline), the expression of p21 mRNA of RAG1-40-53-C1 at concentrations of 0.03, 0.1, 0.24, 0.3, and 1 mg increased by 1.3-, 2.2-, 2-, 2.2-, and 2-fold, respectively, and its expression was the highest at the concentration of 0.1 mg, indicating that saRNA can activate the expression of p21 mRNA at low doses.

## EXAMPLE 12. P21 ACTIVATING SARNA CAN SLOW DOWN THE OCCURRENCE OF PVR IN ANIMAL MODELS.

(1) Construction of rabbit PVR model

Five female New Zealand white rabbits were ordered and raised in a common rabbit house, single cage with free feeding and drinking. Five female rabbits were randomly divided into three groups, with 2-3 rabbits in each group. The vitreous injection method was performed as described in Example 10, 0.1 ml of a suspension containing $2.5\times10^5$ ARPE-19 cells was injected into rabbit vitreous for modeling. After modeling, the success of PVR model was observed by fundus microscope and slit lamp microscope.

(2) Administration by intravitreal injection

After the PVR model was successfully constructed, a single vitreous injection was administered, which was divided into the following two groups: normal saline group and RAG1-40-53-C1 group. The vitreous administration method was performed as described in Example 11.

*(3) In vivo* microscopy and *ex vivo* observation scoring

The change in PVR grade was observed by fundus microscope and slit lamp microscope before administration (33 days after cell injection) and 13 and 27 days after administration. The change in PVR grade was observed *ex vivo* 28 days after administration. The grades were divided according to Fastenberg grade (Fastenberg et al. 1982), with Grade 0 representing normal, Grades 1-5 representing the severity of PVR, and Grade 5 being the most serious. See Table 7 for details. After drug treatment, the *in vivo* microscopic examination and *ex vivo* observation scoring results are shown in Table 8.

**TABLE 7 FASTENBERG GRADE SCALE**

| Fastenberg grade | |
|---|---|
| Grade 0 | No obvious lesion |
| Grade 1 | Presence and visible anterior retinal membrane |
| Grade 2 | Anterior retinal membrane with focal retinal traction |
| Grade 3 | Local medullary ray detachment |
| Grade 4 | Extensive retinal detachment (complete detachment around medullary rays and retinal papilla) |

(continued)

| Fastenberg grade | |
|---|---|
| Grade 5 | Complete retinal detachment with fixed retinal folds |

**TABLE 8 FASTENBERG GRADING RESULTS OF IN VIVO MICROSCOPY AND EX VIVO OBSERVATION SCORING**

| Group | Animal ID | Fastenberg grade | | | |
|---|---|---|---|---|---|
| | | fundus microscope and slit lamp microscope | | | *ex vivo* observation |
| | | Before administration (33 days after cell injection) | 13 days after administration | 27 days after administration | 28 days after administration |
| Normal saline | #2 | 1 | 2 | 3 | 2 |
| | #3 | 2 | 3 | 4 | 3 |
| RAG1-40-53-C1 | #6 | 1 | 2 | 2 | 1 |
| | #1 | 2 | 2 | 1 | 1 |
| | #5 | 2 | 2 | 2 | 1 |

As mentioned above, when the PVR model was successfully constructed, the subjects were randomized and given drug (RAG1-40-53-C1) treatment. The PVR grade evaluated by fundus and slit lamp images before administration was 1 or 2, which showed mild change in PVR. The therapeutic effects of normal saline group and RAG1-40-53-C1 on rabbit eyes were observed 13 days and 28 days after administration. 13 days after administration and treatment, the PVR grade of the normal saline (control) group evaluated by fundus and slit lamp images was 2 or 3, and that of the RAG1-40-53-C1 treatment group was 2. 27 days after administration and treatment, the PVR grade of the normal saline treatment group evaluated by fundus and slit lamp images was 3 or 4, and that of the RAG1-40-53-C1 treatment group was 1 or 2. 28 days after administration, the rabbit eyeballs were removed to further observe the therapeutic effect of RAG1-40-53-C1 on PVR under direct vision. *Ex vivo* observation showed that the PVR grade of normal saline group was 2 or 3, and that of RAG1-40-53-C1 treatment group was 1. The above results suggest that RAG1-40-53-C1 treatment can slow down or inhibit the occurrence and development of PVR compared with saline (control) group, and long-term treatment is expected to improve the symptoms of PVR.

## EXAMPLE 13. SARNA PROMOTES P21 MRNA EXPRESSION IN HUMAN BLADDER CANCER CELLS

Human bladder cancer cells (Ku-7-LG) (ATCC) were cultured in McCoy medium (Gibco) containing 10% calf serum (Sigma-Aldrich) and 1% penicillin/streptomycin (Gibco). Cells were cultured in 5% $CO_2$ at 37 °C. Ku-7-LG cells were plated at $10 \times 10^4$ cells/well into 12-well plates, and saRNA was transfected at a concentration of 10 nM (unless otherwise specified) using RNAiMax according to the manufacturer's instructions for 72 hours, using 2 duplicate wells for each treatment. Two step RT-qPCR was performed as described in Example 2.

The relative p21 mRNA levels in Ku-7-LG cells after treatment with different saRNAs are shown in Fig. 14, wherein the blank transfection control was used as the control group. In comparison with the control group, the relative expression levels of mRNA in RAG-332, RAG-331, RAG-330, RAG-329, RAG-327, RAG-325, RAG-323, RAG-322, RAG-321, RAG-299 and RAG-297 groups increased by 1.5-, 3.8-, 1.7-, 1.6-, 1.2-, 1.3-, 2.0-, 6.4-, 5.6-, 1.6- and 1.4-fold, respectively, and the activation effect of RAG-331, RAG-322 and RAG-321 was particularly significant. This indicates that randomly selected functional saRNAs could promote the expression of p21 mRNA in Ku-7-LG9 cells to varying degrees.

## EXAMPLE 14. CHEMICALLY MODIFIED SARNA PROMOTES THE EXPRESSION OF P21 MRNA AND PROTEIN IN RETINAL PIGMENT EPITHELIAL CELLS

To increase the stability and activity of saRNA, all nucleotides of saRNA RAG-322 (also known as RAG1-0) were chemically modified, including the introduction of 2'-O-methyl modification at specific sites, 2'-fluoro modification

and phosphorothioate bond modification between nucleotides to obtain saRNA RAG1-0M4 (Table 4). To further improve the delivery efficiency of saRNA RAG1-0M4, a lipid group was conjugated at the 3' end of the oligonucleotide (oligo) sense strand through a solid-phase support CPG (controllable microporous glass) to obtain saRNA RAG1-0M4-C3 (3') (Table 4). The structure of the conjugated lipid group was as follows:

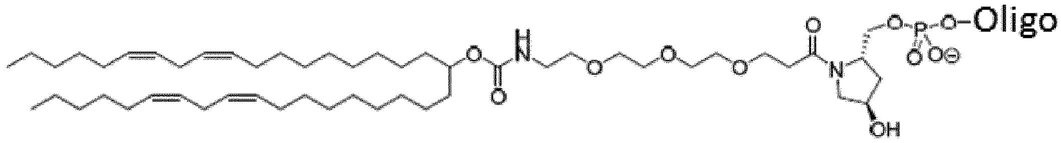

Cell culture was performed as described in Example 2. ARPE-19 cells were plated at 10 $\times$ $10^4$ cells/well into 12-well plates. Small activating RNAs were transfected using RNAiMax at a final concentration of 25 nm for 1, 2, 3, 4, 5, 6, and 7 days, respectively. Two duplicate wells were set for each treatment. Two step RT-qPCR was performed as described in Example 2. Protein collection and detection were performed as described in Example 5.

Fig. 15A shows the expression level of p21 mRNA in ARPE-19 cells treated with saRNA RAG1-0M4 for different days. The blank transfection control was used as a control group. In comparison with the control group, the expression level of p21 mRNA increased by 1.2-, 2.0-, 1.9-, 1.7-, 1.9- and 2.0-fold after treatment with saRNA RAG1-0M4 for 2, 3, 4, 5, 6 and 7 days, respectively. The expression of p21 mRNA started to increase (1.2-fold) 2 days after RAG1-0M4 treatment, peaked (2.0-fold) 3 days after the treatment, began to decrease (still high activity) 4 days after the treatment, started to rise again 6 days after the treatment and peaked again 7 days after the treatment. This indicates that the functional saRNA RAG1-0M4 can promote the expression of p21 mRNA 3 days after treatment in ARPE-19 cells and the maintenance of a high expression level.

Figs. 15B and 15C show the expression levels of p21 protein in ARPE-19 cells treated with saRNA RAG1-0M4 for different days. Fig. 15B shows the protein band diagram, and Fig. 15C shows the statistical diagram obtained by analyzing the protein band according to Fig. 15B using Image J software. The blank transfection control was used as a control group. In comparison with the control group, the p21 protein expression level increased by 3.9-, 8.0-, 11.2-, 8.7-, 5.5-, 4.4- and 2.8-fold after treatment with saRNA RAG1-0M4 for 1, 2, 3, 4, 5, 6 and 7 days, respectively. The p21 protein expression began to increase (3.9-fold) 1 day after of RAG1-0M4 treatment, peaked (11.2-fold) 3 days after the treatment, and gradually decreased over time 4 days after the treatment. This indicates that the functional saRNA RAG1-0M4 can promote p21 protein expression and reach the peak 3 days after treatment in ARPE-19 cells.

The results showed that saRNA RAG1-0M4 treatment for different days could promote the expression of p21 mRNA and protein to different degrees in ARPE-19 cells, and the activity of saRNA RAG1-0M4 was the best 3 days after the treatment.

## EXAMPLE 15. CHEMICALLY MODIFIED SARNA PROMOTES THE EXPRESSION OF P21 MRNA AND PROTEIN IN RETINAL PIGMENT EPITHELIAL CELLS

Cell culture and transfection

Cell culture was performed as described in Example 2. ARPE-19 cells were plated at 10 $\times$ $10^4$ cells/well into 12-well plates. The highest transfection concentration of saRNA RAG1-0M4 was 100 nM, which was used for half dilution (50, 25, 12.5, 6.25, 3.13, 1.56, 0.78, 0.39, 0.2, and 0.1 nM) and transfected with RNAiMax for 3 days. Two duplicate wells were set for each treatment. Two step RT-qPCR was performed as described in Example 2. Protein collection and detection were performed as described in Example 5, and chemical modification of saRNA RAG1-0M4 was performed as described in Example 14.

Fig. 16A shows the expression levels of p21 mRNA in ARPE-19 cells treated with different doses of saRNA. The blank transfection control was used as a control group. In comparison with the control group, the p21 mRNA expression of saRNA RAG1-0M4 increased by 2.2-, 2.4-, 2.1-, 2.5-, 2.8-, 2.1-, 1.7- and 1.4-fold after treatment with various concentrations of 100, 50, 25, 12.5, 6.25, 3.13, 1.56 and 0.78 nM for 3 days, respectively. The expression of p21 mRNA by saRNA RAG1-0M4 began to rise (1.4-fold) at 0.78 nM, peaked (2.8-fold) at 6.25 nM, and then began to decline, and maintained at a stable level (2.0-fold) at 25-100 nM. This indicated that different doses of functional saRNA RAG1-0M4 could promote the expression of p21mRNA in ARPE-19 cells, and reach the peak at the transfection concentration of 6.25 nM.

Figs. 16B and 16C show the expression levels of p21 protein in ARPE-19 cells treated with different doses of saRNA RAG1-0M4. Fig. 16B shows the protein band diagram, and Fig. 16C shows the statistical diagram obtained by analyzing the protein band according to Fig. 16B using Image J software. The blank transfection control was used as a control group. In comparison with the control group, the p21 protein expression of saRNA RAG1-0M4 increased by 2.8-,

4.7-, 4.5-, 4.1-, 2.2-, 2.2-, 1.8-, 1.7-, 1.6-, and 1.2-fold after treatment for 3 days with various concentrations of 100, 50, 25, 12.5, 6.25, 3.13, 1.56, 0.78, 0.39, 0.2 and 0.1 nM, respectively. This indicates that the p21 protein expression induced by different doses of functional saRNA RAG1-0M4 in the range of 6.25-50 nM was high and stable, and the p21 protein expression reached the peak at 12.5 nM.

The results showed that different doses of saRNA RAG1-0M4 can promote the expression of p21 mRNA and protein to different degrees in ARPE-19 cells, and the activity of saRNA RAG1-0M4 was the best at 6.25-12.5 nM.

## EXAMPLE 16. PHARMACOKINETICS OF CHEMICALLY MODIFIED SARNA IN A RABBIT MODEL

In Example 10, four female New Zealand white rabbits from the same source were raised in a common rabbit house, single cage, and were free to eat and drink.

saRNA RAG1-0M4-C3 (3') lyophilized powder was centrifuged at 13000 rpm for 5 min at room temperature, re-dissolved in PBS to 60 mg/ml, and diluted to 2 mg/ml (0.1 mg) and 6 mg/ml (0.3 mg) with PBS.

The weight of the rabbit was weighed and fixed with a rabbit box. 3% sodium pentobarbital solution was injected intravenously into the ear margin at the dose of 30 mg/kg for anesthesia. 1-2 drops of compound tropicamide were dripped into the eye for mydriasis (about 15 minutes), and 2-3 drops of lidocaine were dripped at intervals of 1-2 minutes for local anesthesia. After that, 0.05 ml aqueous humor was drawn from the anterior chamber with a 30 G syringe, a single injection of 0.05 ml of saRNA RAG1-0M4-C3 (3') solutions at corresponding concentrations (0.1 mg and 0.3 mg) was given to the vitreous of each eye. Samples were taken at 4 hours, 1, 3, 7 and 14 days, respectively, and the amount of saRNA RAG1-0M4-C3 (3') in rabbit vitreous, plasma and retina was detected. The day of the first injection was recorded as Day 0.

Stem-loop PCR was used to detect the amount of saRNA RAG1-0M4-C3 (3') in rabbit vitreous, plasma and retina at different time points. The stem-loop PCR method was performed as described in Example 10.

Fig. 17A shows the amount of the drug in rabbit vitreous, plasma and retina at different time points after one-time vitreous injection of saRNA RAG1-0M4-C3 (3') (0.1 mg). The drug was quickly absorbed by the retina after intravitreal injection, resulting in the highest concentration of the drug in the retina, followed by the vitreous, and the lowest concentration in the plasma, indicating that the systemic exposure of saRNA RAG1-0M4-C3 (3') after intravitreal injection is largely limited. In the retina and vitreous, the concentration of RAG1-0M4-C3 (3') began to decrease 1 day after administration, and maintained at a stable level after 3 days until Day 14, indicating that saRNA RAG1-0M4-C3 (3') has good stability and slow metabolism in rabbit eyes.

Fig. 17B shows the amount of saRNA RAG1-0M4-C3 (3') (0.3 mg) in the vitreous, plasma and retina of rabbits at different time points after one-time vitreous injection. The drug was quickly absorbed by the retina after intravitreal injection, resulting in the highest concentration of the drug in the retina, followed by the vitreous, and the lowest concentration in the plasma, indicating that the systemic exposure of saRNA RAG1-0M4-C3 (3') after intravitreal injection is highly limited. The drug concentration in retina was maintained at a stable level after administration until Day 14, while in vitreous, the concentration of RAG1-0M4-C3 (3') began to decline 1 day after administration, and fell below the concentration in plasma by Day 14. In the plasma, the drug concentration showed a slow upward trend from Day 3 to Day 14, reflecting the transfer of the drug from the rabbit eye to the whole body. These results provide data support for us in the arrangement of dose and administration time.

## EXAMPLE 17. PHARMACODYNAMIC STUDY OF CHEMICALLY MODIFIED SARNA IN A RABBIT MODEL

Nine female New Zealand white rabbits from the same source as Example 10 were raised in a common rabbit house, single cage, and free to eat and drink. They were randomly divided into 4 groups with 2-3 rabbits in each group. The drug preparation is performed as described in Example 16.

The saRNA RAG1-0M4-C3 (3') lyophilized powder was centrifuged at 13000 rpm for 5 min at room temperature, redissolved in PBS to 6 mg/ml, and the 0.3mg/0.05 mL concentration of saRNA RAG1-0M4-C3 (3') was mixed with 2 × 10^6 ARPE-19 cells/0.05 ml *in vitro* to a final volume of 0.1 ml for injection into rabbit vitreous.

The administration method of vitreous injection was performed as described in Example 16. The experimental group received a single vitreous injection of 0.1 ml solution of saRNA RAG1-0M4-C3 (3') at a concentration of 0.3 mg. The saline group received a single simultaneous injection of the same volume of saline as the control group. The p21 mRNA amount of rabbit vitreous was detected on Days 3 and 7, and the day of the first injection was recorded as Day 0. The sample sampling and processing method and the two-step RT-qPCR method were performed as described in Example 11.

Fig. 18 shows the expression of p21 mRNA of saRNA RAG1-0M4-C3 (3') in rabbit vitreous. Normal saline was used as a control group. In comparison with the control group, the expression of p21 mRNA of saRNA RAG1-0M4-C3 (3') increased by 4.0 and 5.3-fold on Day 3 and 7, respectively, and the highest expression was on Day 7, which indicated that saRNA RAG1-0M4-C3 (3') can promote the expression of p21 mRNA in rabbit vitreous in a short time.

## EXAMPLE 18. CHEMICALLY MODIFIED SARNA PROMOTES P21 MRNA EXPRESSION IN RETINAL PIGMENT EPITHELIAL CELLS

To increase the stability and activity of saRNA, 5'-(E)-vinyl phosphonate was added to saRNA RAG1-0M4 for chemical modification to obtain saRNA RAG1-0M4v (Table 4). To further improve the delivery efficiency of saRNA RAG1-0M4v, a lipid structural group was conjugated at the 3' end of its sense strand by solid-phase support CPG (controllable microporous glass), and the saRNA RAG1-0M4v-C3 (3') with a delivery structure was obtained (Table 4). The conjugated lipid structural group was the same as in Example 14.

Cell culture was performed as described in Example 2. ARPE-19 cells were plated at $10 \times 10^4$ cells/well into 12-well plates. Small activating RNAs were transfected using RNAiMax at final concentrations of 3, 10, and 30 nM for 3 days. Two duplicate wells were set for each treatment. Two step RT-qPCR was performed as described in Example 2.

Fig. 19 shows the expression of p21 mRNA in ARPE-19 cells transfected with different concentrations of saRNA RAG1-0M4v. The blank transfection control was used as a control group. In comparison with the control group, the expression of p21 mRNA of saRNA RAG1-0M4v at 3 nM, 10 nM and 30 nM increased by 2.0, 2.3 and 2.2-fold, respectively. This indicates that the functional saRNA RAG1-0M4v could induce the expression of p21 mRNA at a lower concentration.

## EXAMPLE 19. THE EFFECT OF CHEMICALLY MODIFIED SARNA ON INHIBITING THE GROWTH OF RETINAL PIGMENT EPITHELIAL CELLS IS NOT CAUSED BY APOPTOSIS

After cell transfection, 100 μl of cell culture medium was pipetted and transferred to Caspase-3/7 (Catalog no: Promega G8092, company: Promega) special whiteboard for experiment. 15 μl of Caspase-3/7 reaction substrate was added into 100 μl of cell culture medium and incubated in a cell incubator for 20 minutes. After incubation, a microplate reader was used to detect the fluorescence intensity. The fluorescence intensity reflects the Caspase-3/7 activity of cells, which is an indicator of apoptosis, wherein apoptosis was determined by detecting its activity. Chemical modification, cell culture and transfection were performed as described in Example 18, and CCK8 experiments were performed as described in Example 9.

Fig. 20A shows the number of ARPE-19 cells after treatment with different transfection concentrations of saRNA RAG1-0M4v for 3 days. The blank transfection control was used as a control group. In comparison with the control group, the relative proliferation proportion of saRNA RAG1-0M4v at 3 nM, 10 nM and 30 nM was 57%, 54% and 34%, respectively. Fig. 20B shows the activity of Caspase-3/7 in ARPE-19 cells treated with different transfection concentrations of saRNA RAG1-0M4v for 3 days. In comparison with the control group, there was no significant difference in Caspase-3/7 activity for cells treated with saRNA RAG1-0M4v cells at 3 nM, 10 nM and 30 nM. This indicates that saRNA RAG1-0M4v can inhibit the growth of the cells as the dose increases, but this inhibitory effect on cell growth is not caused by apoptosis.

## EXAMPLE 20. CHEMICALLY MODIFIED SARNA PROMOTES THE EXPRESSION OF P21 MRNA BUT NOT APOPTOSIS IN RETINAL PIGMENT EPITHELIAL CELLS

Cell culture was performed as described in Example 2. ARPE-19 cells were plated at $10 \times 10^4$ cells/well into 12-well plates. saRNAs RAG1-0, RAG1-0M4v, and RAG1-0M4v-C3 (3') were used at a transfection concentration of 10 nM with RNAiMax for 3 days, using 2 duplicate wells for each treatment. The two-step RT-qPCR was performed as described in Example 2, and the chemical modification of saRNA RAG1-0M4v and RAG1-0M4v-C3 (3') was performed as described in Example 18. Caspase-3/7 activity experiments were performed as described in Example 19.

Fig. 21A shows the expression of p21 mRNA in ARPE-19 cells after treatment with 10 nM of different saRNAs for 3 days. The blank transfection control was used as a control group. In comparison with the control group, the expression of p21 mRNA in ARPE-19 cells treated with saRNAs RAG1-0, RAG1-0M4v and RAG1-0M4v-C3 (3') increased by 4.2, 4.0 and 2.9-fold, respectively. This indicates that the saRNA RAG1-0M4v-C3 (3') remains active in activating the p21 gene after chemical modification. Fig. 21B shows the activity of Caspase-3/7 in ARPE-19 cells after treatment with different saRNAs at 10 nM for 3 days. The blank transfection control was used as a control group. In comparison with the control group, the activities of Caspase-3/7 in ARPE-19 cells treated with saRNA RAG1-0, RAG1-0M4v and RAG1-0M4v-C3 (3') were 1.3-, 1.3- and 1.1-fold, respectively. The activity of Caspase-3/7 is an indicator of apoptosis, wherein a stronger activity indicates more serious apoptosis. This indicates that the activity of promoting apoptosis of saRNA RAG1-0M4v-C3 (3') is reduced after chemical modification.

The results showe that the chemically modified saRNA RAG1-0M4v-C3 (3') not only promotes the expression of p21 mRNA in ARPE-19 cells, but also reduces the risk of apoptosis.

## EXAMPLE 21. CHEMICALLY MODIFIED SARNA PROMOTES THE EXPRESSION OF P21 MRNA AND PROTEIN IN RETINAL PIGMENT EPITHELIAL CELLS

Cell culture was performed as described in Example 2. ARPE-19 cells were plated at $10 \times 10^4$ cells/well into 12-well plates. saRNA RAG1-0M4v-C3 (3') was used with 100 nM as the highest transfection concentration, diluted three times (33.3, 11.1, 3.7, 1.2, 0.412, 0.137, 0.046, 0.015, 0.005, and 0.002 nM) therefrom and subjected to transfection with RNAiMax for 3 days. Two duplicate wells were set for each treatment. The two-step RT-qPCR was performed as described in Example 2. The protein collection and detection were performed as described in Example 5, and the chemical modification of saRNA RAG1-0M4v-C3 (3') was performed as described in Example 18.

Fig. 22A shows the expression of p21 mRNA in ARPE-19 cells treated with different doses of saRNA RAG1-0M4v-C3 (3'). The blank transfection control was used as a control group. In comparison with the control group, the expression of p21 mRNA increased by 1.9-, 1.9-, 1.7-, 1.5- and 1.4-fold after treatment with saRNA RAG1-0M4v-C3 (3') at various concentrations of 100, 33.3, 11.1, 3.7 and 1.2 nM for 3 days, respectively. The expression of p21 mRNA of saRNA RAG1-0M4v-C3 (3') started to rise (1.4-fold) for treatment at 1.2 nM, peaked (1.9-fold) for treatment at 33.3 nM, and maintained at a stable level (1.9-fold) for treatment at 33.3-100 nM. This indicated that different doses of functional saRNA RAG1-0M4v-C3 (3') can promote the expression of p21 mRNA in ARPE-19 cells, and reach the peak at the transfection concentration of 33.3 nM.

Figs. 22B and 22C show the expression levels of p21 protein in ARPE-19 cells treated with different doses of saRNA RAG1-0M4v-C3 (3') and saRNA RAG1-0M4v. Fig. 22B is the protein band graph, and Fig. 22C is the statistical graph of band brightness obtained by using Image J software according to the protein band of Fig. 21B. The blank transfection control was used as a control group. In comparison with the control group, the p21 protein expression of saRNA RAG1-0M4v-C3 (3') at 3, 10 and 30 nM increased by 4.5-, 5.3- and 4.8-fold, respectively. For treatment at the same dose (10 nM), the p21 protein expression of saRNA RAG1-0M4v-C3 (3') was higher than that of saRNA RAG1-0M4v-C3 (4.0-fold). This indicates that the saRNA RAG1-0M4v-C3 (3') with a delivery structure can not only improve the p21 protein expression, but also produce a better p21 protein expression than the saRNA RAG1-0M4v without a delivery structure.

The results showed that saRNA RAG1-0M4v-C3 (3') with a delivery structure can promote the expression of p21 mRNA and protein in ARPE-19 cells to varying degrees, and the so-induced expression of p21 protein was better than that of saRNA RAG1-0M4v without a delivery structure.

## EXAMPLE 22. CHEMICALLY MODIFIED SARNA REDUCES THE IMMUNOSTIMULATORY RESPONSE OF INFLAMMATORY FACTORS IN PBMC CELLS

Peripheral blood mononuclear cells (PBMCs, Jiangsu Sidier Biotechnology Co., Ltd.) were cultured in RPMI1640 medium (Gibco) containing 10% calf serum (Sigma-Aldrich) and 1% penicillin/streptomycin (Gibco). Cells were cultured in 5% $CO_2$ at 37 °C. PBMC cells were plated at $100 \times 10^4$ cells/well into 96-well plates, and the saRNAs RAG1-0, RAG1-0M4v, and RAG1-0M4v-C3 (3') were used with RNAiMax at a transfection concentration of 133 nM for 24 hours, using 2 duplicate wells for each treatment. LPS (50 ng/ml) and RAG-IS-1 were used as a positive control group, and the blank transfection control was used as a control group. Untreated means that no transfection reagent was added, and only PBMC cells and culture medium were used.

saRNA RAG1-0M4v and RAG1-0M4v-C3 (3') were chemically modified as described in Example 18.

After the completion of cell transfection, three portions of 100 μL cell suspension for each samples were collected ($3 \times 100$ μL) for the detection of the amounts of inflammatory factors human IL-6, IFN-α and TNF-α, specific operation methods were performed according to the instructions of kits for each inflammatory factor. The specific kit information is as follows: IL-6 ELISA kit (Catalog no: 1110602 company: Shanghai Dakewe Biotechnology Co., Ltd.), IFN-α ELISA kit (Catalog no: 70-EK199-96, company: Hangzhou Lianke Biotechnology Co., Ltd.) and TNF-α ELISA kit (Catalog no: 1117202, company: Shanghai Dakewe Biotechnology Co., Ltd.).

Fig. 23A shows the amounts of human IL-6 in PBMC cells treated with saRNA RAG1-0M4v and RAG1-0M4v-C3 (3'). The amounts of human IL-6 in PBMC cells of untreated, control, dsCon2, LPS (50 ng/ml), RAG1-0M4v and RAG1-0M4v-C3 (3') groups were 3.6, 5.9, 15.5, 443.0, 3.4 and 1.8 pg/ml, respectively. The amount of IL-6 in saRNA RAG1-0M4v and RAG1-0M4v-C3 (3') groups was much lower than that in the control group, and the amount of human IL-6 in saRNA RAG1-0M4v-C3 (3') group was the lowest. This indicates that the saRNA RAG1-0M4v-C3 (3') modified by the delivery structure exhibits an inhibited stimulatory effect on IL-6 inflammatory factor compared with the saRNA without the delivery structure.

Fig. 23B shows the amount of human IFN-α in PBMC cells treated with saRNA RAG1-0M4v and RAG1-0M4v-C3 (3'). The amounts of human IFN-α in PBMC cells from untreated, control, dsCon2, RAG-IS-1, RAG1-0M4v, and RAG1-0M4v-C3 (3') groups were 9.7, 38.8, 27.8, 176.3, 23.5, and 20 pg/ml, respectively, wherein the amounts of human IFN-α in the saRNA RAG1-0M4v and RAG1-0M4v-C3 (3') groups were much lower than that in the control group. The

amount of human IFN-α in RAG1-0M4v-C3 (3') group was the lowest. This indicates that saRNA RAG1-0M4v-C3 (3') modified with a delivery structure exhibits an inhibited stimulatory effect on IFN-α inflammatory factor compared with saRNA without the delivery structure.

Fig. 23C shows the amounts of human TNF-α in PBMC cells treated with saRNA RAG1-0M4v and RAG1-0M4v-C3 (3'). The amounts of human IFN-α in PBMC cells from untreated, control, dsCon2, LPS (50 ng/mL), RAG1-0M4v and RAG1-0M4v-C3 (3') groups were 61.0, 134.5, 154.6, 330.3, 115.4, and 124.0 pg/ml, respectively. The amount of saRNA RAG1-0M4v and RAG1-0M4v-C3 (3') groups were much lower than that of the control group. This indicates that the modified saRNA RAG1-0M4v-C3 (3') exhibits an inhibited stimulatory effect on TNF-α inflammatory factor compared with saRNA without the delivery structure.

## EXAMPLE 23. PHARMACODYNAMIC STUDY OF CHEMICALLY MODIFIED SARNA IN A RABBIT MODEL

Nine female New Zealand white rabbits from the same source as Example 10 were raised in a common rabbit house, single cage, and free to eat and drink. They were randomly divided into 4 groups with 2-3 rats in each group.

The drug preparation was performed as described in Example 17, except that the saRNAs used were RAG1-0M4v and RAG1-0M4v-C3 (3'). The chemical modification of saRNAs RAG1-0M4v and RAG1-0M4v-C3 (3') was performed as described in Example 18.

The vitreous injection administration method was performed as described in Example 16. Each experimental group received a single vitreous injection of 0.1 ml solution of saRNA RAG1-0M4v and RAG1-0M4v-C3 (3') at a concentration of 0.3 mg, or 0.1 ml of dsCon2M4 group at a concentration of 0.3 mg (as the scramble sequence control group), and the same volume of normal saline was injected as the control group. The samples were taken 3 days later and the amount of p21 mRNA in rabbit vitreous was detected. The day of the first injection was recorded as Day 0. The sample sampling and processing method and the two-step RT-qPCR method were performed as described in Example 11.

Fig. 24 shows the expression of p21 mRNA in rabbit vitreous treated with saRNA RAG1-0M4v and RAG1-0M4v-C3 (3'). Normal saline was used as a control group. In comparison with the control group, the expression of p21 mRNA in rabbit vitreous treated with saRNAs RAG1-0M4v and RAG1-0M4v-C3 (3') increased by 0.9- and 3.5-fold, respectively. The expression of p21 mRNA of saRNA RAG1-0M4v-C3 (3') with delivery structure modification was higher in rabbit vitreous, which indicated that the saRNA RAG1-0M4v-C3 (3') modified with a delivery structure can promote the expression of p21 mRNA and further improve the efficiency of *in vivo* delivery.

To sum up, human p21 saRNAs can activate the expression of p21 gene and inhibit the growth of human retinal pigment epithelial cells, and further improve or treat diseases caused by the reduction or deficiency in p21, such as proliferative vitreoretinopathy.

The present application up-regulates the transcription and expression of p21 gene with the mechanism of small activating RNAs, which is verified in both *in vitro* and *in vivo* experiments. It was proved in ARPE-19 cells that increasing the expression of p21 can inhibit the proliferation and migration of the cells, indicating that up-regulating the expression of p21 can effectively inhibit the proliferation and migration of the cells. The rabbit PVR model was used in the *in vivo* experiment, and the symptoms of PVR were significantly improved after the treatment of the present application. The high selectivity, targeting and accuracy of this gene therapy method provide a new direction for the treatment of PVR.

Each of the examples of the present application has been described above. The above description is exemplary, not exhaustive, and is not limited to the disclosed examples. Without departing from the scope and spirit of the described examples, many modifications and changes are obvious to those skilled in the art. The selection of terms used in the present application is intended to best explain the principle, practice or technical improvement in the market of the examples, or to enable other skilled in the art to understand the examples disclosed in the present application.

References:

Audo, I., S. R. Darjatmoko, C. L. Schlamp, J. M. Lokken, M. J. Lindstrom, D. M. Albert, and R. W. Nickells. 2003. 'Vitamin D analogues increase p53, p21, and apoptosis in a xenograft model of human retinoblastoma', Invest Ophthalmol Vis Sci, 44: 4192-9.

Fillies, T., M. Woltering, B. Brandt, J. P. Van Diest, R. Werkmeister, U. Joos, and H. Buerger. 2007. 'Cell cycle regulating proteins p21 and p27 in prognosis of oral squamous cell carcinomas', Oncol Rep, 17: 355-9.

Heatley, G., J. Kiland, B. Faha, J. Seeman, C. L. Schlamp, D. G. Dawson, J. Gleiser, D. Maneval, P. L. Kaufman, and R. W. Nickells. 2004. 'Gene therapy using p21WAF-1/Cip-1 to modulate wound healing after glaucoma trabeculectomy surgery in a primate model of ocular hypertension', Gene Ther, 11: 949-55.

Kwon, O. W., J. H. Song, and M. I. Roh. 2016. 'Retinal Detachment and Proliferative Vitreoretinopathy', Dev Ophthalmol, 55: 154-62.

Mudhar, H. S. 2020. 'A brief review of the histopathology of proliferative vitreoretinopathy (PVR) ', Eye (Lond) , 34: 246-50.

Romanov, V. S., and K. L. Rudolph. 2016. 'p21 shapes cancer evolution', Nat Cell Biol, 18: 722-4.

Sadaka, A., and G. P. Giuliari. 2012. 'Proliferative vitreoretinopathy: current and emerging treatments', Clin Ophthalmol, 6: 1325-33.

Ueda, Y., S. Kanazawa, T. Kitaoka, Y. Dake, A. Ohira, A. M. Ouertani, and T. Amemiya. 2001. 'Immunohistochemical study of p53, p21 and PCNA in pterygium', Acta Histochem, 103: 159-65.

Zandi, S., I. B. Pfister, P. G. Traine, C. Tappeiner, A. Despont, R. Rieben, M. Skowronska, and J. G. Garweg. 2019. 'Biomarkers for PVR in rhegmatogenous retinal detachment', PloS one, 14: e0214674.

Fastenberg, D. M., K. R. Diddie, N. Sorgente, and S. J. Ryan. 1982. 'A comparison of different cellular inocula in an experimental model of massive periretinal proliferation', Am J Ophthalmol, 93: 559-64.

## Claims

1. A small activating nucleic acid molecule, comprising a first oligonucleotide strand and a second oligonucleotide strand that form a duplex structure with complete or incomplete complementation, the first oligonucleotide strand having at least 75% homology or complementarity with a segment of 16-35 consecutive nucleotides in SEQ ID NO:469; wherein the small activating nucleic acid molecule (1) has a GC content of between 35 and 65%; (2) does not contain 5 or more consecutive identical nucleotides; and (3) does not contain 3 or more duplicated or triplicated nucleotides.

2. The small activating nucleic acid molecule according to claim 1, wherein the first oligonucleotide strand has at least 75% homology or complementarity with any segment of 16-35 consecutive nucleotides in any sequence shown in SEQ ID NOs: 5, 6, 7, 8, 9, 10, 11, and 12.

3. The small activating nucleic acid molecule according to claim 2, wherein the first oligonucleotide strand has at least 75%, at least 80%, at least 85%, at least 90%, or at least 95% complementarity or homology with any sequence shown in SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412, 415-416, 419-424, 429 -432, 439-442, 447-450, 453-458, 463-468, and 479-486.

4. The small activating nucleic acid molecule according to claim 3, wherein the first oligonucleotide strand or the second oligonucleotide strand is identical to or complementary with, or has 1-2 different or mismatched bases with any nucleotide sequence of SEQ ID NOs: 13-30, 35-46, 59-62, 67-74, 77-80, 85-96, 103-108, 111-118, 121-132, 139-140, 147-180, 185-186, 189-190, 195-198, 201-202, 209-212, 215-218, 225-240, 243-246, 249-258, 261-262, 265-270, 275-280, 283-300, 303-308, 317-320, 323-324, 329-348, 351-352, 357-358, 361-366, 371-392, 399-400, 405-412 , 415-416, 419-424, 429-432, 439-442, 447-450, 453-458, 463-468, and 479-486.

5. The small activating nucleic acid molecule according to claim 1, wherein the small activating nucleic acid molecule is a double-stranded nucleic acid, and the first oligonucleotide strand and the second oligonucleotide strand are separately located on the two strands of the double-stranded nucleic acid.

6. The small activating nucleic acid molecule according to claim 5, wherein one or both of the first oligonucleotide strand and the second oligonucleotide strand have an overhang of 0-6 nucleotides, and the overhang is located at the 5' end or 3' end, or the 5' end and 3' end of the oligonucleotide strands.

7. The small activating nucleic acid molecule according to claim 6, wherein the overhang is dTdT or dTdTdT or identical to or complementary with nucleotide(s) at a corresponding position(s) of a target gene.

**8.** The small activating nucleic acid molecule according to claim 1, wherein the small activating nucleic acid molecule is a consecutive single-stranded nucleic acid, wherein the first oligonucleotide strand and the second oligonucleotide strand are located on the consecutive single-stranded nucleic acid and have a complementary region that can form a duplex structure, so that the single-stranded nucleic acid has a hairpin structure capable of forming a duplex region.

**9.** The small activating nucleic acid molecule according to claim 1, wherein the nucleotides constituting the small activating nucleic acid molecule are naturally occurring nucleotides without chemical modification.

**10.** The small activating nucleic acid molecule according to claim 1, wherein one or more nucleotides in the small activating nucleic acid molecule are chemically modified nucleotides.

**11.** The small activating nucleic acid molecule according to claim 10, wherein the chemical modification is selected from one or more of a group consisting of modification of phosphodiester bond of nucleotide, modification of 2'-OH of ribose in nucleotide, modification of base in nucleotide, inclusion of a locked nucleic acid, and vinyl phosphonate modification of 5' terminal nucleotide of the first oligonucleotide strand or the second oligonucleotide strand.

**12.** The small activating nucleic acid molecule according to claim 11, wherein the chemical modification is selected from one or more of a group consisting of phosphorothioate modification, boranophosphate modification, 2'-fluoro modification, 2'-O-methyl modification, 2'-oxyethylenemethoxy modification, 2,4'-dinitrophenol modification, locked nucleic acid modification, 2'-amino modification, 2'-deoxy modification, 5'-bromouracil modification, 5'-iodouracil modification, N-methyluracil modification and 2,6-diaminopurine modification.

**13.** The small activating nucleic acid molecule according to claim 10, wherein the small activating nucleic acid molecule is conjugated with one or more of chemical groups selected from a group consisting of lipids, fatty acids, fluorescent groups, ligands, sugars, high molecular compounds, polypeptides, antibodies, and cholesterol.

**14.** The small activating nucleic acid molecule according to claim 13, wherein the chemical group conjugated with the small activating nucleic acid molecule is selected from one or more of structures (A), (B), or (C):

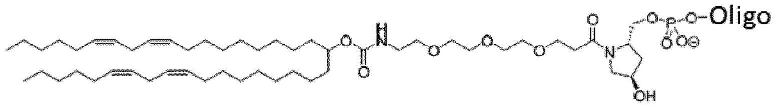

(A)

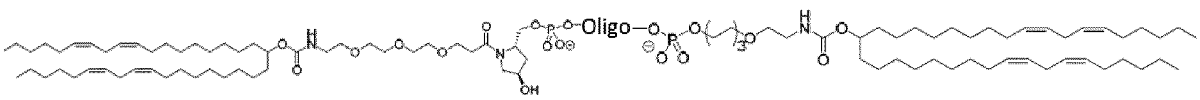

(B)

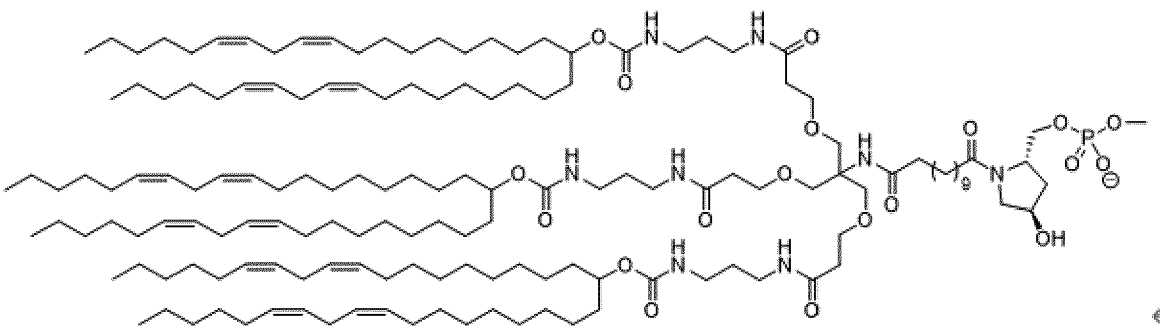

(C).

**15.** The small activating nucleic acid molecule according to claim 1, wherein the small activating nucleic acid molecule up-regulates the expression of p21 gene by at least 10%, at least 20% or at least 30%.

**16.** The small activating nucleic acid molecule according to any one of claims 1-4 or 10, wherein the first oligonucleotide strand and the second oligonucleotide strand are shown in the follows sequences:

(1) SEQ ID NO: 479 and SEQ ID NO: 480;
(2) SEQ ID NO: 481 and SEQ ID NO: 482;
(3) SEQ ID NO: 483 and SEQ ID NO: 484;
(4) SEQ ID NO: 485 and SEQ ID NO: 486;
(5) SEQ ID NO: 487 and SEQ ID NO: 488;
(6) SEQ ID NO: 489 and SEQ ID NO: 488;
(7) SEQ ID NO: 61 and SEQ ID NO: 62;
(8) SEQ ID NO: 496 and SEQ ID NO: 497;
(9) SEQ ID NO: 498 and SEQ ID NO: 499;
(10) SEQ ID NO: 498 and SEQ ID NO: 500;
(11) SEQ ID NO: 501 and SEQ ID NO: 499;
(12) SEQ ID NO: 501 and SEQ ID NO: 500;
(13) SEQ ID NO: 502 and SEQ ID NO: 503;
(14) SEQ ID NO: 504 and SEQ ID NO: 505;
(15) SEQ ID NO: 506 and SEQ ID NO: 507;
(16) SEQ ID NO: 508 and SEQ ID NO: 509; or
(17) SEQ ID NO: 510 and SEQ ID NO: 511.

**17.** A nucleic acid molecule, compirsing a segment encoding the small activating nucleic acid molecule according to any one of claims 1-4 and 16.

**18.** The nucleic acid molecule according to claim 17, wherein the nucleic acid molecule is an expression vector.

**19.** A cell, wherein the cell comprises the small activating nucleic acid molecule according to any one of claims 1-4 and 16 or the nucleic acid molecule according to claim 17 or 18.

**20.** A pharmaceutical composition, wherein the pharmaceutical composition comprises: the small activating nucleic acid molecule according to any one of claims 1-4 and 16, or the nucleic acid molecule according to claim 17 or 18, and optionally, a pharmaceutically acceptable carrier.

**21.** A preparation, wherein the preparation comprises the small activating nucleic acid molecule according to any one of claims 1-4 and 16, or the nucleic acid molecule according to claim 17 or 18, or the cell according to claim 19, or the pharmaceutical composition according to claim 20.

**22.** A kit, wherein the kit comprises the small activating nucleic acid molecule according to any one of claims 1-4 and 16, or athenucleic acid molecule according to claim 17 or 18, or the cell according to claim 19, or the pharmaceutical composition according to claim 20.

**23.** Use of the small activating nucleic acid molecule according to any one of claims 1-4 and 16, or the nucleic acid molecule according to claim 17 or 18, or the cell according to claim 19, or the pharmaceutical composition according to claim 20 in preparing drugs or preparations for activating or up-regulating expression of p21 gene in cells.

**24.** The use according to claim 23, wherein the use is for preparing drugs or preparations for treating or alleviating proliferative vitreoretinopathy.

**25.** A method for treating or alleviating proliferative vitreoretinopathy, comprising administering to an individual in need thereof the small activating nucleic acid molecule according to any one of claims 1-4 and 16, or the nucleic acid molecule according to claims 17 or 18, or the cell according to claim 19, or the pharmaceutical composition according to claim 20, or the preparation according to claim 21.

P21 promoter sequence (1 kb)

```
-1000 gcaggaggca aaagtcctgt gttccaacta tagtcatttc tttgctgcat gatctgagtt
 -940 aggtcaccag acttctctga gccccagttt ccccagcagt gtatacgggc tatgtgggga
 -880 gtattcagga gacagacaac tcactcgtca aatcctcccc ttcctggcca acaaagctgc
 -820 tgcaaccaca gggatttctt ctgttcaggt gagtgtaggg tgtagggaga ttggttcaat
 -760 gtccaattct tctgtttccc tggagatcag gttgcccttt tttggtagtc tctccaattc
 -700 cctccttccc ggaagcatgt gacaatcaac aactttgtat acttaagttc agtggacctc
 -640 aatttcctca tctgtgaaat aaacgggact gaaaaatcat tctggcctca agatgctttg
 -580 ttggggtgtc taggtgctcc aggtgcttct gggagaggtg acctagtgag ggatcagtgg
 -520 gaatagaggt gatattgtgg ggcttttctg gaaattgcag agaggtgcat cgtttttata
 -460 atttatgaat ttttatgtat taatgtcatc ctcctgatct tttcagctgc attgggtaaa
 -400 tccttgcctg ccagagtggg tcagcggtga gccagaaagg gggctcattc taacagtgct
 -340 gtgtcctcct ggagagtgcc aactcattct ccaagtaaaa aaagccagat ttgtggctca
 -280 cttcgtgggg aaatgtgtcc agcgcaccaa cgcaggcgag ggactggggg aggagggaag
 -220 tgccctcctg cagcacgcga ggttccggga ccggctggcc tgctggaact cggccaggct
 -160 cagctggctc ggcgctgggc agccaggagc ctgggccccg gggagggcgg tcccgggcgg
 -100 cgcggtgggc cgagcgcggg tcccgcctcc ttgaggcggg cccgggcggg gcggttgtat
  -40 atcagggccg cgctgagctg cgccagctga ggtgtgagca gct
```

**Fig. 1**

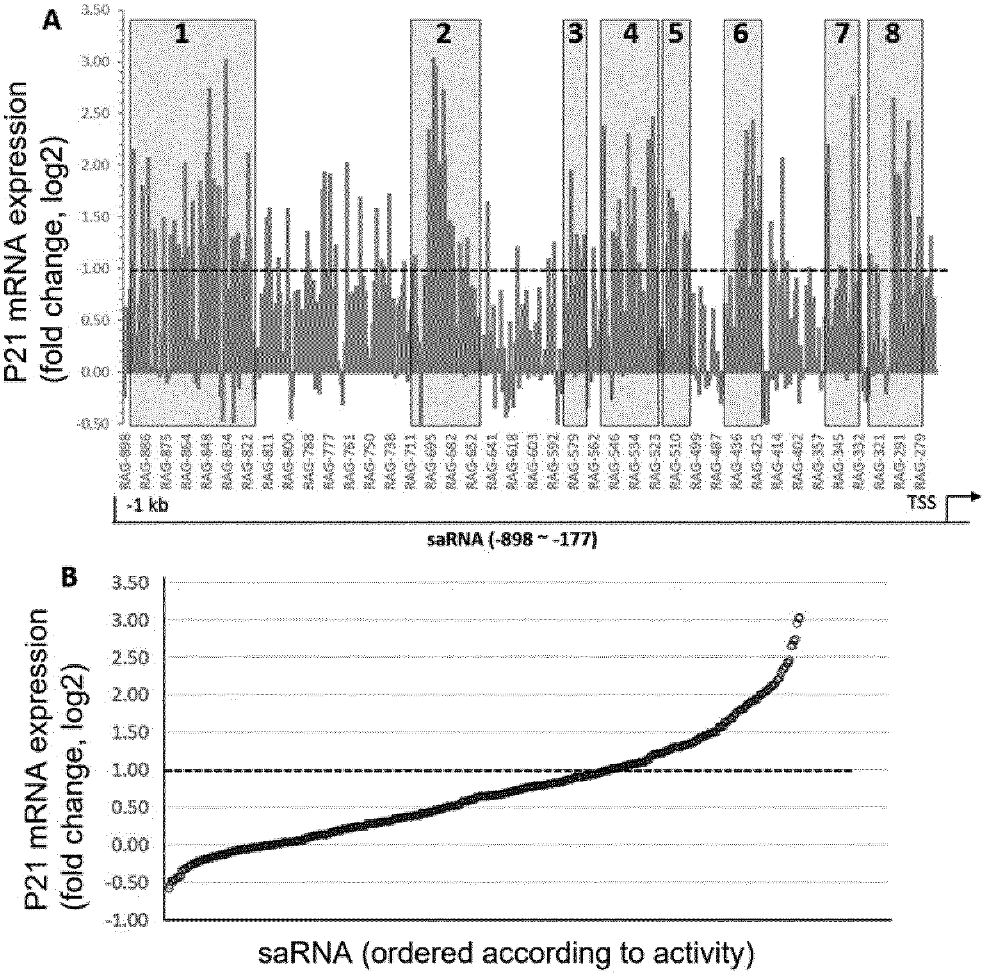
A
1
2
3
4
5
6
7
8
P21 mRNA expression
(fold change, log2)
-1 kb
TSS
saRNA (-898 ~ -177)
B
P21 mRNA expression
(fold change, log2)
saRNA (ordered according to activity)

Fig. 2

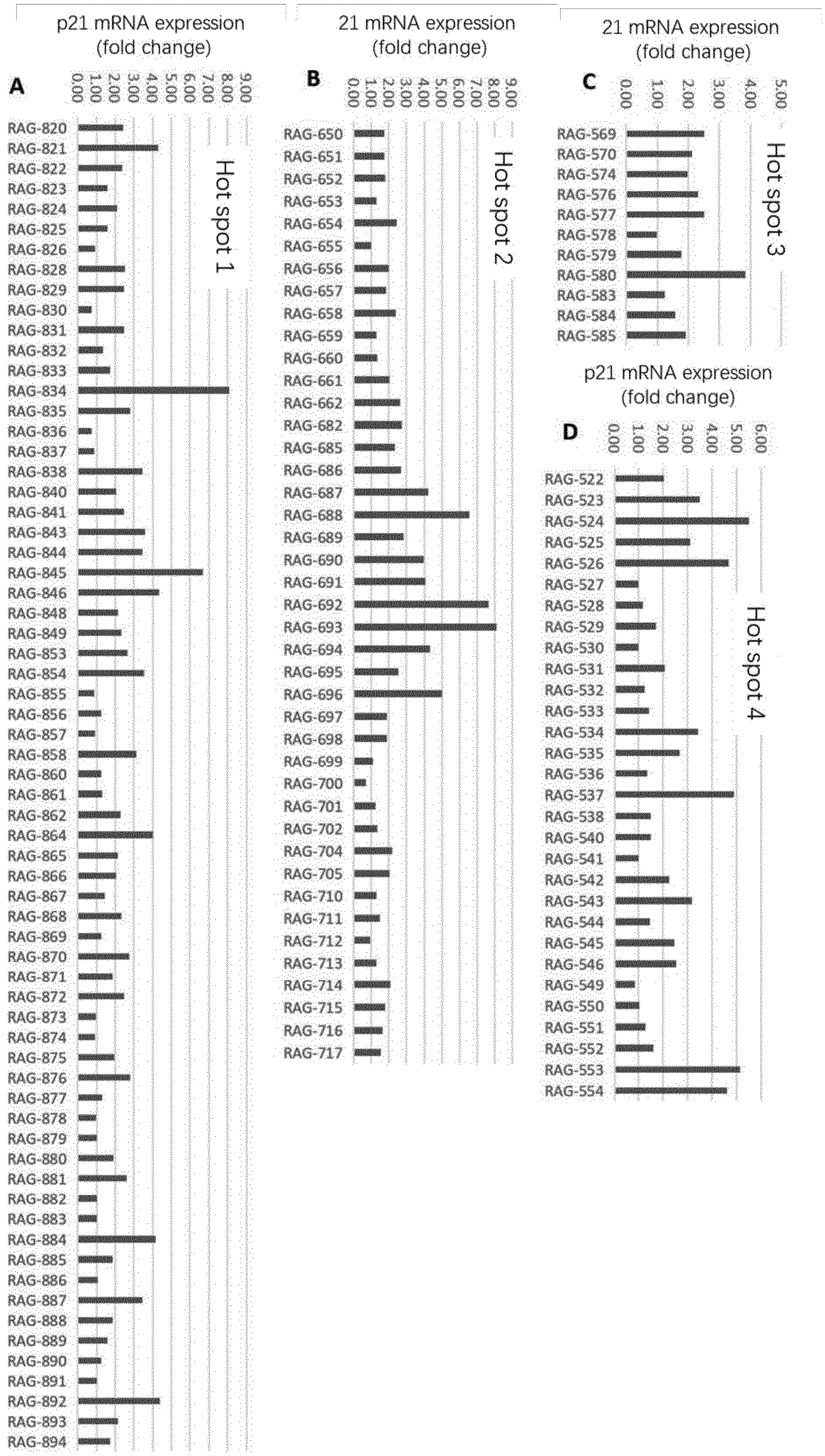
A
p21 mRNA expression
(fold change)
Hot spot 1
B
21 mRNA expression
(fold change)
Hot spot 2
C
21 mRNA expression
(fold change)
Hot spot 3
D
p21 mRNA expression
(fold change)
Hot spot 4

Figs. 3A-3D

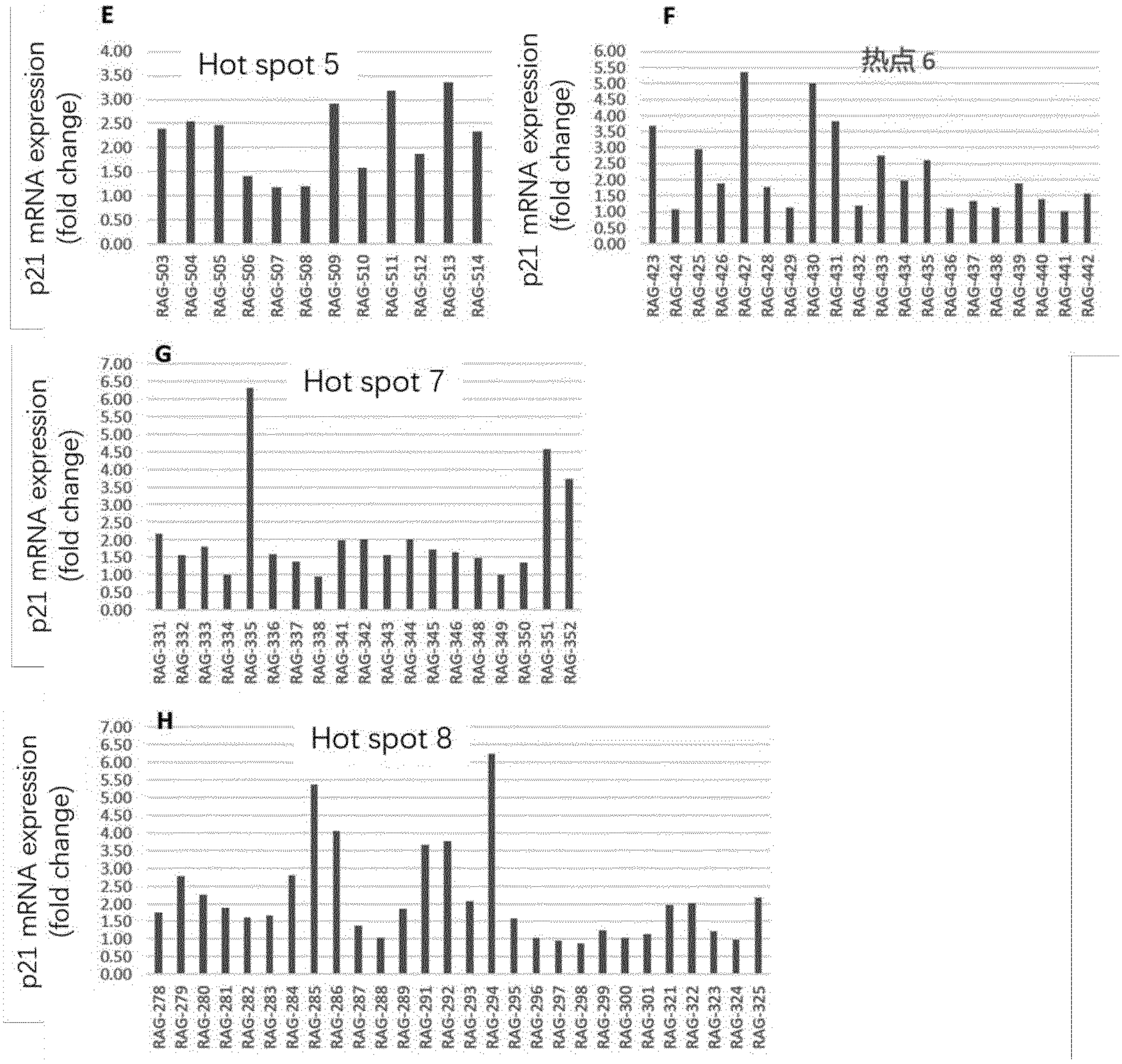
E
Hot spot 5
p21 mRNA expression (fold change)
F
热点 6
p21 mRNA expression (fold change)
G
Hot spot 7
p21 mRNA expression (fold change)
H
Hot spot 8
p21 mRNA expression (fold change)

**Figs. 3E-3H**

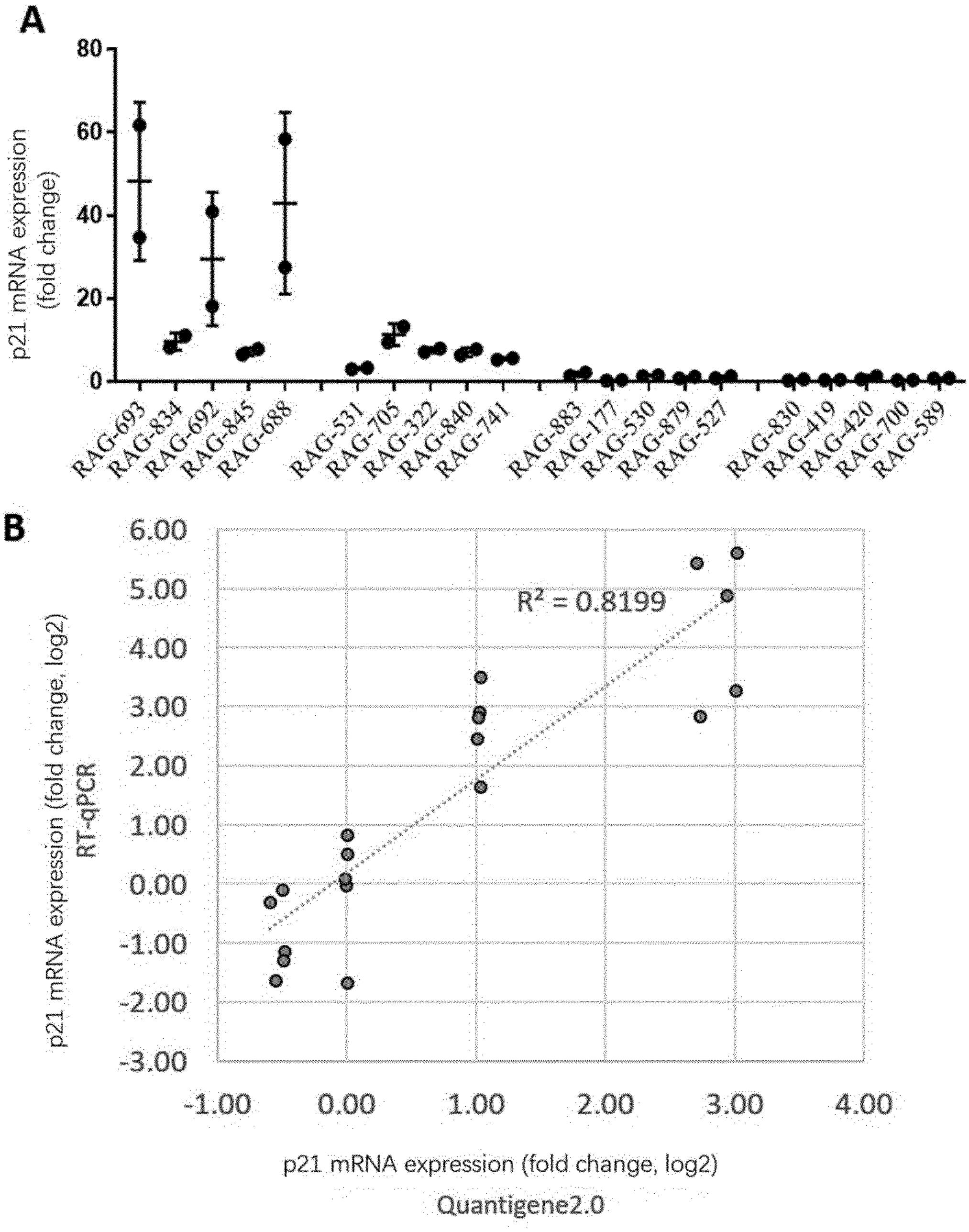
A
80
60
40
20
0
p21 mRNA expression
(fold change)
RAG-693
RAG-834
RAG-692
RAG-845
RAG-688
RAG-531
RAG-705
RAG-322
RAG-840
RAG-741
RAG-883
RAG-177
RAG-530
RAG-879
RAG-527
RAG-830
RAG-419
RAG-420
RAG-700
RAG-589
B
6.00
5.00
4.00
3.00
2.00
1.00
0.00
-1.00
-2.00
-3.00
-1.00
0.00
1.00
2.00
3.00
4.00
R² = 0.8199
p21 mRNA expression (fold change, log2)
RT-qPCR
p21 mRNA expression (fold change, log2)
Quantigene2.0

Fig. 4

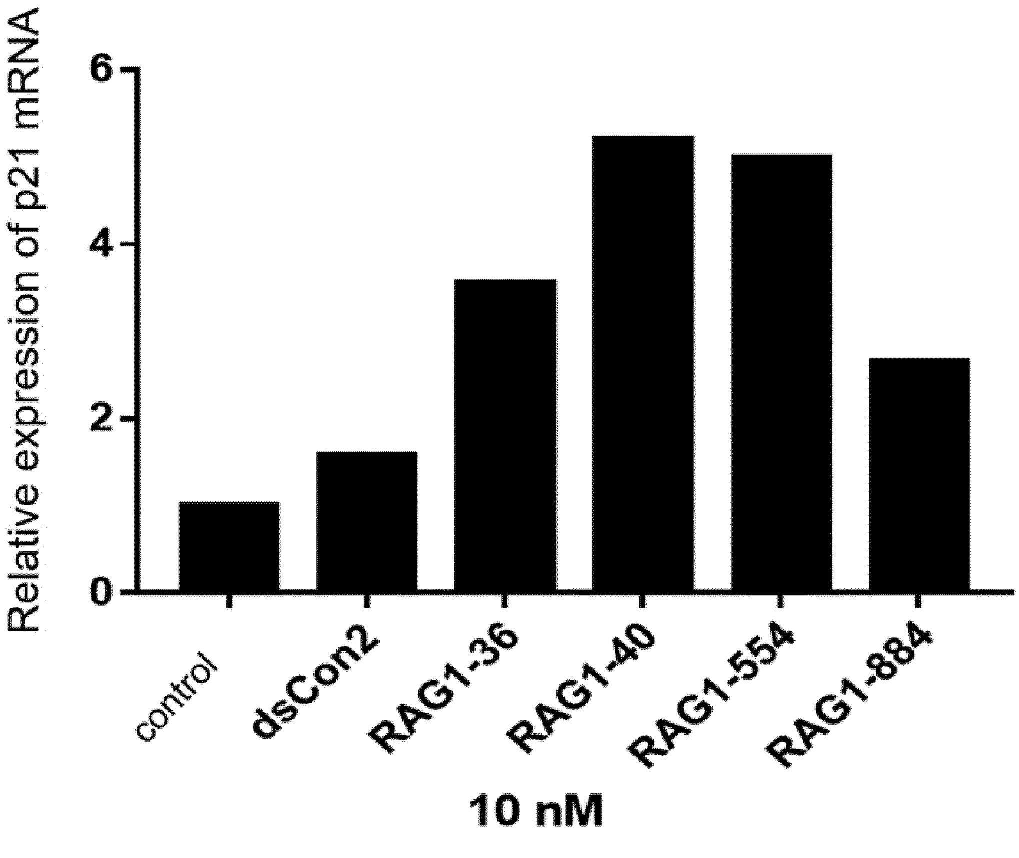
Relative expression of p21 mRNA
6
4
2
0
control
dsCon2
RAG1-36
RAG1-40
RAG1-554
RAG1-884
10 nM

Fig. 5

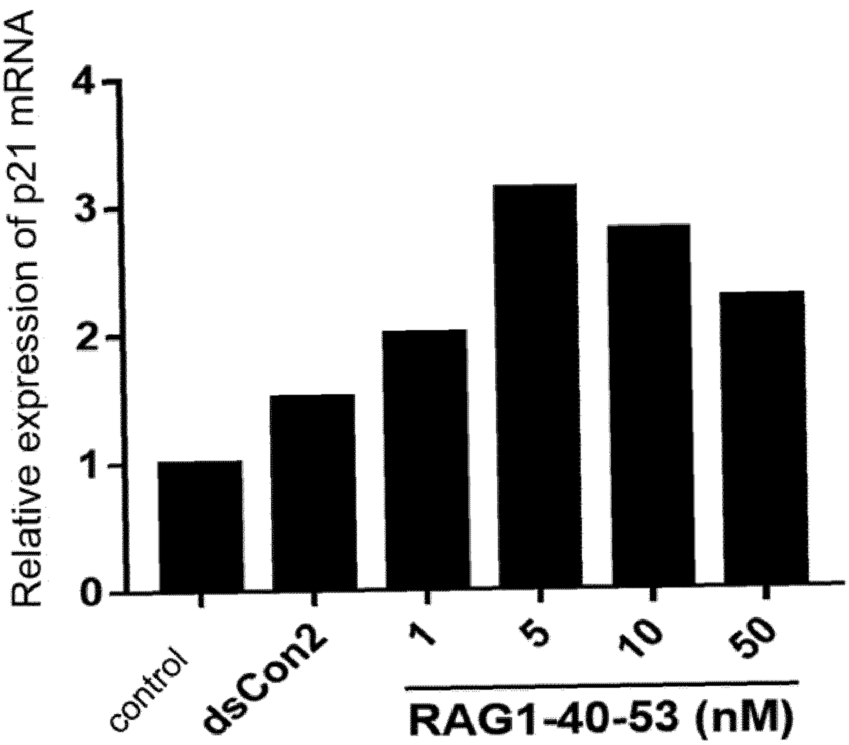
Relative expression of p21 mRNA
4
3
2
1
0
control
dsCon2
1
5
10
50
RAG1-40-53 (nM)

Fig. 6

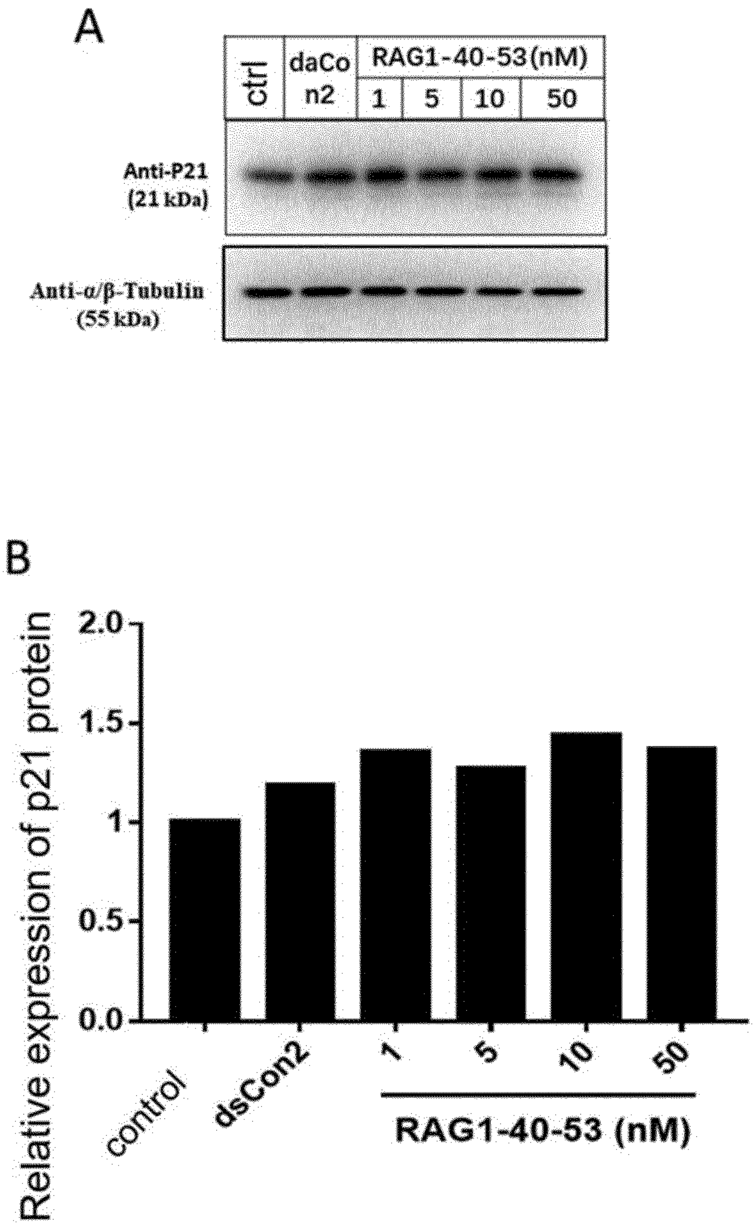
A
ctrl
daCo n2
RAG1-40-53(nM)
1
5
10
50
Anti-P21 (21 kDa)
Anti-α/β-Tubulin (55 kDa)
B
Relative expression of p21 protein
2.0
1.5
1
0.5
0.0
control
dsCon2
1
5
10
50
RAG1-40-53 (nM)

Fig. 7

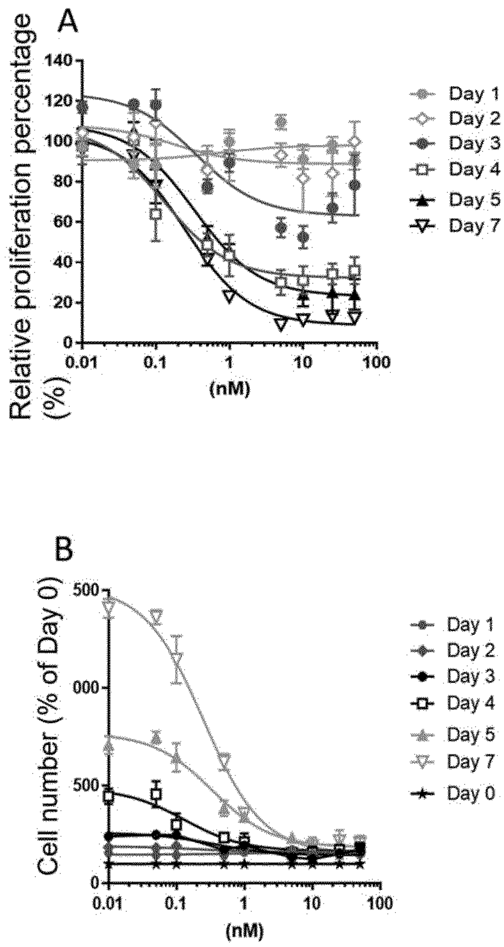
A
Relative proliferation percentage (%)
140
120
100
80
60
40
20
0
0.01
0.1
1
10
100
(nM)
Day 1
Day 2
Day 3
Day 4
Day 5
Day 7
B
Cell number (% of Day 0)
500
000
500
0
0.01
0.1
1
10
100
(nM)
Day 1
Day 2
Day 3
Day 4
Day 5
Day 7
Day 0

**Fig. 8**

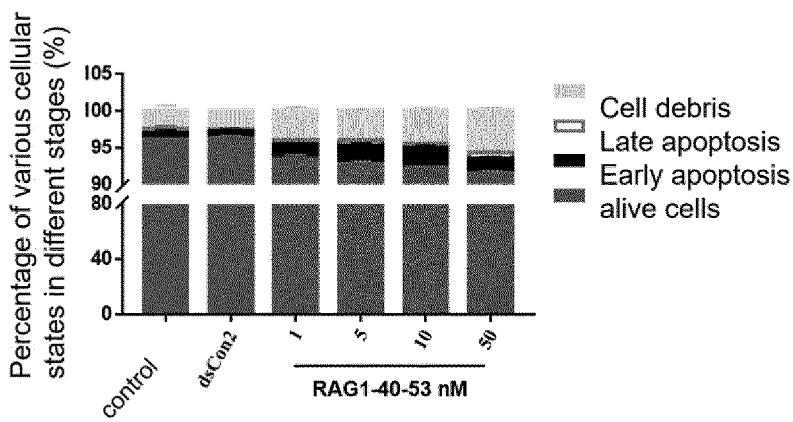
Percentage of various cellular states in different stages (%)
105
100
95
90
80
40
0
control
dsCon2
1
5
10
50
RAG1-40-53 nM
Cell debris
Late apoptosis
Early apoptosis
alive cells

**Fig. 9**

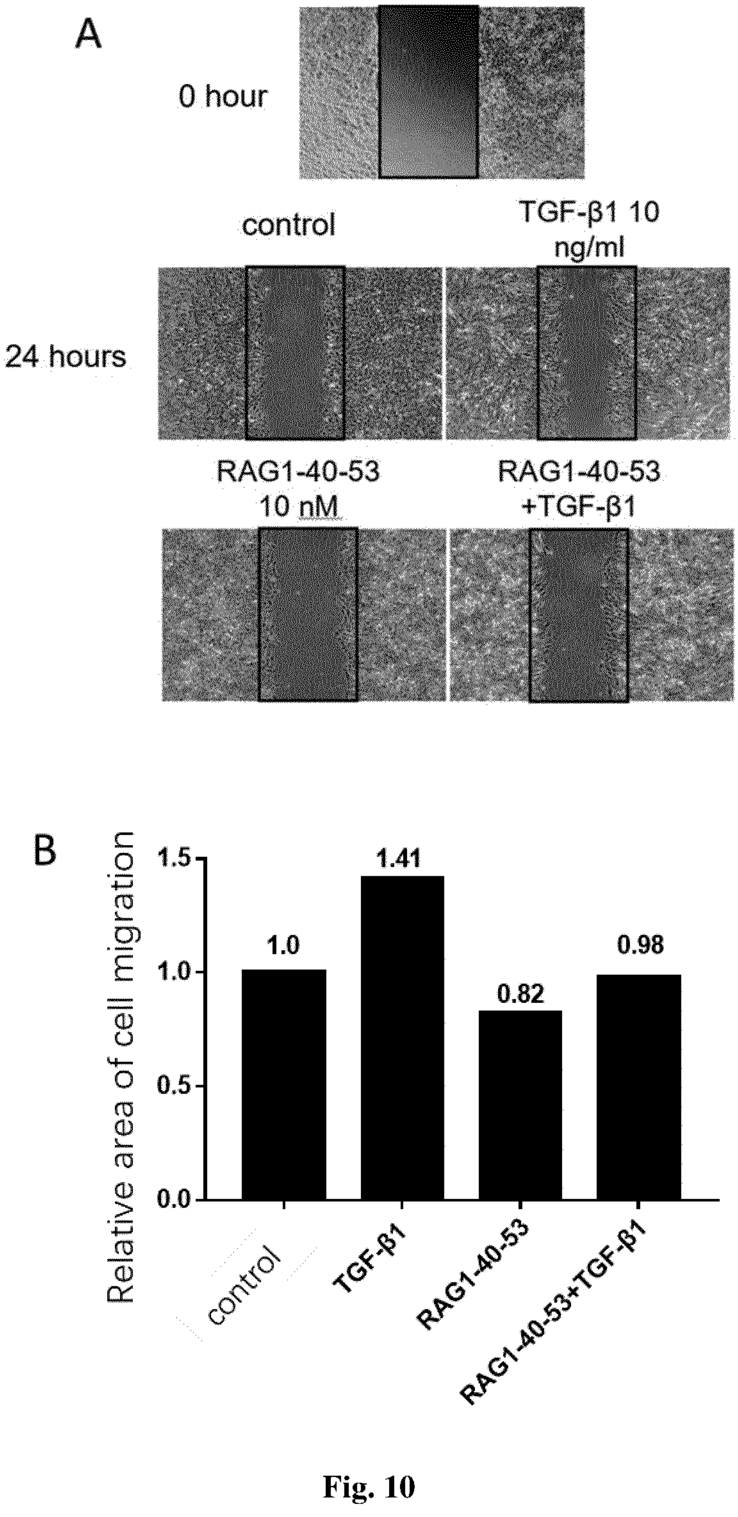
A
0 hour
control
TGF-β1 10 ng/ml
24 hours
RAG1-40-53 10 nM
RAG1-40-53 +TGF-β1
B
Relative area of cell migration
1.5
1.0
0.5
0.0
1.0
1.41
0.82
0.98
control
TGF-β1
RAG1-40-53
RAG1-40-53+TGF-β1

Fig. 10

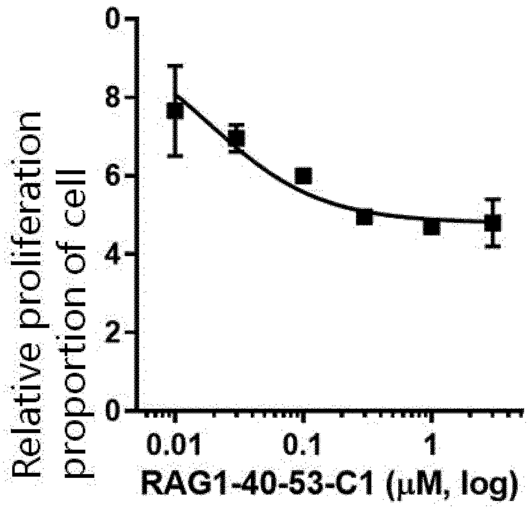
Relative proliferation proportion of cell
0
8
6
4
2
0
0.01
0.1
1
RAG1-40-53-C1 (μM, log)

Fig. 11

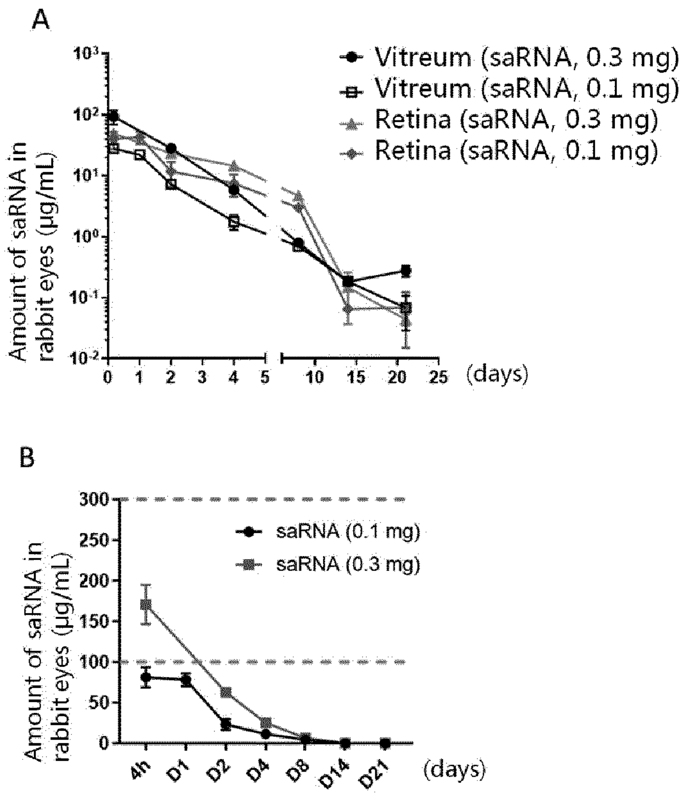
A
Vitreum (saRNA, 0.3 mg)
Vitreum (saRNA, 0.1 mg)
Retina (saRNA, 0.3 mg)
Retina (saRNA, 0.1 mg)
Amount of saRNA in rabbit eyes (μg/mL)
(days)
B
saRNA (0.1 mg)
saRNA (0.3 mg)
Amount of saRNA in rabbit eyes (μg/mL)
4h
D1
D2
D4
D8
D14
D21
(days)

Fig. 12

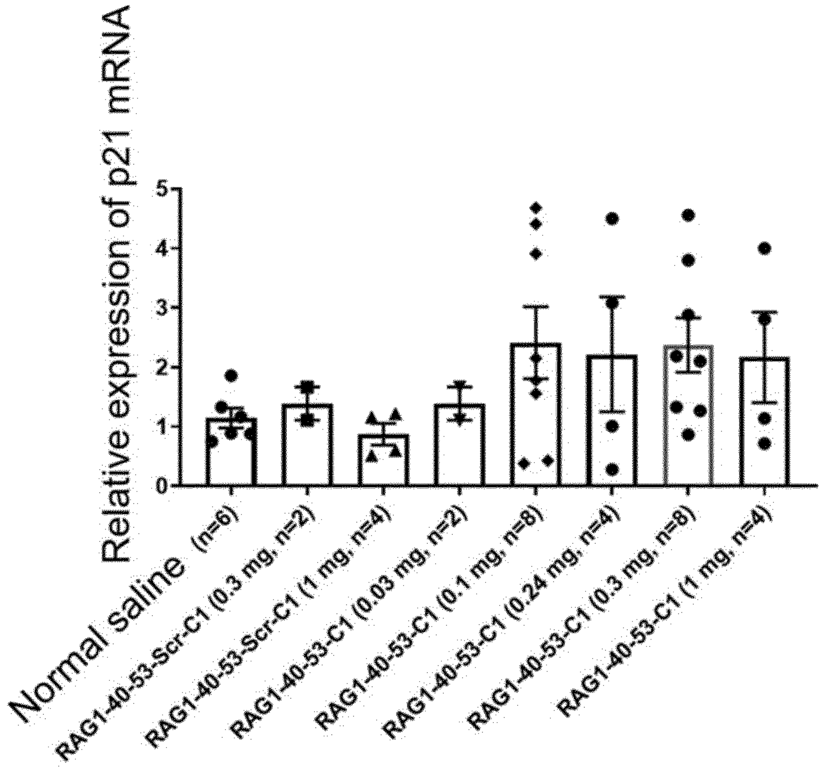
Relative expression of p21 mRNA
5
4
3
2
1
0
Normal saline (n=6)
RAG1-40-53-Scr-C1 (0.3 mg, n=2)
RAG1-40-53-Scr-C1 (1 mg, n=4)
RAG1-40-53-C1 (0.03 mg, n=2)
RAG1-40-53-C1 (0.1 mg, n=8)
RAG1-40-53-C1 (0.24 mg, n=4)
RAG1-40-53-C1 (0.3 mg, n=8)
RAG1-40-53-C1 (1 mg, n=4)

Fig. 13

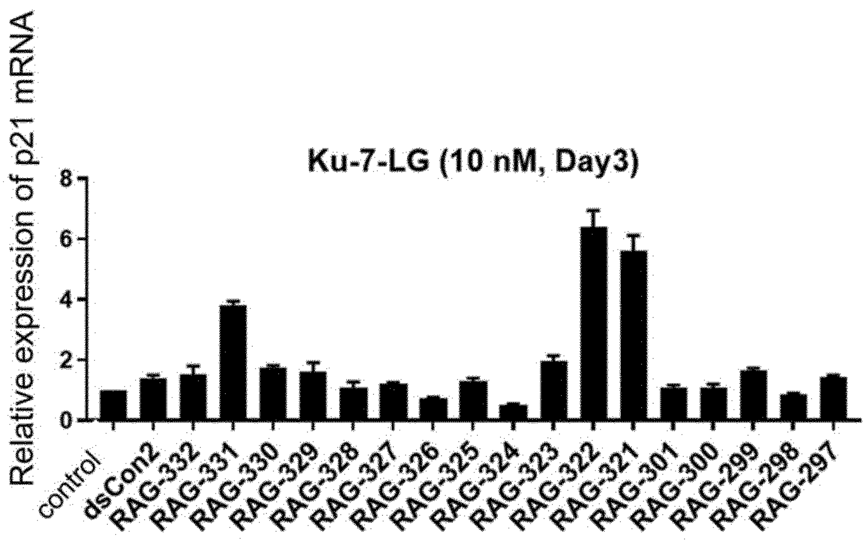
Ku-7-LG (10 nM, Day3)
Relative expression of p21 mRNA
8
6
4
2
0
control
dsCon2
RAG-332
RAG-331
RAG-330
RAG-329
RAG-328
RAG-327
RAG-326
RAG-325
RAG-324
RAG-323
RAG-322
RAG-321
RAG-301
RAG-300
RAG-299
RAG-298
RAG-297

Fig. 14

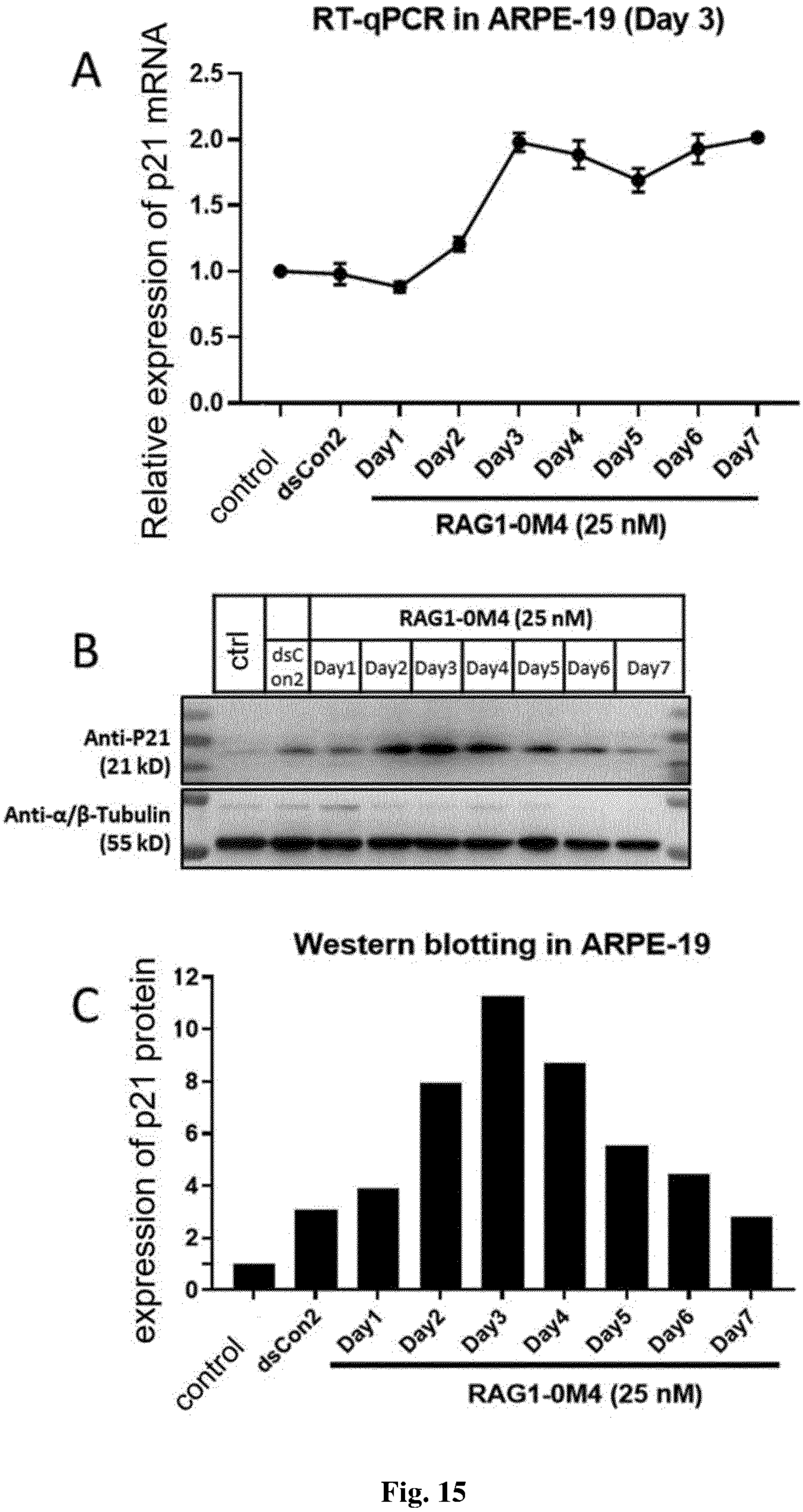
A
RT-qPCR in ARPE-19 (Day 3)
Relative expression of p21 mRNA
2.5
2.0
1.5
1.0
0.5
0.0
control
dsCon2
Day1
Day2
Day3
Day4
Day5
Day6
Day7
RAG1-0M4 (25 nM)
B
ctrl
dsCon2
RAG1-0M4 (25 nM)
Day1
Day2
Day3
Day4
Day5
Day6
Day7
Anti-P21
(21 kD)
Anti-α/β-Tubulin
(55 kD)
C
Western blotting in ARPE-19
expression of p21 protein
12
10
8
6
4
2
0
control
dsCon2
Day1
Day2
Day3
Day4
Day5
Day6
Day7
RAG1-0M4 (25 nM)

Fig. 15

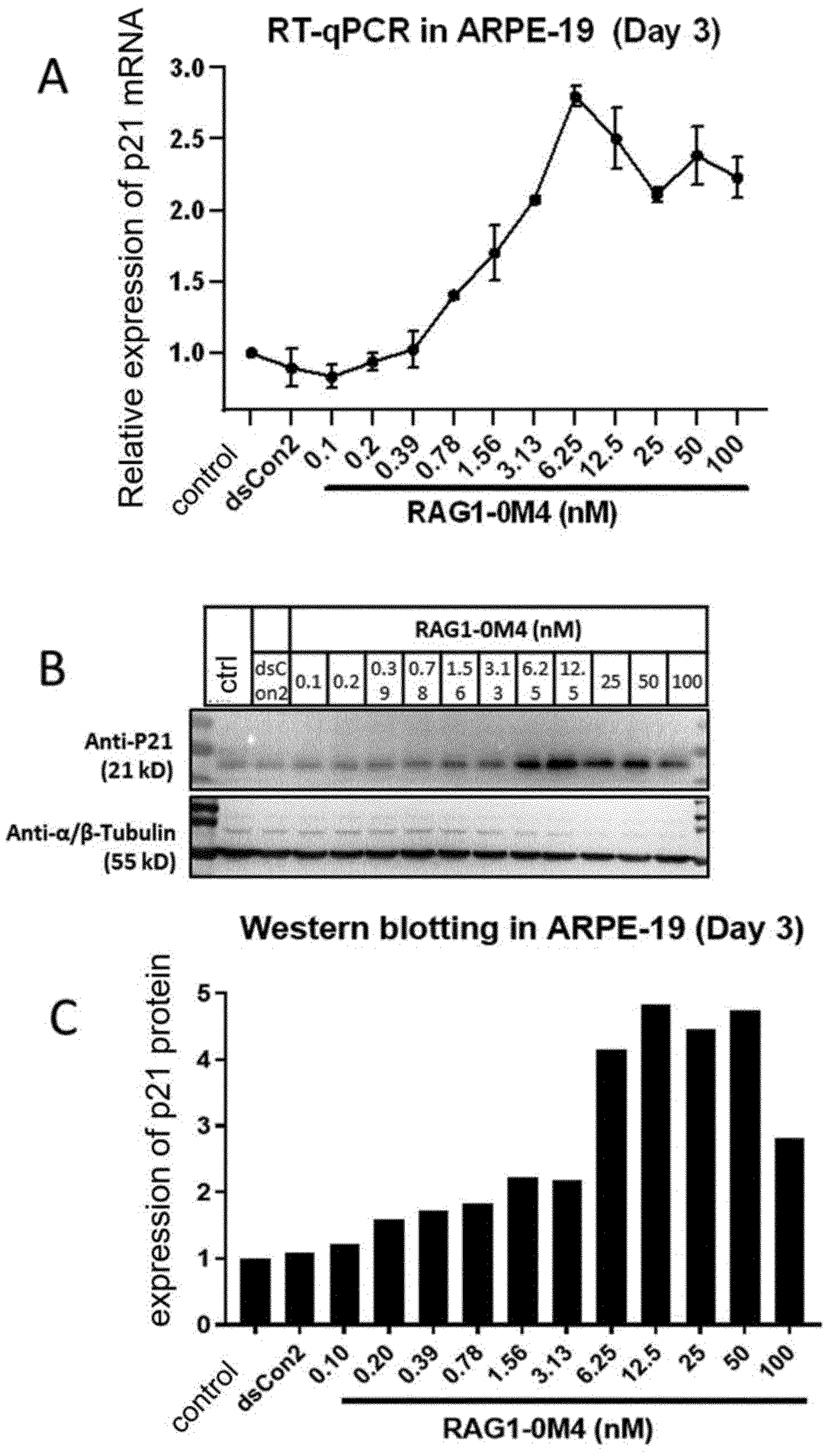
A
RT-qPCR in ARPE-19 (Day 3)
Relative expression of p21 mRNA
3.0
2.5
2.0
1.5
1.0
control
dsCon2
0.1
0.2
0.39
0.78
1.56
3.13
6.25
12.5
25
50
100
RAG1-0M4 (nM)
B
ctrl
dsCon2
RAG1-0M4 (nM)
0.1
0.2
0.39
0.78
1.56
3.13
6.25
12.5
25
50
100
Anti-P21
(21 kD)
Anti-α/β-Tubulin
(55 kD)
C
Western blotting in ARPE-19 (Day 3)
expression of p21 protein
5
4
3
2
1
0
control
dsCon2
0.10
0.20
0.39
0.78
1.56
3.13
6.25
12.5
25
50
100
RAG1-0M4 (nM)

Fig. 16

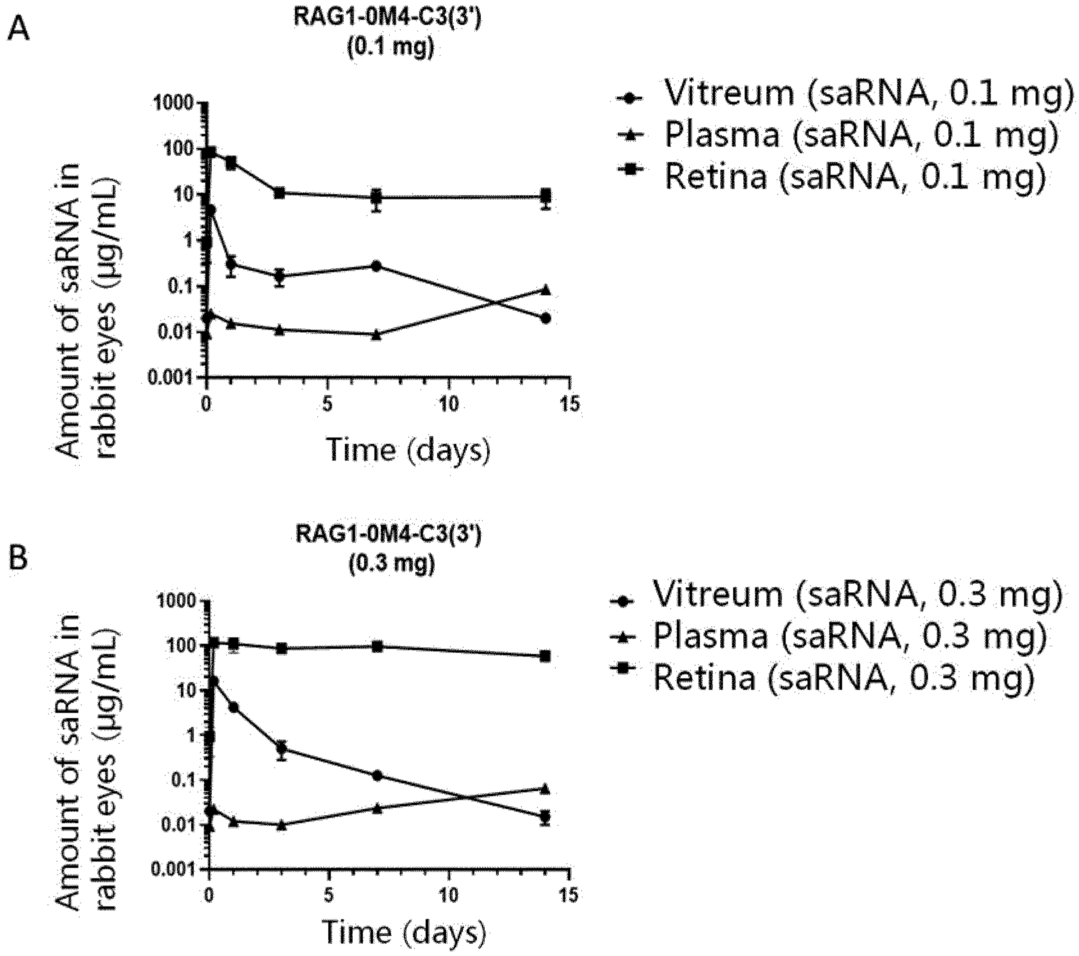
A
RAG1-0M4-C3(3')
(0.1 mg)
Vitreum (saRNA, 0.1 mg)
Plasma (saRNA, 0.1 mg)
Retina (saRNA, 0.1 mg)
Amount of saRNA in rabbit eyes (µg/mL)
1000
100
10
1
0.1
0.01
0.001
0
5
10
15
Time (days)
B
RAG1-0M4-C3(3')
(0.3 mg)
Vitreum (saRNA, 0.3 mg)
Plasma (saRNA, 0.3 mg)
Retina (saRNA, 0.3 mg)
Amount of saRNA in rabbit eyes (µg/mL)
Time (days)

Fig. 17

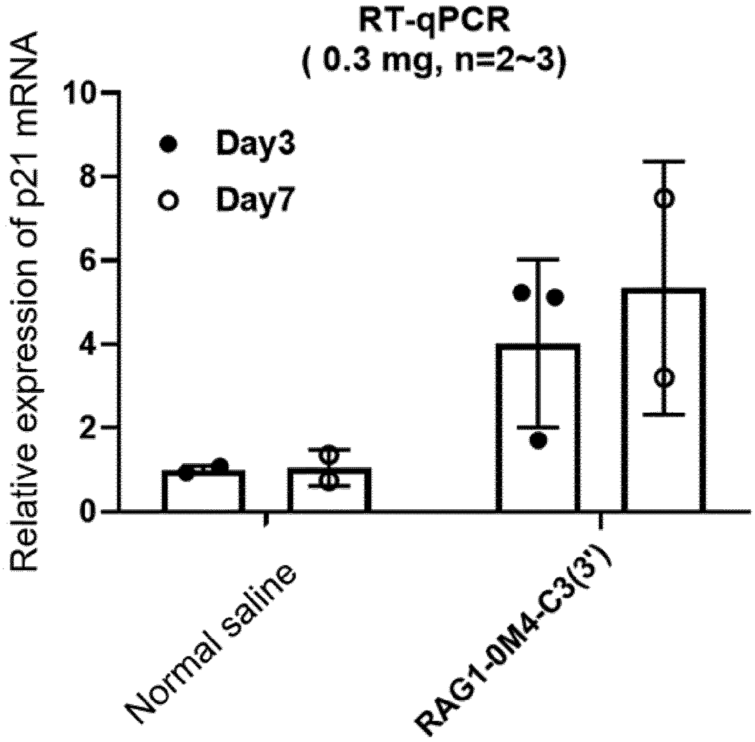
RT-qPCR
( 0.3 mg, n=2~3)
Relative expression of p21 mRNA
Day3
Day7
0
2
4
6
8
10
Normal saline
RAG1-0M4-C3(3')

Fig. 18

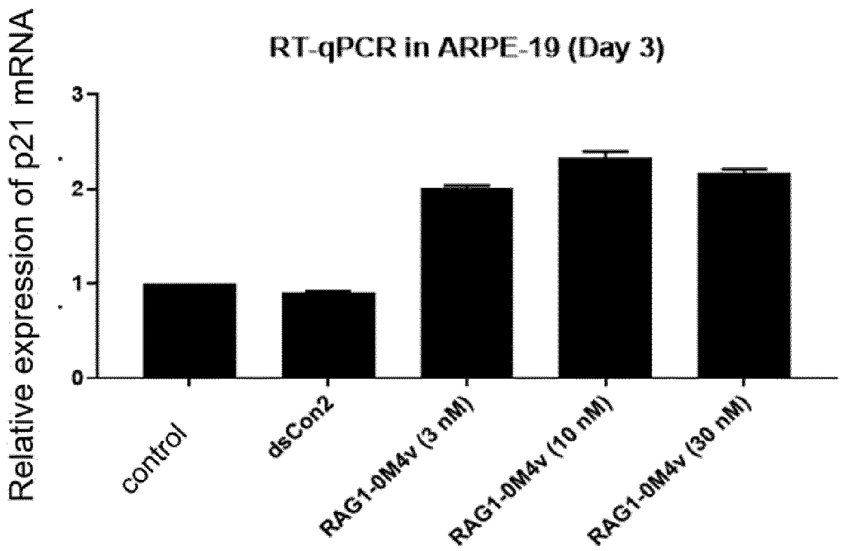
RT-qPCR in ARPE-19 (Day 3)
Relative expression of p21 mRNA
0
1
2
3
control
dsCon2
RAG1-0M4v (3 nM)
RAG1-0M4v (10 nM)
RAG1-0M4v (30 nM)

Fig. 19

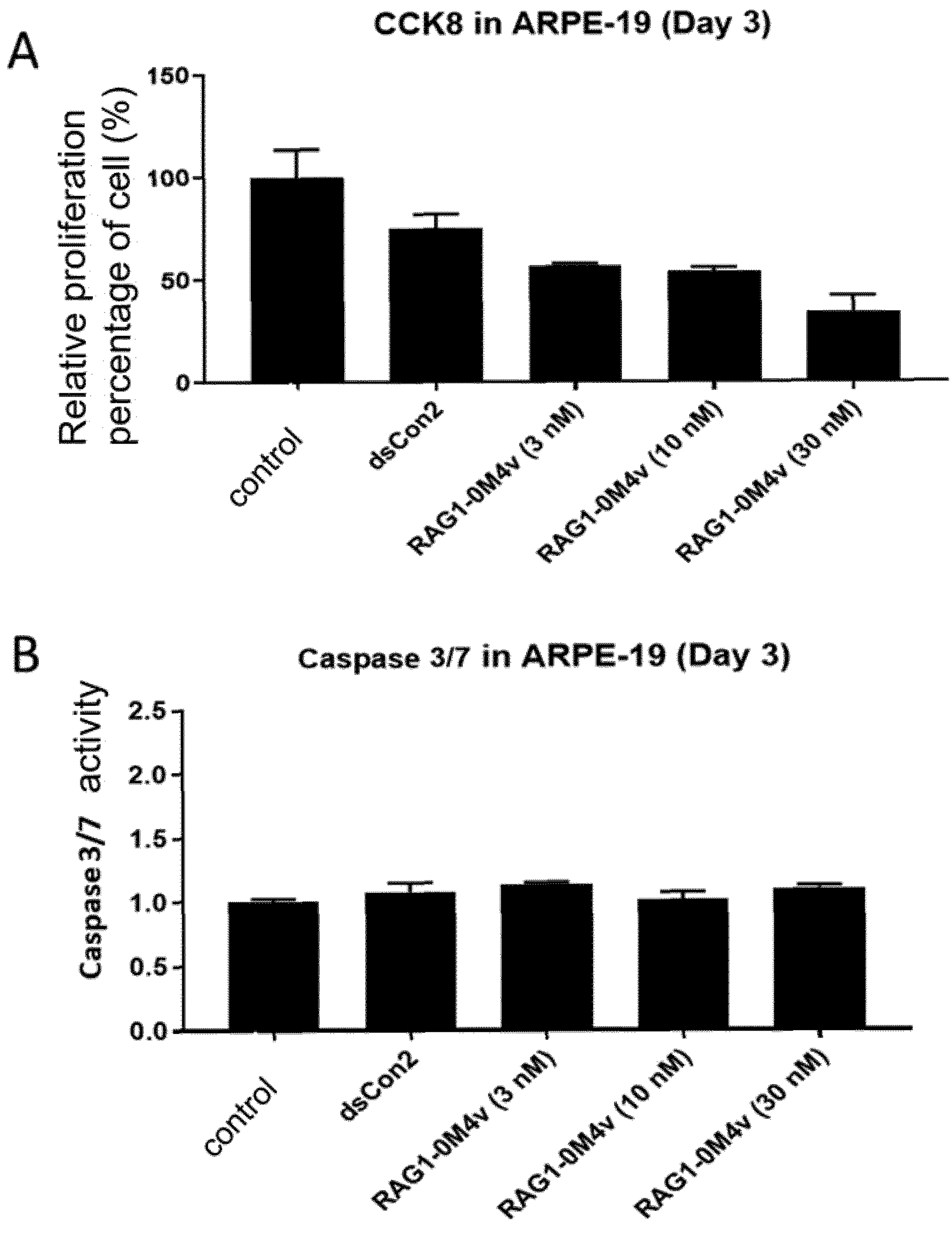
A
CCK8 in ARPE-19 (Day 3)
Relative proliferation percentage of cell (%)
150
100
50
0
control
dsCon2
RAG1-0M4v (3 nM)
RAG1-0M4v (10 nM)
RAG1-0M4v (30 nM)
B
Caspase 3/7 in ARPE-19 (Day 3)
Caspase 3/7 activity
2.5
2.0
1.5
1.0
0.5
0.0
control
dsCon2
RAG1-0M4v (3 nM)
RAG1-0M4v (10 nM)
RAG1-0M4v (30 nM)

Fig. 20

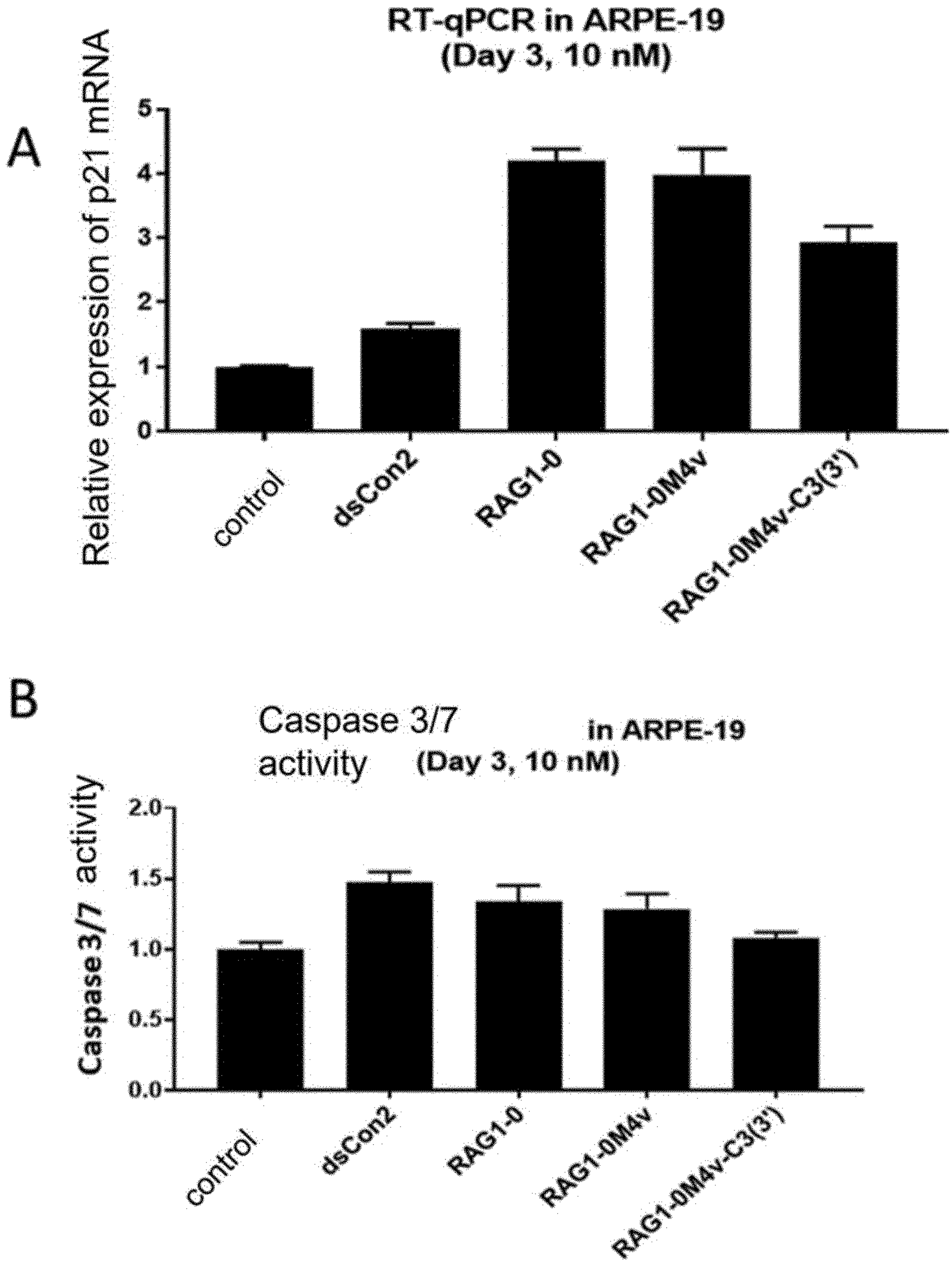
A
RT-qPCR in ARPE-19
(Day 3, 10 nM)
Relative expression of p21 mRNA
0
1
2
3
4
5
control
dsCon2
RAG1-0
RAG1-0M4v
RAG1-0M4v-C3(3')
B
Caspase 3/7 activity
in ARPE-19
(Day 3, 10 nM)
Caspase 3/7 activity
0.0
0.5
1.0
1.5
2.0
control
dsCon2
RAG1-0
RAG1-0M4v
RAG1-0M4v-C3(3')

Fig. 21

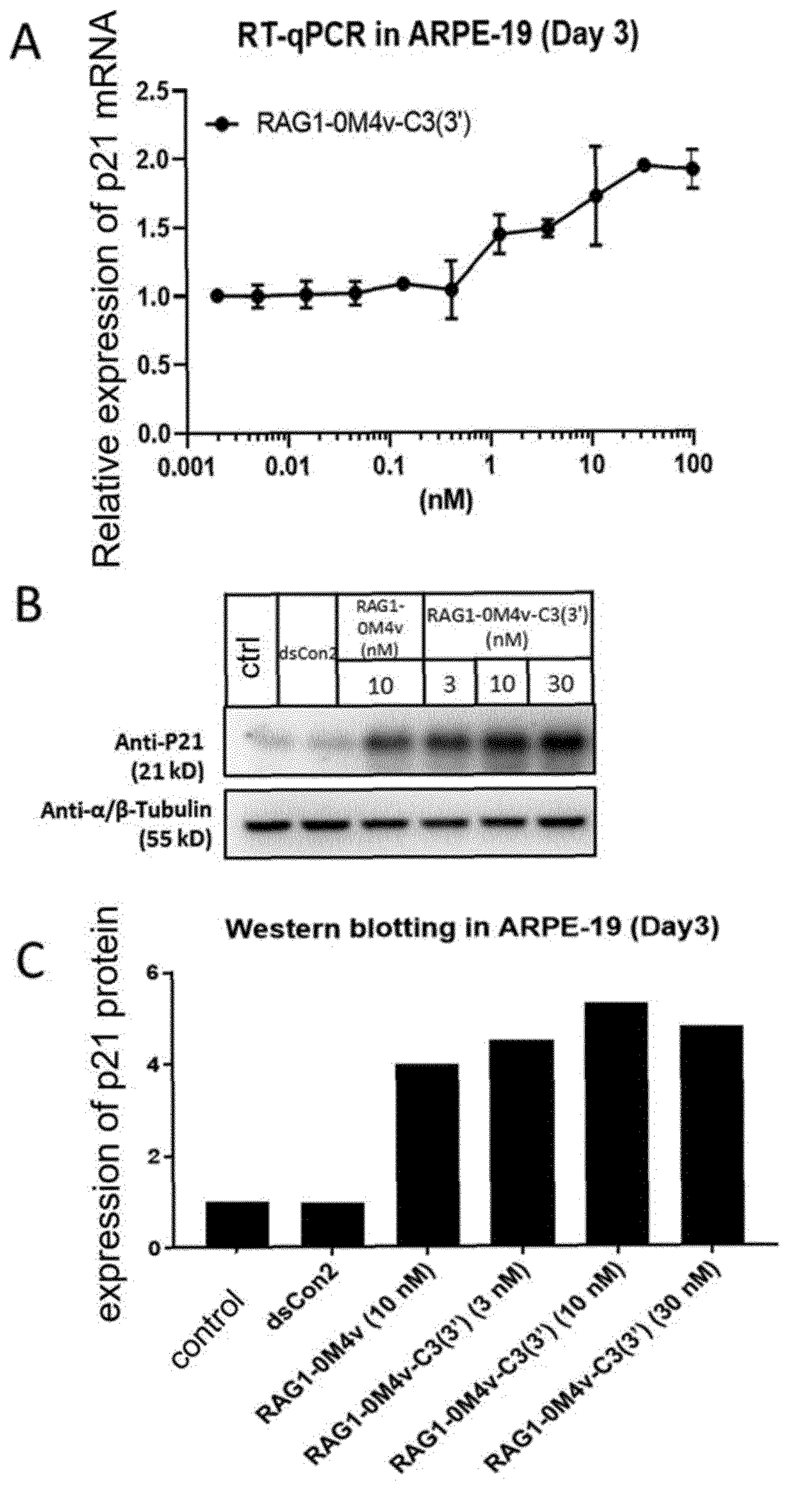
A
RT-qPCR in ARPE-19 (Day 3)
Relative expression of p21 mRNA
RAG1-0M4v-C3(3')
2.5
2.0
1.5
1.0
0.5
0.0
0.001
0.01
0.1
1
10
100
(nM)
B
ctrl
dsCon2
RAG1-0M4v (nM)
RAG1-0M4v-C3(3') (nM)
10
3
10
30
Anti-P21 (21 kD)
Anti-α/β-Tubulin (55 kD)
C
Western blotting in ARPE-19 (Day3)
expression of p21 protein
6
4
2
0
control
dsCon2
RAG1-0M4v (10 nM)
RAG1-0M4v-C3(3') (3 nM)
RAG1-0M4v-C3(3') (10 nM)
RAG1-0M4v-C3(3') (30 nM)

Fig. 22

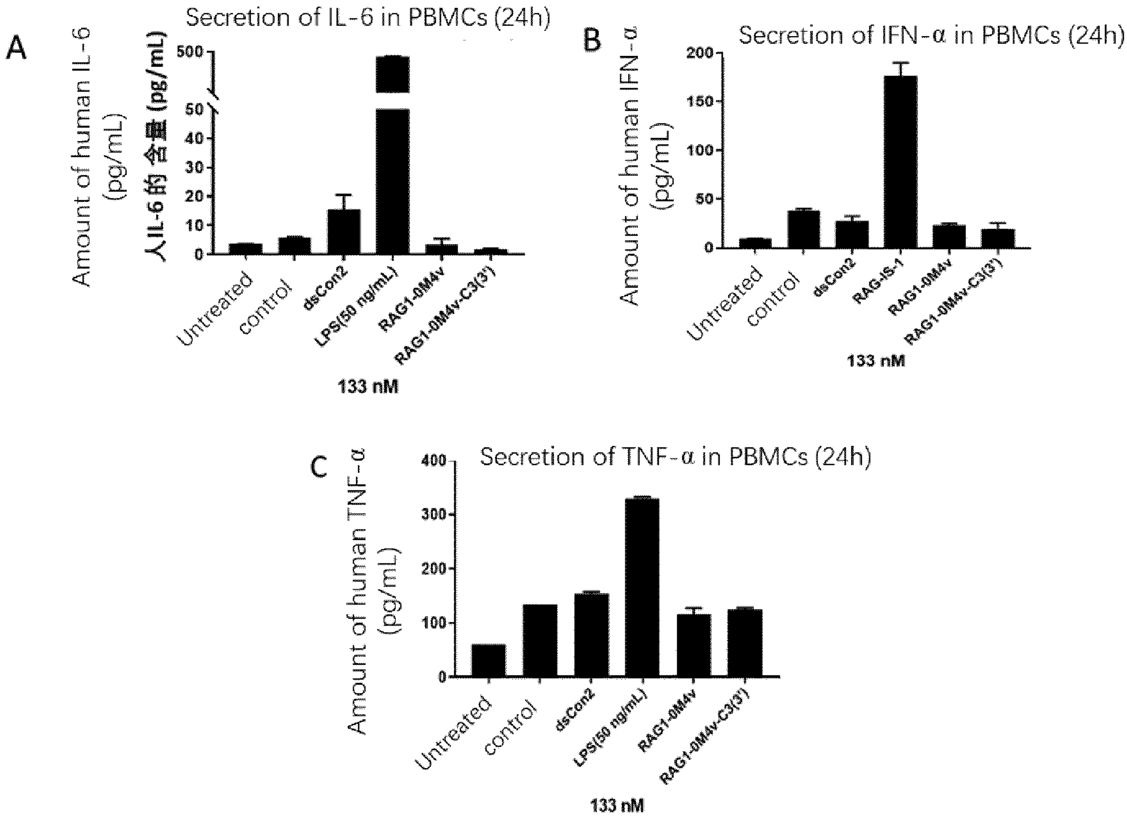
A
Secretion of IL-6 in PBMCs (24h)
Amount of human IL-6 (pg/mL)
人IL-6的含量 (pg/mL)
500
50
40
30
20
10
0
Untreated
control
dsCon2
LPS(50 ng/mL)
RAG1-0M4v
RAG1-0M4v-C3(3')
133 nM
B
Secretion of IFN-α in PBMCs (24h)
Amount of human IFN-α (pg/mL)
200
150
100
50
0
Untreated
control
dsCon2
RAG-IS-1
RAG1-0M4v
RAG1-0M4v-C3(3')
133 nM
C
Secretion of TNF-α in PBMCs (24h)
Amount of human TNF-α (pg/mL)
400
300
200
100
0
Untreated
control
dsCon2
LPS(50 ng/mL)
RAG1-0M4v
RAG1-0M4v-C3(3')
133 nM

Fig. 23

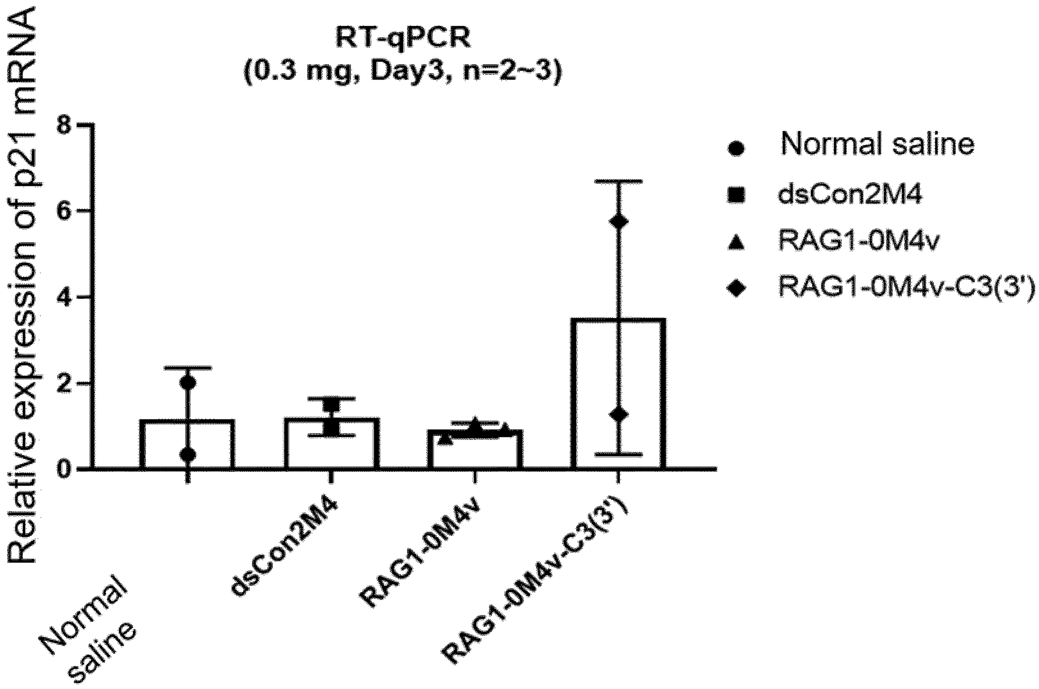
RT-qPCR
(0.3 mg, Day3, n=2~3)
Relative expression of p21 mRNA
8
6
4
2
0
Normal saline
dsCon2M4
RAG1-0M4v
RAG1-0M4v-C3(3')
Normal saline
dsCon2M4
RAG1-0M4v
RAG1-0M4v-C3(3')

Fig. 24

## INTERNATIONAL SEARCH REPORT

International application No.
**PCT/CN2022/074637**

**A. CLASSIFICATION OF SUBJECT MATTER**

C12N 15/113(2010.01)i; C12N 15/10(2006.01)i; A61K 48/00(2006.01)i; A61K 31/713(2006.01)i; A61P 35/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNTXT, CJFD, ENTXTC, CNKI, PUBMED, NCBI, UNIPRO, 中国专利生物序列检索系统, Chinese Patent Biological Sequence Retrieval System, SEQ ID Nos: 1-511, p21基因, p21蛋白, CDKN1A, SARNA, 化学修饰, 基团, 脂质, lipid, 视网膜, 玻璃体, 眼, proliferative vitreoretinopathy, PVR, eye.

**C. DOCUMENTS CONSIDERED TO BE RELEVANT**

| Category* | Citation of document, with indication, where appropriate, of the relevant passages | Relevant to claim No. |
|---|---|---|
| PX | WO 2021032777 A1 (MINA THERAPEUTICS LTD. et al.) 25 February 2021 (2021-02-25)<br>claims 1-19, and description, p. 36 | 1-23 |
| X | WO 2019196883 A1 (SINO-US INSTITUTE OF RNA TECHNOLOGY) 17 October 2019 (2019-10-17)<br>claims 1-23, and description, pp. 2-4 and 11-23 | 1-11, 15-23 |
| X | WO 2019196887 A1 (SINO-US INSTITUTE OF RNA TECHNOLOGY) 17 October 2019 (2019-10-17)<br>claims 1-18, and description, pp. 19-30, and tables 1-4 | 1-11, 15-23 |
| X | CN 106032532 A (PEKING UNION MEDICAL COLLEGE HOSPITAL, CHINESE ACADEMY OF MEDICAL SCIENCES) 19 October 2016 (2016-10-19)<br>claims 1-10, and description, pp. 2 and 3, and a sequence table | 1-10, 19-23 |
| Y | WO 2019196883 A1 (SINO-US INSTITUTE OF RNA TECHNOLOGY) 17 October 2019 (2019-10-17)<br>claims 1-23, and description, pp. 2-4 and 11-23 | 11-14, 16-25 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 April 2022** | **26 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/074637**

**C. DOCUMENTS CONSIDERED TO BE RELEVANT**

| Category* | Citation of document, with indication, where appropriate, of the relevant passages | Relevant to claim No. |
|---|---|---|
| Y | WO 2019196887 A1 (SINO-US INSTITUTE OF RNA TECHNOLOGY) 17 October 2019 (2019-10-17)<br>claims 1-18, and description, pp. 19-30, and tables 1-4 | 11-14, 16-25 |
| Y | CN 111849968 A (SINO-US INSTITUTE OF RNA TECHNOLOGY) 30 October 2020 (2020-10-30)<br>description, pp. 6 and 7 | 11-14, 16-25 |
| Y | US 2020208152 A1 (MINA THERAPEUTICS LTD.) 02 July 2020 (2020-07-02)<br>description, p. 23 | 11-14, 16-25 |
| Y | 杨建刚等 (YANG, Jiangang et al.). "细胞周期蛋白依赖性激酶抑制剂在眼科的研究进展 (Non-official translation: Research Progress of Cell Cycle Protein Dependent Kinase Inhibitor in Ophthalmology)"<br>*世界核心医学期刊文摘 眼科学 (Digest of the World Core Medical Journals: Ophthalmology),* Vol. 1, No. 8, 31 August 2005 (2005-08-31),<br>p. 2 | 23-25 |
| A | 赵利建等 (ZHAO, Lijian et al.). "核酸药物，时代已至 (Non-official translation: Nucleic Acid Drugs, the Era Is Coming)"<br>*中金公司证券研究报告 (Non-official translation: Securities Research Report of CICC),* 08 January 2021 (2021-01-08),<br>p. 1, right column | 1-25 |

**INTERNATIONAL SEARCH REPORT**

International application No.
**PCT/CN2022/074637**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2022/074637**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **25**
because they relate to subject matter not required to be searched by this Authority, namely:

[1] Claim 25 relates to a method for treating and alleviating proliferative vitreoretinopathy, which belongs to a disease treatment method as defined in PCT Rule 39.1(iv). However, a search has been performed on the basis of a pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/074637**

| Patent document cited in search report | Publication date (day/month/year) | Patent family member(s) | Publication date (day/month/year) |
|---|---|---|---|
| WO 2021032777 A1 | 25 February 2021 | None | |
| WO 2019196883 A1 | 17 October 2019 | US 2021024915 A1 | 28 January 2021 |
| | | KR 20200143428 A | 23 December 2020 |
| | | JP 2021520220 A | 19 August 2021 |
| | | EP 3778893 A1 | 17 February 2021 |
| | | CN 110678549 A | 10 January 2020 |
| | | CN 113584027 A | 02 November 2021 |
| WO 2019196887 A1 | 17 October 2019 | CA 3094575 A1 | 17 October 2019 |
| | | EP 3778892 A1 | 17 February 2021 |
| | | US 2021332366 A1 | 28 October 2021 |
| | | IL 277921 D0 | 30 November 2020 |
| | | CN 110959042 A | 03 April 2020 |
| | | AU 2019252204 A1 | 26 November 2020 |
| | | JP 2021520221 A | 19 August 2021 |
| | | KR 20200140377 A | 15 December 2020 |
| CN 106032532 A | 19 October 2016 | None | |
| CN 111849968 A | 30 October 2020 | None | |
| US 2020208152 A1 | 02 July 2020 | EP 3679140 A1 | 15 July 2020 |
| | | WO 2019048645 A1 | 14 March 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ALTSCHUL et al.** *J. Mol. Biol.,* 1990, vol. 215, 403-410
- **SAMBROOK.** Molecular Cloning: A Laboratory Manual. Cold Spring Harbor Laboratory Press, 1989
- **AUDO, I. ; S. R. DARJATMOKO ; C. L. SCHLAMP ; J. M. LOKKEN ; M. J. LINDSTROM ; D. M. ALBERT ; R. W. NICKELLS.** Vitamin D analogues increase p53, p21, and apoptosis in a xenograft model of human retinoblastoma. *Invest Ophthalmol Vis Sci,* 2003, vol. 44, 4192-9
- **FILLIES, T. ; M. WOLTERING ; B. BRANDT ; J. P. VAN DIEST ; R. WERKMEISTER ; U. JOOS ; H. BUERGER.** Cell cycle regulating proteins p21 and p27 in prognosis of oral squamous cell carcinomas. *Oncol Rep,* 2007, vol. 17, 355-9
- **HEATLEY, G. ; J. KILAND ; B. FAHA ; J. SEEMAN ; C. L. SCHLAMP ; D. G. DAWSON ; J. GLEISER ; D. MANEVAL ; P. L. KAUFMAN ; R. W. NICKELLS.** Gene therapy using p21WAF-1/Cip-1 to modulate wound healing after glaucoma trabeculectomy surgery in a primate model of ocular hypertension. *Gene Ther,* 2004, vol. 11, 949-55
- **KWON, O. W. ; J. H. SONG ; M. I. ROH.** Retinal Detachment and Proliferative Vitreoretinopathy. *Dev Ophthalmol,* 2016, vol. 55, 154-62
- **MUDHAR, H. S.** A brief review of the histopathology of proliferative vitreoretinopathy (PVR). *Eye (Lond),* 2020, vol. 34, 246-50
- **ROMANOV, V. S. ; K. L. RUDOLPH.** p21 shapes cancer evolution. *Nat Cell Biol,* 2016, vol. 18, 722-4
- **SADAKA, A. ; G. P. GIULIARI.** Proliferative vitreoretinopathy: current and emerging treatments. *Clin Ophthalmol,* 2012, vol. 6, 1325-33
- **UEDA, Y. ; S. KANAZAWA ; T. KITAOKA ; Y. DAKE ; A. OHIRA ; A. M. OUERTANI ; T. AMEMIYA.** Immunohistochemical study of p53, p21 and PCNA in pterygium. *Acta Histochem,* 2001, vol. 103, 159-65
- **ZANDI, S. ; I. B. PFISTER ; P. G. TRAINE ; C. TAPPEINER ; A. DESPONT ; R. RIEBEN ; M. SKOWRONSKA ; J. G. GARWEG.** Biomarkers for PVR in rhegmatogenous retinal detachment. *PloS one,* 2019, vol. 14, e0214674
- **FASTENBERG, D. M. ; K. R. DIDDIE ; N. SORGENTE ; S. J. RYAN.** A comparison of different cellular inocula in an experimental model of massive periretinal proliferation. *Am J Ophthalmol,* 1982, vol. 93, 559-64